# EUROPEAN PATENT APPLICATION

(11) **EP 2 388 271 A2**
(43) Date of publication of application: **23.11.2011**
(21) Application number: 11177723.1
(22) Date of filing: 03.02.2006
(51) Int. Cl.: C07K 16/00, C07K 19/00, C07K 16/32, C07K 16/28, C07K 16/42, C07K 16/24, C07K 16/46, C07K 14/18, C07K 14/415, C07K 14/815

(54) **Human Antibodies and Proteins**

(30) Priority: 03.02.2005 GB 0502201; 16.02.2005 GB 0503190; 05.04.2005 GB 0506945
(62) Divisional of application: 06709606.5
(71) Applicant: Antitope Limited, Babraham, Cambridge CB2 4AT (GB)
(72) Inventor: Jones, Timothy David, Babraham, Cambridge CB2 4AJ (GB); Baker, Matthew Paul, Babraham, Cambridge CB2 4AT (GB)
(74) Representative: Chapman, Paul William

(57) **Abstract**

The present invention provides composite antibody variable regions or fragments thereof, which show reduced immunogenicity. In particular, composite antibody variable regions for use in humans are provided, in particular variable regions which have been modified to remove one or more T-cell epitopes. Methods for generating such variable regions are also provided.

## Description

The present invention relates to generation of antibodies and proteins which combine two or more segments of amino acid sequence from a human antibody or protein within the final antibody or protein molecule. In particular, the present invention provides such combinations of sequence segments such that the number of T cell epitopes in the final antibody or protein molecule is reduced or avoided. The invention particularly relates to the generation of antibodies and proteins for use as pharmaceutical agents in humans or as *in vivo* diagnostic agents.

The last 20 years has seen great advances in the generation of recombinant monoclonal antibodies for use as potential pharmaceuticals in man. The techniques of chimerization, humanization and human antibody cloning either by phage display or transgenic mice have provided antibodies which are generally well tolerated when administered to man with less immunogenicity than with non-human monoclonal antibodies. However, several antibodies generated by these techniques have been shown to elicit immunogenicity in patients even where the genetic origins of such antibodies are human. For example, the human antibody Humira®, elicits immunogenicity in 12% of rheumatoid arthritis patients and the humanized antibody CAMPATH® elicits immunogenicity in about 50% of patients. Such induction of immunogenicity is likely to result from the presence, within the antibody variable region, of tracts of non-self amino acid sequences which, in some cases, can create T cell epitopes which induce T cell responses resulting in immunogenicity. There is therefore a need for improved techniques and antibody compositions which have a high human origin but which avoid, as much as possible, creation of sequences which might induce T cell responses.

The present invention provides methods and resultant antibody compositions whereby, for therapeutic use, such antibodies (herein termed "composite antibodies") combine two or more segments of amino acid sequence from a human antibody within the final antibody molecule.

Thus, in a first aspect, the present invention provides modified antibody or antigen-binding fragment thereof wherein the heavy and light chain variable regions of the modified antibody or antigen-binding fragment are each composed of two or more segments of amino acid sequence from one or more other antibodies or antigen-binding fragments, whereby the segments are neither whole CDRs nor framework regions.

In the context of the present invention, the term "segments" refers to contiguous amino acid sequence found within an antibody molecule, such segments ranging in size from 2 to 125 amino acids long, preferably ranging from 2 to 31 amino acids long where such segments are neither whole CDRs nor whole framework regions. For therapeutic use, composite antibodies of the present invention will typically combine two or more segments of amino acid sequence from different human antibodies within the variable regions of the composite antibody. In particular, the present invention relates to composite antibody heavy and light chain variable regions (VH and VL respectively) where each VH and VL is composed entirely of segments of sequence from two or more human antibody variable regions and where typically each composite VH and VL includes segments of human variable region sequence positions corresponding to their positions in the source human antibody VHs and VLs, for example amino acids 1 to 10 in the composite VH sequence will derive from amino acids 1 to 10 in a human antibody. Alternatively, segments of human VH or VL sequence in the composite antibody may be positioned at any sequence location irrespective of the sequence position in the source human antibody VH or VL. The source human antibody VHs and VLs will be any existing human antibody variable (V) region amino acid sequence, for example as provided in databases of human monoclonal antibody V region sequences, and may include sequences from affinity-matured antibodies with V region somatic mutations and other variations differing from germ-line, sequences from germ-line V regions, sequences from artificially constructed antibody V regions created from segments of sequence from antibodies of the species such as antibodies with a set of fixed V region frameworks but with variable CDRs, sequences selected from human antibody libraries such as phage display libraries, and sequences of human antibodies derived from transgenic animals expressing genes encoding human antibodies or antibody fragments.

In a preferred embodiment of the present invention, composite antibodies of the invention for therapeutic use are constructed by combining multiple human VH and VL sequence segments in combinations which limit or avoid human T cell epitopes in the final composite antibody V regions.

Human T cell epitopes in this respect are amino acid sequences which can bind to human MHC class II molecules and, through presentation to CD4⁺ T cells, induce a helper T cell response. Human VH and VL sequence segments and combinations of segments can be chosen which limit or avoid T cell epitopes in the final composite antibody. This can be achieved by use of segments which do not contain T cell epitopes, such as from human germ-line sequences, and by joining of adjacent segments to create a new sequence which does not contain T cell epitopes, for example by creation of a non-MHC binding sequence at the junction of two segments, by creation of another human germ-line sequence, or by creation of a sequence which does not induce a helper T cell response despite a non-germ-line sequence.

In another preferred embodiment of the present invention, additional amino acid sequences can be added or created within the composite antibody molecules which provide for one or more regulatory T cell epitopes ("Tr epitopes"). For the purpose of the invention, Tr epitopes are MHC binding peptides which stimulate CD4+ CD25+ T cells with the ability to regulate immune responses by the secretion of inhibitory cytokines such as IL-10 and TGF-β, as well as contact dependent mechanisms. As such, within the scope of the invention, regulatory T cell epitopes can include peptides shown to induce one or more activities *in vitro* or *in vivo* which could contribute to regulation of immune responses under certain conditions. For example, regulatory T cell epitopes will include peptides with the action of inducing or activating CD4+ CD25+ T cells, with the action of inducing release of inhibitory cytokines such as IL-10 and/or TGF-β, or with other measurable immunosuppression-related activities either *in vitro* or *in vivo,* in all cases where the actions are related to the action of CD4+ CD25+ T cells. Thus, such Tr epitopes can provide an additional measure to limit or avoid immunogenicity in the composite antibody. Tr epitopes can be introduced into the composite antibody VH or VL by incorporation of segments of human VH and VL containing these epitopes or by creation of such epitopes via combination of two or more human sequence segments or by screening for new Tr epitopes, for example from peptides corresponding to segments of human antibody or protein sequence, for induction or activation of CD4+ CD25+ T cells, for example by measurement of release of inhibitory cytokines such as IL-10 and/or TGF-β (e.g. Hall et al., Blood, vol.100 (2002) p4529-36). Alternatively, known Tr epitopes can be incorporated within composite antibody V regions at positions within VH and/or VL which do not inhibit binding or function or expression of the composite antibody or can be incorporated at one terminus of the composite VH or VL sequence, for example at the N terminus of VH. Alternatively, Tr epitopes can be incorporated into one or both constant regions of a composite antibody at locations which do not interfere with function of the composite antibody (e.g. within the hinge regions) or cause some other deleterious effect such as lack of expression. Alternatively, for one or both of composite VH and VL's within antibody fusion proteins, antibody conjugates, Fab and Fv-type forms (including single chain antibodies (SCAs) with VH and VL linked), single domain antibodies, or homodimeric antibodies, Tr epitopes can be incorporated at locations which do not interfere with function of the composite antibody or cause some other deleterious effect such as lack of expression. For example, in SCAs, an especially preferred location for a Tr epitope is within the linker region joining VH and VL. Optimally, Tr epitopes will be flanked by appropriate sequences to optimise the release and presentation of regulatory T cell epitopes on MHC class II molecules, for example by flanking the epitope with sequences that are sensitive to the action of endocytic proteases. Typically, flanking residues at positions ranging from P-20 to P30 (with the core nonomer defined as P1-P9) that will target the action of proteases during antigen processing are introduced, if necessary using additional segments of human antibody sequence.

As discussed herein, the present invention also provides methods for the production of modified or composite antibodies. Thus, in another aspect, the present invention provides a method for producing a modified antibody comprising the steps;
(1) preparing antibody variable region genes by combining segments of amino acid sequence from a range of other antibody variable regions in order to generate a library of different variable region genes
(2) cloning the library of antibody variable region genes into an expression vector
(3) screening the library of antibody variable regions and recovering members of the library with desirable properties

In a first preferred method 'A' of the present invention, a library of composite human antibodies is generated and screened for antibodies with desirable properties such as binding to a specific antigen. This method involves 6 steps as follows;
(1) design of composite VH and VL genes
(2) cloning of composite VH and VL genes
(3) expression of composite VH and VL genes
(4) screening and selection of composite antibodies with desirable properties
(5) optimisation of lead composite antibodies
(6) (optional) avoidance of T cell epitopes

For step (1), the library of composite VH and VL sequences are designed by selecting segments of VH and VL sequence from known human V region sequences such as those available in the Kabat antibody database (www.bioinf.org.uk/abs/simkab.html), the NCBI database (www.ncbi.nlm.nih.gov) and from protein databases, such as UniProt (www.ebi.uniprot.org) and PRF/SEQDB (www.prf.or.jp). In addition, these can be supplemented by collection of human VH and VL sequences by direct sequencing of amplified VH and VL mRNA from one or more individual donors. Various combinations of sequence segments can be considered for design of VH and VL genes. One method used is to fix the length of the composite VH and VL sequences and to design these using fixed length sequence segments from corresponding Kabat numbering positions in different human V regions.

For example, the library would comprise VH and VL regions of 121 and 107 amino acids respectively and would include, for example, an assortment of different segments for VH amino acids 1-27 using Kabat numbering. For VH with CDRs corresponding to Kabat numbering CDR1:30-35, CDR2:50-66 and CDR3:95-106, sequence segments for the following Kabat positions are used as one option: 1-27, 28-31, 32-36, 37-42, 43-50, 51-56, 57-60, 61-63, 64-69, 70-82a, 82b-96, 97-98, 99-101, 102-117. For VL with CDRs corresponding to Kabat numbering CDR1:24-34, CDR2: 50-56, CDR3:89-97, sequence segments for the following Kabat positions are used as one option: 1-22, 23-27, 28-30, 31-33, 34-35, 36-47, 48-52, 53-55, 56-59, 60-87,88-92,93-94,95-107. Therefore, in this example, composite VHs are composed of 14 human segments and composite VLs are composed of 13 human segments. In practice, a computer program is used to generate combinations of these segments. Preferably, the program includes an algorithm to avoid non-preferred combinations of certain segments which might, for example, avoid certain canonical structures of CDRs or which might disrupt VH and/or VL folding or VH/VL interaction. As an optional addition, the program could include an algorithm to limit the number of T cell epitopes formed by the combination of sequence segments (see *in silico* methods in step (6) below)..

For step (2), having designed a library of composite human sequences, composite VH and VL genes are then generated preferably using synthetic oligonucleotides. Typically, synthetic oligonucleotides encoding longer segments of V region sequence will be ligated to a mixture of oligonucleotides which encode two or more consecutive segments of V region sequence. Alternatively, composite V regions could be assembled by other methods such as overlapping PCR or other amplification techniques using existing human VH and VL genes as templates. For example, using PCR, small segments of V regions can be amplified separately and then joined by overlapping PCR reactions.

In other methods, mixed synthetic oligonucleotides can be produced to create a range of sequence segments preferably using doping methods to enrich for sequences encoding specific V region segments. Composite human VH and VL genes with extensive variability of human V region segment representation can be assembled in many ways using techniques known to those skilled in the art such as those described in Molecular Cloning: A Laboaratory Manual; 3rd Ed., vols. 1-3 (2001) Cold Spring Harbor Laboratory Press and using standard PCR methods for immunoglobulins such as those described in Orlandi et al., Proc Natl Acad Sci U S A.,86 (1989) 3833-3837. For step (3), once composite human VH and VL genes are generated, these can be cloned into a variety of expression vectors for production of either complete antibody molecules or antigen-binding fragments such as Fv's, Fab's, Fab2, SCAs, single domain antibodies (e.g. comprising VHs only) and multimeric derivatives of each of these. Alternatively, VH and VL genes can be fused to genes encoding other molecules to generate fusion proteins. Also included might be sequences encoding detectable markers such as poly-histidine tags at the C terminus of one chain of an Fv or Fab. Expression vectors include those for expression in mammalian cells, bacterial cells, bacteriophage, yeast, fungus and other micro-organisms. Such vectors also include those for expression *in vivo* from transgenic animals and those for expression using *in vitro* systems such as *in vitro* translation using ribosome preparations.

For step (4), screening of libraries of composite human antibodies is usually for binding to one or more specific antigens of interest. There are many screening methods known to those skilled in the art, the selection of which will depend on the form of expression of the composite human antibodies and the composition of the antibody molecules i.e. complete antibody or Fab, Fv, SCA, single domain antibody etc. In some cases where an existing antibody is available which binds to the antigen of interest, either VH or VL from this antibody may be combined with the composite human VL or VH respectively and tested for binding.

Screening methods will range from immobilising individual members of the library or pools of such members on a solid phase to immobilising the antigen of interest either individually or in pools. Where antibodies are immobilised, the antigen of interest is then added and is either detectable directly or indirectly by addition of one or more additional reagents. For example, if the antigen is a fusion protein or conjugate with an enzyme such as alkaline phosphatase, detection can be achieved by subsequent addition from a wide range of substrates which produce colour, fluorescence or chemiluminescent signals. Where antibody pools are immobilised in one location (e.g. the well of a microtitre dish) and a signal results from addition of antigen, this pool can then be dereplicated prior to rescreening of either individual members of the pool or smaller pools. Where the antigen of interest is immobilised, the composite antibody library may be screened in several ways ranging from addition of individual antibodies to the antigen of interest which is immobilised at a specific location, to addition of pools of antibody, to addition of the whole composite library and subsequent recovery of antibodies bound to the antigen of interest. In the last case, a common strategy is to immobilise antigen on a solid phase such as in a column or on beads, to add the library, to subsequently wash the solid phase for example with a low salt buffer (to detach loosely associated members of the library), and to then elute antibodies which bind to the antigen using, for example, a high salt buffer. Common formats for expression of members of the library for this purpose are phage display, yeast display, ribosome display and bead display, in each case where nucleic acid encoding composite VH and VL chains remains attached to the composite V region which binds to the antigen.

Screening methods will also include functional or biological tests which may be substituted for direct antigen binding tests where a functional or biological activity is measured such as *in vitro* tests involving cell growth, cell growth inhibition, cell differentiation or cell migration, or alternative *in vivo* tests involving measuring responses to the antigen at the level of the whole organism, for example changes in blood cell counts in a mouse or growth inhibition of a transplanted tumour.

For step (5), following selection of one or more "lead" composite human antibodies with desirable properties such as binding to an antigen of interest, optionally the properties of the lead antibody may be improved, for example by increasing affinity for binding to the antigen or fusing the antibody to an additional moiety. Increased affinity may be achieved by mutagenesis of composite variable region sequences in order to select for mutations in the selected composite V region sequences which increase or alter binding in a desirable way. The present invention includes novel methods for mutagenesis of variable region sequence by replacing one or more individual V region sequence segments from the lead antibody with corresponding sequence segments from one or more human antibody sequences. In particular, segments overlapping with or within CDR region may be replaced by one or more alternative segments from other human antibodies including segments of different lengths. Within the scope of the invention, specific segments may be included from human antibodies with related properties to the selected lead antibody, for example from antibodies which bind to the same antigen, or from non-human antibodies with related properties, or from human antibodies with sequence segments which retain certain key amino acids which appear important for function in a non-human antibody with related sequence. One or more composite human antibodies subject to such mutagenesis can then be screened for improved properties.

For the optional step (6), following selection of a lead composite human antibody, T cell epitopes are limited or avoided by, where required, exchanging V region segments contributing to or encoding a T cell epitope with alternative segments which avoid T cell epitopes. Such T cell epitopes can be detected by a range of methods. For example, peptides corresponding to one or more loci in the composite V region sequence can be synthesised and tested in T cell assays to determine the presence of T cell epitopes. Typically such peptides will be 15 amino acids in length and, where it is desirable to test a longer contiguous V region sequence, overlapping peptides from the sequence such as 15mers with 12 amino acid overlaps are used. For detection of T cell epitopes, a range of different T cell assays can be used for measurement of activation or proliferation of CD4+ T cells such as those measuring cytokine release, proliferation (for example, by uptake of 3H-thymidine), Ca2+ flux, surface marker expression, gene transcription etc.

Alternatively, overlapping peptides corresponding to the composite V region sequences are analysed for binding to human MHC class II molecules either using in *vitro* methods or *in silico* methods, in each case to determine potential T cell epitopes i.e. MHC binding peptides which may induce a T cell response. *In silico* methods will include methods involving modelling of peptide-MHC class II binding interactions, methods involving identification of motifs common for binding to MHC class II and methods using databases of peptides or specific amino acids within peptides with known in vitro MHC binding properties. Other methods can be used such as producing longer peptides from composite V region sequences or whole antibodies containing composite V region sequences and testing these in T cell assays or in MHC binding assays, for example by testing for MHC-peptide tetramers, or by searching the proposed or constructed sequences in a database of known human T cell epitopes. Avoidance of T cell epitopes in composite human V regions can also be assisted by avoidance of MHC class II binding motifs or avoidance of particular amino acids which anchor the binding of peptides to MHC class II. In the preferred method for avoidance of T cell epitopes from one or more lead composite human antibodies, *in silico* methods are initially applied to analyse the composite human antibody V regions for potential T cell epitopes and, where these are identified, new segments of human VH or VL sequence are introduced to avoid these epitopes and to avoid introduction of new T cell epitopes.

Following any such introduction of new human V region segments and rescreening of such modified lead composite human antibodies for desirable properties, one or more final lead composite human V region can then be further tested in human T cell assays either by testing overlapping peptides typically of 15 to 45 amino acids in length, for example 15mer peptides with 12 amino acid overlaps from the composite human V region sequences (whole V regions or parts thereof) or by testing whole composite human antibodies directly in human T cell assays. A final analysis using T cell assays for testing whole composite human antibody is preferred allowing for direct testing for T cell activation against the whole antibody.

In a second preferred method `B' of the present invention, a library of composite human antibodies is generated to include desirable amino acids from one or more reference antibodies with desirable properties. This method involves 7 steps as follows;
(1) sequence analysis of one or more reference antibodies
(2) design of composite VH and VL genes
(3) (optional) avoidance of T cell epitopes
(4) cloning of composite VH and VL genes
(5) expression of composite VH and VL genes
(6) screening and selection of composite antibodies with desirable properties
(7) optimisation of lead composite antibodies including optional avoidance of T cell epitopes

In step (1), typical reference antibodies will be rodent, especially mouse, with properties and/or binding specificities which are desirable in a human form of antibody. Where one or more reference antibody V region sequences are available, these are analysed to determine sequences of the CDRs and to identify amino acids which might be important for the desirable properties of the antibody such as binding specificity. For a reference antibody, such analysis is performed, for example, by alignment of the reference V region sequences with other sequences of the same species and also, if the reference antibody is non-human, human V region sequences. Such aligments are performed, for example, using the program CLUSTAL (Thompson et al., Nucleic Acids Res. 22 (1994) p4673-80). Such alignments can identify unusual or rare amino acids in the V region of the reference antibody and homologous V region families. In addition; conserved V region structures such as canonical structures of the CDRs can be identified using, for example, the Protein Data Bank (Berman et al.: The Protein Data Bank, Nucleic Acids Research, 28 (2000) 235-242). In addition, the reference antibody variable regions can be modelled, where a structure is not known, using modelling software such as MODELLER (Sali and Blundell, J. Mol. Biol. 234 (1993) p779-815) and, in some cases, models of antibody-antigen interactions can be generated. Such analyses of the reference antibody V regions are used to guide on selection of segments of human V region sequence for the composite human antibody.

For step (2), having determined amino acids which might be important for the desirable properties of the composite human antibody, segments of human V region sequences are then selected to include some or all of these amino acids. A library of composite human V region sequences is thereby designed including selected segments with typically one or more alternative human V region segments at particular loci where the effect of such segments on properties of the composite human antibody is uncertain. Such composite human antibody sequences can be further analysed as with the reference antibody(s) by alignment with other human antibody sequences and conserved structures and, in addition, further modelling of the structure of composite human antibody V regions can be undertaken in order to refine, as required, the combinations of human V region segments used in the composite human antibodies to avoid defects in protein structure, intermolecular and intramolecular interactions within composite V regions, and incorrect structural orientations of important amino acids.

For the optional step (3), as an additional criteria for selection of segments, those segments or combinations of segments which limit or avoid T cell epitopes in the final composite human V regions are selected. T cell epitopes are analysed by the methods described in method A, step (6) above using *in silico* or *in vitro* methods, preferably by use of *in silico* methods at the stage of designing composite human V region sequences.

For step (4), having designed a library of composite human sequences, composite VH and VL genes are then generated preferably using synthetic oligonucleotides. Typically, synthetic oligonucleotides encoding longer segments of V region sequence will ligated to a mixture of oligonucleotides which encode alternative segments of sequence to generate different members of the library of composite human V regions. Alternatively, each member of the library of composite human V regions will be generated separately using oligonucleotides encoding the sequence of the specific human V region. Alternatively, composite V regions can be assembled by other methods such as overlapping PCR or other amplification techniques using existing human VH and VL genes as templates or using one or more reference antibody V region genes as template.

Steps (5) and (6) for method B are as described in method A, steps (4) and (5).

Optional step (7) will be employed as in method A, step (6) where further avoidance of T cell epitopes is required in the lead composite human antibody(s). A final analysis using T cell assays for testing whole composite human antibody is preferred allowing for direct testing for T cell activation from the whole antibody.

It will be understood to those skilled in the art that, in addition to methods A and B, there will be other methods for creating and testing composite human antibodies and for optimising the properties of such antibodies. Composite human antibodies of the present invention are new and, as a result of the total human origin of the V regions, should be less immunogenic in humans than other antibodies containing non-human sequences. Additional optional features of composite human antibodies, namely the avoidance of T cell epitopes and/or the addition of Tr epitopes, may also contribute to lower immunogenicity. It will be understood by those skilled in the art that the object of lower immunogenicity may be achieved using less preferred composite antibodies containing V regions without all human sequence segments, for example composite human antibodies including segments at sequence positions in the composite antibody different from their sequence positions in the source human antibody, composite antibodies with only partial incorporation of segments of human V region sequence, composite antibodies with segments of non-human sequence, or composite antibodies with human sequence which has been mutated, for example to increase binding affinity to an antigen or to avoid a T cell eptiope.

It will be understood that V region sequence segments and their combinations within composite human antibodies might be selected to meet a range of criteria including the optional avoidance of T cell epitopes as above. For example, segments of human V region sequence and combinations thereof can be selected for avoidance of B cell epitopes and other epitopes such as MHC class I-restricted epitopes, for avoidance of amino acid sequences which might be deleterious to expression of composite antibodies, for avoidance of sequences which might direct inappropriate modification of composite antibodies such as N-glycosylation, for inclusion of certain functions such as inclusion of helper T cell epitopes and/or B cell epitopes (for example, in vaccine applications), for subsequent conjugation to other moieties such as one or more surface lysine residues, and for a range of other criteria.

It will also be understood by those skilled in the art that, in addition to human, composite antibodies with V region segments derived from other species either wholly or in part can be generated and should be considered within the scope of the invention. For example, for studies in mice, composite mouse antibodies can be generated comprising V region sequence segments wholly or partly of mouse origin.

The present invention also applies to proteins other than antibodies whereby, for therapeutic use, such proteins (herein termed "composite proteins") combine two or more segments of amino acid sequence from a human protein within the final protein molecule.

Thus, in a further aspect, the present invention provides a modified protein having improved immunogenicity through insertion of one or more segments of amino acid sequence.

In relation to proteins, the term "segments" refers to contiguous amino acid sequence found within a protein molecule, such segments ranging in size from 2 to 250 amino acids long. For therapeutic use, composite proteins of the present invention will typically combine two or more segments of amino acid sequence from different human proteins within the composite protein. In particular, the present invention relates to composite proteins with insertions composed entirely of segments of sequence from two or more human proteins. Where human proteins exist with homology to the composite protein or with homologous regions to regions of the composite protein, segments of human protein sequence at sequence positions in the composite protein sequences corresponding to their sequence positions in the source human protein may be used, for example amino acids 1 to 10 in the composite protein sequence will derive from amino acids 1 to 10 in a source human protein. Alternatively, segments of human protein sequence may be positioned in the composite protein at any sequence location in the composite protein irrespective of the sequence position in the source human protein. The source human proteins will be any existing human protein amino acid sequence, for example as provided in databases of human protein sequences, and may include sequences from naturally mutated or rearranged forms of the human protein and other variations differing from germ-line, sequences from artificially constructed human-derived proteins and sequences derived from human genes or RNA whether the corresponding proteins are expressed or not.

In a preferred embodiment of this aspect of the present invention, composite proteins for therapeutic use are constructed by combining or inserting human protein sequence segments in combinations which limit or avoid human T cell epitopes in the final composite protein. A preferred aspect of the invention as applied to composite proteins is to modify an existing reference protein such as a non-human protein by insertion of human protein sequence segments in order to limit or avoid T cell epitopes in the final composite protein.

In a preferred method of the present invention for generation of composite proteins, a library of composite human proteins is generated to include desirable amino acids from one or more reference proteins with desirable properties such as an absence of T cell epitopes. This method involves 7 steps as follows;
(1) sequence analysis of one or more reference proteins including optional analysis of T cell epitopes
(2) design of composite protein genes
(3) (optional) avoidance of T cell epitopes
(4) cloning of composite protein genes
(5) expression of composite protein genes
(6) screening and selection of composite proteins with desirable properties
(7) optimisation of lead composite proteins including optional avoidance of T cell epitopes

In step (1), typical reference proteins will be non-human with properties which are desirable in a composite protein. For therapeutic application, typically the reduction or elimination of immunogenicity in the composite protein will be an objective. Where one or more reference protein sequences are available, these are analysed to identify amino acids which might be important for the desirable properties of the protein. In addition, any known structure of the reference protein can be analysed or, alternatively, a structure modelled using modelling software. Where homologues of the reference protein are available, either *inter*species or i*ntra*species, these can be sometimes be used to determine relationships between sequence differences and differences in properties between homologues. Where the protein interacts with another molecule, models of this interaction can sometimes be generated and amino acids important for the interaction determined. As an optional addition to step 1, the sequence location of T cell epitopes in the reference protein are determined, in particular using *in vitro* human T cell assays as detailed for composite human antibodies above. Alternatively, *in silico* methods for analysing T cell epitopes can be used. Such analyses of the reference proteins are used to guide on segments of human protein sequence selected for the composite protein. For composite proteins where a reduction or elimination of immunogenicity compared to a reference protein, especially non-human, is the objective, commonly one or more human sequence segments corresponding to locations of T cell epitopes will be used in the composite protein in combination with segments of sequence from the reference protein from other locations without T cell epitopes.

For step (2), having determined amino acids which might be important for the desirable properties of the composite protein, segments of protein sequences are then selected to include some or all of these amino acids. A library of composite human protein sequences is thereby designed including selected segments with typically one or more alternative human protein segments at particular loci where the effect of such segments on properties of the composite protein is uncertain. Such composite protein sequences can be further analysed as with the reference protein by alignment with any homologues or by modelling of the structure of composite proteins or by other analyses in order to refine, as required, the combinations of human protein segments used in the composite human proteins to avoid defects in protein structure and incorrect structural orientations of important amino acids.

For the optional step (3), as an additional criteria or only criteria for selection of segments, those segments or combinations of segments which limit or avoid T cell epitopes in the final composite proteins are selected. T cell epitopes are analysed by the methods described for composite human antibodies above using *in silico* or *in vitro* methods.

For step (4), having designed a library of composite proteins, composite protein genes are then generated preferably using synthetic oligonucleotides. Typically, synthetic oligonucleotides encoding longer segments of protein sequence will ligated to a mixture of oligonucleotides which encode alternative segments of sequence to generate different members of the library of composite proteins. Alternatively, each member of the library of composite proteins will be generated separately using oligonucleotides encoding the sequence of the specific composite protein. Alternatively, composite proteins can be assembled by other methods such as overlapping PCR or other amplification techniques using existing human protein genes as templates or using one or more reference protein genes as template.

For step (5), screening of libraries of composite proteins is usually for one or more desirable properties of the composite protein. There are many screening methods known to those skilled in the art, the selection of which will depend on the form of expression of the composite proteins and the protein function. Screening methods will range from immobilising individual members of the library or pools of such members on a solid phase, to screening member of the library in solution phase, to immobilising another molecule with which the composite protein is designed to interact by binding either individually or in pools. Screening methods may also include functional or biological tests where a functional or biological activity is measured such as *in vitro* tests involving cell growth, cell growth inhibition, cell differentiation or cell migration, or alternative *in vivo* tests involving measuring responses to the composite protein at the level of the whole organism, for example changes in blood cell counts in a mouse or growth inhibition of a transplanted tumour.

For step (6), following selection of one or more "lead" composite proteins with desirable properties, optionally the properties of the lead protein may be improved, for example by increasing the specific activity of an enzyme or by increasing the binding of a protein ligand to a receptor. An improvement in properties may be achieved by mutagenesis of composite protein sequences in order to select for mutations which alter properties of the composite protein in a desirable way. The present invention includes novel methods for mutagenesis of a protein sequence by replacing one or more individual protein sequence segments from the protein with sequence segments from one or more human protein sequences. One or more composite proteins subject to such mutagenesis can then be screened for improved properties.

For the optional step (7), following selection of a lead composite protein, T cell epitopes are limited or avoided by, where required, exchanging protein sequence segments contributing to or encoding a T cell epitope with alternative segments which avoid T cell epitopes. Such T cell epitopes can be detected by a range of methods. For example, peptides corresponding to one or more loci in the composite protein can be synthesised and tested in T cell assays to determine the presence of T cell epitopes. Typically such peptides will be 15 amino acids in length and, where it is desirable to test a longer contiguous sequence, overlapping peptides from the sequence such as 15mers with 12 amino acid overlaps are used. Alternatively, overlapping peptides corresponding to the composite protein sequences are analysed for binding to human MHC class II molecules either using *in vitro* methods or *in silico* methods, in each case to determine potential T cell epitopes i.e. MHC binding peptides which may induce a T cell response. *In silico* methods will include methods involving modelling of peptide-MHC class II binding interactions, methods involving identification of motifs common for binding to MHC class II and methods using databases of peptides or specific amino acids within peptides with known in vitro MHC binding properties. Other methods can be used such as producing longer peptides from composite protein sequences or whole composite proteins and testing these in T cell assays or in MHC binding assays on antigen presenting cells. Avoidance of T cell epitopes in composite proteins can also be assisted by avoidance of MHC class II binding motifs or avoidance of particular amino acids which anchor the binding of peptides to MHC class II. In the preferred method for avoidance of T cell epitopes from one or more lead composite proteins, *in silico* methods are initially applied to analyse the composite protein for potential T cell epitopes and, where these are determined, new segments of human protein sequence are introduced to avoid these epitopes and to avoid introduction of new T cell epitopes. Following such introduction of new human segments if required to avoid T cell epitopes and rescreening for modified lead composite proteins for desirable properties, one or more final lead composite proteins can optionally tested in human T cell assays either by testing overlapping peptides typically of 15 to 45 amino acids in length, for example 15mer peptides with 12 amino acid overlaps from the composite protein sequences (whole proteins or parts thereof) or by testing whole composite proteins directly in human T cell assays. A final analysis using T cell assays for testing whole composite protein is preferred allowing for direct testing for T cell activation from the whole protein.

It will be understood to those skilled in the art that there will be other methods for creating and testing composite proteins and for optimising the properties of such proteins. Composite proteins of the present invention are new and, where used for therapeutic purposes, the human origin of some or all protein sequence segments should render the composite protein less immunogenic in humans than other comparable or non-human reference proteins containing non-human sequences. Additional optional features of composite proteins, namely the avoidance of T cell epitopes and/or the addition of Tr epitopes, may also contribute to lower immunogenicity. It will be understood by those skilled in the art that the object of lower immunogenicity may be achieved using composite proteins without all human sequence segments and may also include composite proteins with human sequence segments which have been mutated to eliminate a T cell eptiope or segments of non-human protein homologous to the reference protein. It will be understood that protein segments and their combinations within composite proteins might be selected to meet a range of criteria including the optional avoidance of T cell epitopes. For example, segments of human protein sequence and combinations thereof can be selected for avoidance of B cell epitopes and other epitopes such as MHC class I-restricted epitopes, for avoidance of amino acid sequences which might be deleterious to expression of composite proteins, for avoidance of sequences which might direct inappropriate modification of composite proteins such as N-glycosylation, for inclusion of certain functions such as inclusion of helper T cell epitopes and/or B cell epitopes (for example, in vaccine applications), for subsequent conjugation to other moieties, and for a range of other criteria.

It will also be understood by those skilled in the art that, in addition to human, composite proteins with sequence segments derived from other species either wholly or in part can be generated and should be considered within the scope of the invention. For example, for studies in mice, composite proteins including mouse protein sequence segments can be generated. It will also be understood that composite proteins can include protein sequence segments from one species combined with other protein sequence segments from homologous proteins within the same species. For example, the invention will include construction of plant type I RIPs (ribosome inhibitory proteins) where a RIP is assembled using sequence segments from the numerous plant type I RIP sequences available. Such composite RIPs would be assembled by introducing combinations of sequence segments which would retain RIP activity and, if for use in humans, would include avoidance of human T cell epitopes in the final composite sequence.

As in the case of antibodies, the invention includes the option of further modifications to the composite protein sequences by random, semi-random or directed mutagenesis of the composite protein to achieve further improvement in one or more other properties of the final protein. It will be understood that the invention is particularly suitable to producing proteins with low immunogenicity when used in humans or used by humans such as proteins for pharmaceutical use, or proteins for use in food, detergents, cosmetics and other consumer items where allergic responses are limited or eliminated by use of compositions of the present invention. It will be understood that the invention is particularly suitable to producing proteins with low allergenicity in humans especially by producing proteins with allergy associated T cell epitopes removed or replaced by non-allergy associated epitopes (e.g. TH2 for TH1 T cell-inducing epitopes) and/or by addition of Tr epitopes to suppress immune responses in allergic individuals. It will be understood that the invention is particularly suitable to producing proteins with reduced inflammatory properties in humans especially by producing proteins with inflammation associated T cell epitopes removed or replaced by non-inflammation associated epitopes (e.g. TH1 for TH2 T cell-inducing epitopes) and/or by addition of Tr epitopes to suppress inflammatory responses.

As discussed herein, the modified/composite proteins and antibodies of the invention are useful in treating disease and exhibit less immunogenicity. Thus, in yet a further aspect, the present invention provides a pharmaceutical formulation comprising a modified antibody, antigen-binding fragment or protein as defined in any one of claims 1 to 18, optionally together with one or more pharmaceutically acceptable excipients, carriers or diluents.

The compositions of the invention may be presented in unit dose forms containing a predetermined amount of each active ingredient per dose. Such a unit may be adapted to provide 5-100mg/day of the compound, preferably either 5-15mg/day, 10-30mg/day, 25-50mg/day 40-80mg/day or 60-100mg/day. For compounds of formula I, doses in the range 100-1000mg/day are provided, preferably either 100-400mg/day, 300-600mg/day or 500-1000mg/day. Such doses can be provided in a single dose or as a number of discrete doses. The ultimate dose will of course depend on the condition being treated, the route of administration and the age, weight and condition of the patient and will be at the doctor's discretion.

The compositions of the invention may be adapted for administration by any appropriate route, for example by the oral (including buccal or sublingual), rectal, nasal, topical (including buccal, sublingual or transdermal), vaginal or parenteral (including subcutaneous, intramuscular, intravenous or intradermal) route. Such formulations may be prepared by any method known in the art of pharmacy, for example by bringing into association the active ingredient with the carrier(s) or excipient(s).

Pharmaceutical formulations adapted for oral administration may be presented as discrete units such as capsules or tablets; powders or granules; solutions or suspensions in aqueous or non-aqueous liquids; edible foams or whips; or oil-in-water liquid emulsions or water-in-oil liquid emulsions.

Pharmaceutical formulations adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research, 3(6), 318 (1986).

Pharmaceutical formulations adapted for topical administration may be formulated as ointments, creams, suspensions, lotions, powders, solutions, pastes, gels, sprays, aerosols or oils.

For applications to the eye or other external tissues, for example the mouth and skin, the formulations are preferably applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in-oil base.

Pharmaceutical formulations adapted for topical administration to the eye include eye drops wherein the active ingredient is dissolved or suspended in a suitable carrier, especially an aqueous solvent.

Pharmaceutical formulations adapted for topical administration in the mouth include lozenges, pastilles and mouth washes.

Pharmaceutical formulations adapted for rectal administration may be presented as suppositories or enemas.

Pharmaceutical formulations adapted for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable formulations wherein the carrier is a liquid, for administration as a nasal spray or as nasal drops, include aqueous or oil solutions of the active ingredient.

Pharmaceutical formulations adapted for administration by inhalation include fine particle dusts or mists which may be generated by means of various types of metered dose pressurised aerosols, nebulizers or insufflators.

Pharmaceutical formulations adapted for vaginal administration may be presented as pessaries, tampons, creams, gels, pastes, foams or spray formulations.

Pharmaceutical formulations adapted for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets.

Preferred unit dosage formulations are those containing a daily dose or sub-dose, as herein above recited, or an appropriate fraction thereof, of an active ingredient.

It should be understood that in addition to the ingredients particularly mentioned above, the formulations may also include other agents conventional in the art having regard to the type of formulation in question, for example those suitable for oral administration may include flavouring agents.

The following examples should not be considered limiting for the scope of the invention. The figures and tables relate to the examples below and are as follows;
Figure 1/2 - Sequence of VH (figure 1) and VL (figure 2) genes used for Composite Human anti-HER2 antibody
Figure 3 - Inhibition of proliferation of human SK-BR-3 cells after 8 days incubation with chimeric 4D5 IgG1/kappa, Composite Human anti-HER2 antibody and epitope avoided anti-HER2 "EACHAB" with chimeric anti-IgE control (see example 4)
Figures 4/5 - Sequence of VH (figure 4) and VL (figure 5) genes used for Composite Human anti-Lewis Y antibody
Figures 6/7 - Sequence of VH (figure 6) and VL (figure 7) genes used for Composite Human anti-human IgE antibody
Figure 8 - Sequence of VH and VL genes used for Composite Mouse anti-human TNFα antibody including avoidance of human T cell epitopes
Figure 9 - ELISA for binding to human TNFα by Composite Mouse and chimeric anti-human TNFα antibody
Figure 10 - V region sequences of anti-TNFα antibody A2
Figure 11 - Sequences of composite human anti-TNFα VH variants
Figure 12 - Sequences of composite human anti-TNFα VL variants
Figures 13/14 - Oligonucleotides for construction of chimeric mouse:human anti-TNFα VH (figure 13) and VL (figure 14)
Figures 15/16 - Oligonucleotides for construction of primary composite human anti-TNFα VH (figure 15; corresponding to SEQ ID No. 3 figure 11) and VL (figure 16; corresponding to SEQ ID No. 4 figure 12)
Figures 17- Oligonucleotides for construction of secondary composite human anti-TNFα VH and VL variants
Figure 18 - WEHI-164 protection Assay for composite human anti-TNFα antibodies
Figure 19 - Time-Course human T cell assay of lead composite human anti-TNFα
Figure 20 - Activity of composite bouganin molecules with inserted human sequence segments

Tables 1-3 - CDRs used in Composite Human antibody scFv library comprising 186 x 9 residue-long VH CDR3s (table 1), 77 x 8 residue-long VL CDR3s (table 2), and 153 x 10 residue-long VL CDR3s (table 3).
Table 4 - Human sequence segments used for primary composite human anti-TNFα VH and VL variants
Table 5 - Activity of composite human anti-TNFα variants
Table 6 - Immunogenic peptide sequences of bouganin and replacement human segments in bouganin variants

### Example 1- Construction of Composite Human Anti-HER2 Antibody

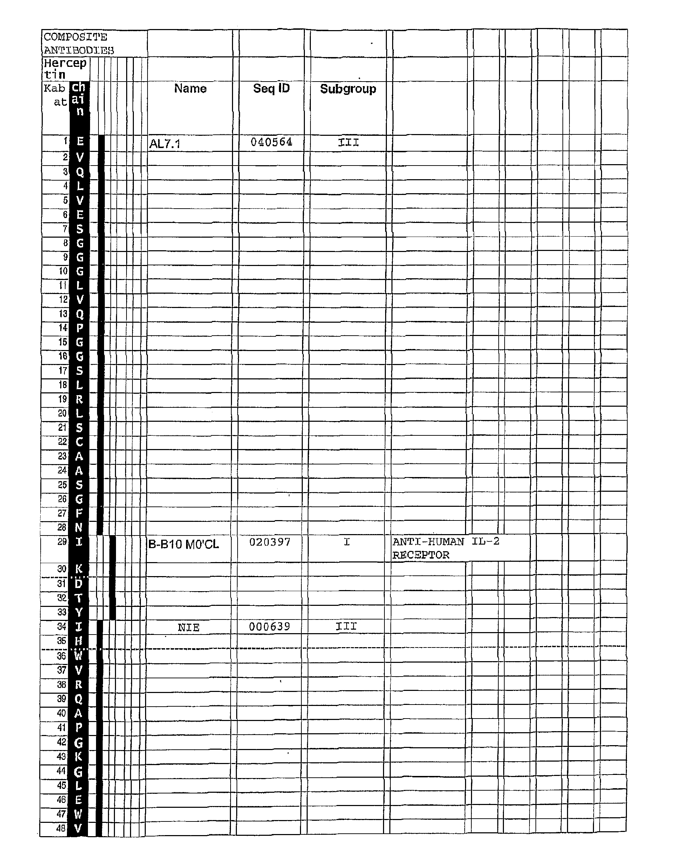

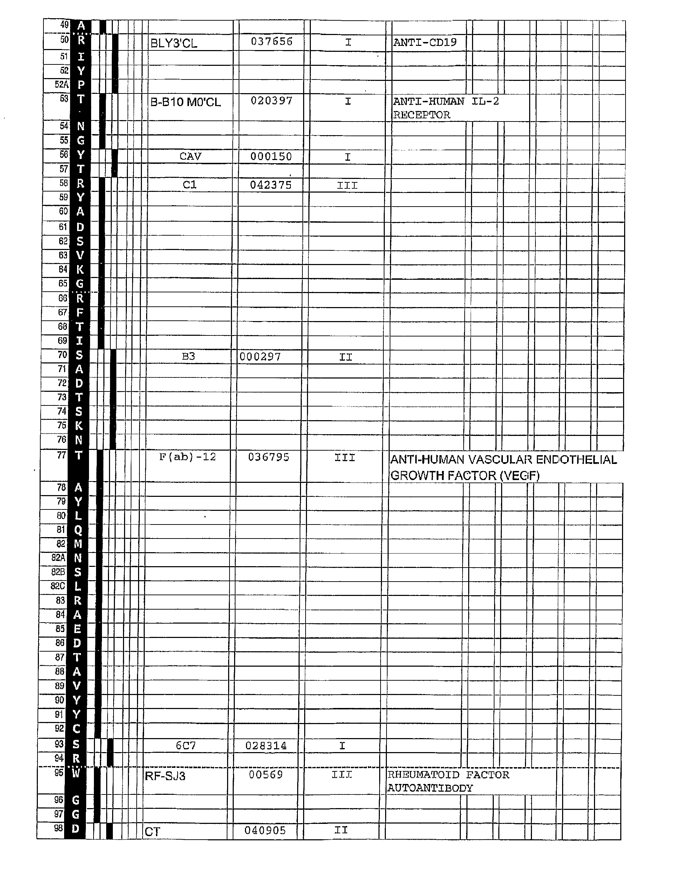

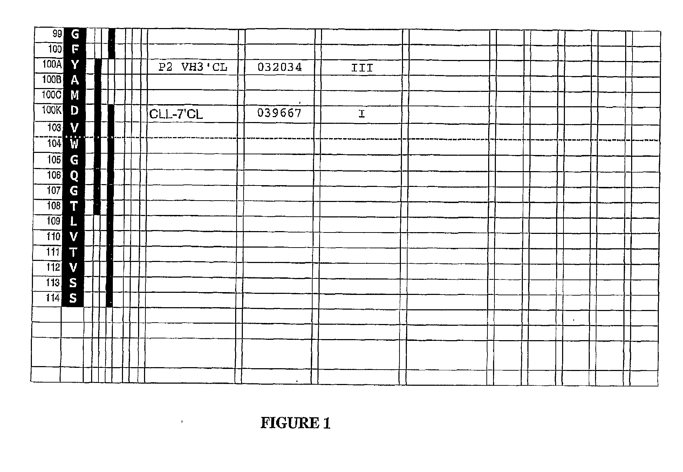

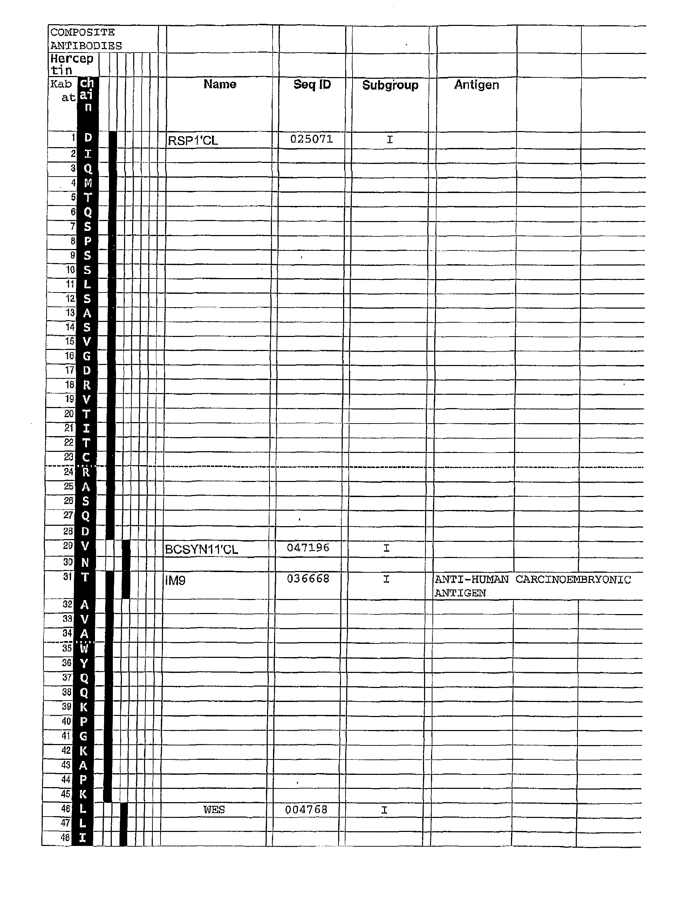

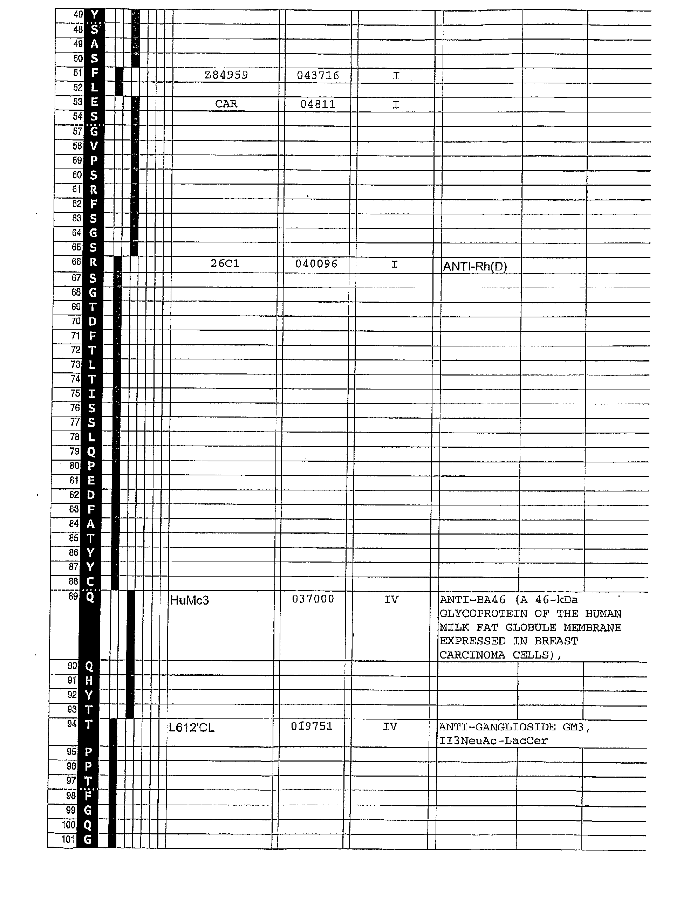

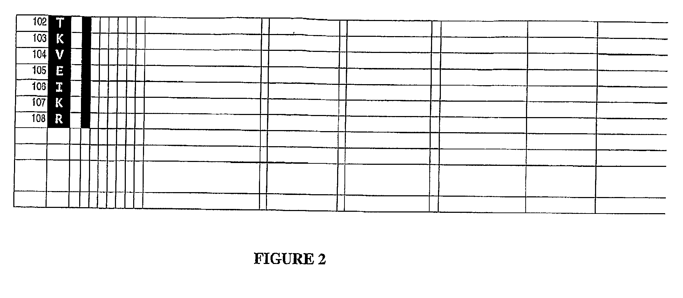

For creation of a human variable region sequence segment library, amino acid sequences from a range of human immunoglobulins were collected into a single database comprising the *in silico* human variable region sequence library including heavy (VH) and light (VL) chain variable region sequences. Sources of sequences included the NCBI Igblast database (www.ncbi.nih.gov), Kabat databases (Kabat et al., Sequences of Proteins of Immunological Interest, NIH publication 91-3242, 5th ed. (1991) (and later updates)), Vbase (www.mrc-cpe.cam.ac.uk/imt.doc), Genbank (Benson et al., Nucl. Acids Res. 25 (1997) p1-6 or via www.bioinf.org.uk/abs) databases. The reference antibody variable region sequences used was a humanised anti-HER2 antibody known as Herceptin® (Carter et al., Proc. Nat. Acad. Sci. USA, vol 89 (1992) p4285, US5821337). Segments from the *in silico* human variable region sequence library were selected for identity to the corresponding amino acids in the Herceptin® variable region sequence and combined to produce the composite human VH and VL sequences as shown in figures 1 and 2 respectively.

Recombinant DNA techniques were performed using methods well known in the art and, as appropriate, supplier instructions for use of enzymes used in these methods. Sources of general methods included Molecular Cloning, A Laboratory Manual, 3rd edition, vols 1-3, eds. Sambrook and Russel (2001) Cold Spring Harbor Laboratory Press, and Current Protocols in Molecular Biology, ed. Ausubel, John Wiley and Sons. Detailed laboratory methods are also described in example 7 below. Composite human VH and VL sequences corresponding to Herceptin® were created using, for each chain, eight synthetic oligonucleotides of 30-60 amino acids in length encoding the entire composite human VH and VL sequences. In parallel, as a control reagent, a chimeric form of the mouse monoclonal antibody 4D5 (Hudziak et al., Mol. Cell. Biol., (March 1989) p1165-1172)), was also created using eight synthetic oligonucleotides per chain. Separate VH and VL oligonucleotides were first phosphorylated, mixed at equal molar ratios, heated to 94oC for 5 min in a thermal cycles followed by cooling to 65oC and incubation at 65oC for 2 min. Incubations were then continued at 45oC for 2 min., 35oC for 2 min., 25°C for 2 min and 4oC for 30 min. Oligonucleotides were then ligated using T4 DNA ligase (Life Technologies, Paisley UK) at 14oC for 18 hours.

To each of the VH and VL oligonucleotide mixtures, additional oligonucleotides encoding a 5' flanking sequence, including a Kozak sequence, the leader signal peptide sequence and the leader intron, and 3' flanking sequence, including the splice site and intron sequence, were added and annealed as above. The Composite Human V_{H} and V_{K} and the 4D5 expression cassettes produced were cloned as HindIII to BamHI fragments into the plasmid vector pUC19 and the entire DNA sequence was confirmed. These were transferred to the expression vectors pSVgpt and pSVhyg which include human IgG1 (V_{H}) or Kappa (V_{K}) constant regions respectively and markers for selection in mammalian cells. The DNA sequence was confirmed to be correct for the Composite Human V_{H} and V_{K} and 4D5 V_{H} and V_{K} in the expression vectors.

The host cell line for antibody expression was NS0, a non-immunoglobulin producing mouse myeloma, obtained from the European Collection of Animal Cell Cultures, Portion, UK (ECACC No 85110503). The heavy and light chain expression vectors were co-transfected into NS0 cells by electroporation. Colonies expressing the gpt gene were selected in Dulbecco's Modified Eagle's Medium (DMEM) supplemented with 10% foetal bovine serum, 0.8 µg/ml mycophenolic acid and 250 µg/ml xanthine. Transfected cell clones were screened for production of human antibody by ELISA for human IgG. Cell lines secreting antibody were expanded and the highest producers selected and frozen down in liquid nitrogen. The modified antibodies were purified using Prosep®-A (Bioprocessing Ltd, Northumberland, UK). The concentration was determined by ELISA for human IgGκ antibody.

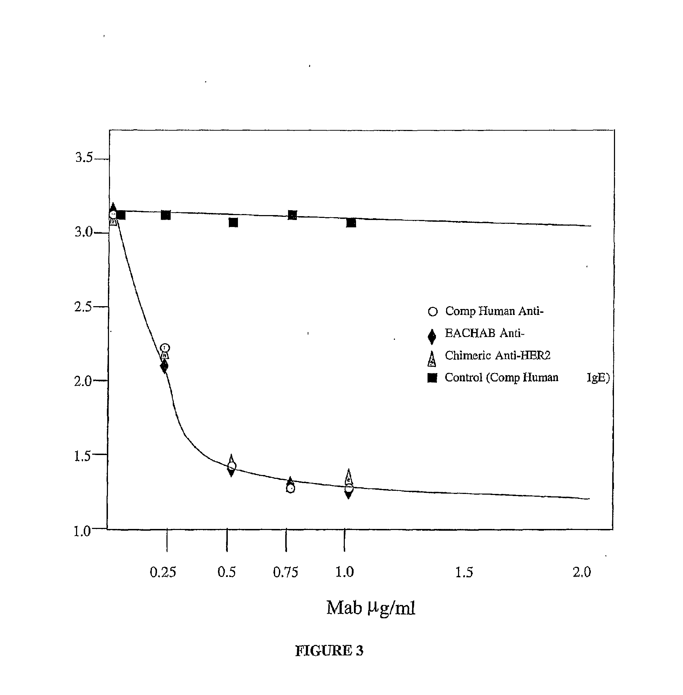

The Composite Human antibody and chimeric 4D5 antibodies were tested for inhibition of proliferation of the HER2+ human breast tumour cell line SK-BR-3 in conjunction with a negative control non-Her-2 binding human IgG1 / Kappa antibody exactly as described by Hudziak et al. (ibid). The results (figure 3) show that Composite Human antibody and the chimeric 4D5 antibodies have equivalent potency in inhibiting growth of SK-BR-3 cells. Figure 3 also shows data for an alternative "epitope avoided" Composite Human antibody produced as below.

In order to test the epitope avoidance option in the invention, the sequences of the Composite Human heavy and light chain variable regions were analysed for non-self human MHC class II binders using Peptide Threading (www.csd.abdn.ac.uk/~gjlk/MHC-thread). This software predicts favourable interactions between amino acid side chains of the peptide and specific binding pockets within the MHC class II binding groove. All overlapping 13mers from the Composite Human heavy and light chain variable sequences were threaded through a database of MHC class II allotypes and scored based on their fit and interactions with the MHC class II molecules. Peptides predicted to bind MHC class II were 13mers beginning at residues 16 and 67 in VH, and 9 and 44 in VL. As a result, new segments of the human variable region sequence library were chosen instead of those used in the Composite Human sequences of figure 1 in order to introduce the amino acid changes VH 18L-A / 69I-G; VL 11L-A, 46L-A. A corresponding "epitope avoided" Composite Human antibody ("EACHAB" = Epitope Avoided Composite Human AntiBody) was made by substituting some of the oligonucleotides used to make the antibody corresponding to the sequence in figure 1 and the EACHAB was made as in the method described above and tested to show inhibition of SK-BR-5 proliferation equivalent to the standard Composite Human antibody (figure 3). This data shows that Composite Human antibodies can be successfully constructed with equal potency to a control chimeric anti-Her-2 antibody and that an EACHAB version of the Composite Human antibody can be generated without loss of potency.

### Example 2 - Immunogenicity of Composite Human Anti-HER2 Antibody

T cell proliferation assays were carried out to compare the immunogenicity of the Composite Human anti-HER2 antibody, the EACHAB variant and the chimaeric 4D5 antibody (see example 1). These antibodies were prepared from NS0 cells grown in serum-free, animal derived component-free, protein-free medium, HyClone HyQ®ADCF-Mab™ (Hyclone Cat No: Cat no: SH30349) supplemented with HyQ®LS1000 Lipid Supplement (Hyclone Cat No: SH30554) and sodium pyruvate (Gibco Cat No: 11360-039). After buffer exchange into 50mM MES pH6 on a Sephadex G25 (PD10 column), the antibodies were each passed through a cation exchange column (Mono-S 10/10) and eluted with a sodium chloride gradient (0 to 0.5M). The antibody containing fractions were then applied to a Superdex 200 preparative column (XK16/60) run in PBS. Peak fractions were pooled and stored at 4°C. The antibody concentrations were determined by ELISA for human IgG.

Immunogenicity analysis was performed using PBMCs (peripheral blood mononuclear cells) that were isolated from healthy human donor blood and cryopreserved in liquid nitrogen. Each donor was tissue-typed using an Allset^{™} PCR based tissue-typing kit (Dynal, Wirral, UK) and 20 healthy donors were selected according to individual MHC haplotypes. 2ml bulk cultures containing 4x10⁶ PBMC in AIM V (Invitrogen, Paisley, UK) were incubated in a 24 well tissue-culture plate with test peptides (5µM final concentration) and proliferation was assessed on days 5, 6, 7, and 8 by gently resuspending the bulk cultures and transferring triplicate 100µl samples of PBMC to a U-bottomed 96 well plate. Cultures were harvested onto glass fibre filter mats using a Tomtec Mach III plate harvester (Receptor Technologies, UK) and counts per minute (cpm) values determined by scintillation counting using a Wallac Microbeta TriLux plate reader (using a paralux high efficiency counting protocol). For each test antibody, the stimulation index (SI) was calculated as the ratio of counts per minute (cpm) of the test antibody : cpm of the negative control with SI>2 considered a significant T cell epitope response. The results showed that the chimaeric 4D5 antibody induced significant proliferative responses on at least one of the four days of proliferation tested (SI greater than 2) in five of twenty healthy donors tested (25%), the Composite Human anti-HER2 antibody induced SI>2 in three of twenty donors (15%) whilst the EACHAB anti-HER2 antibody induced SI>2 in none of twenty donors (0%). These results indicated an order of immunogenicity of chimeric 4D5> Composite Human anti-HER2>EACHAB anti-HER2 with the latter showing no evidence of immunogenicity in any donor blood sample tested.

### Example 3 - Construction of Composite Human Anti-Lewis Y Antibody

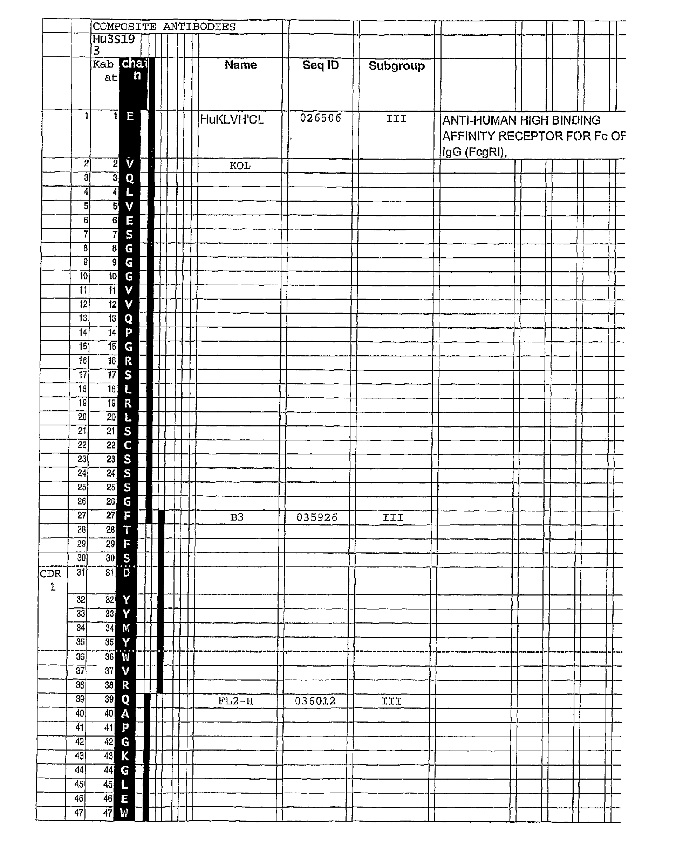

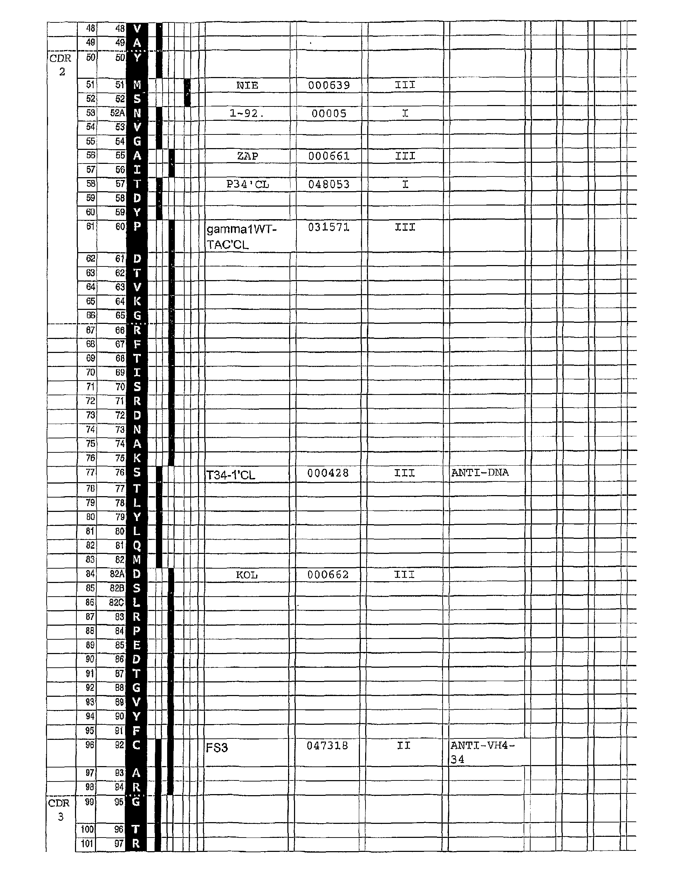

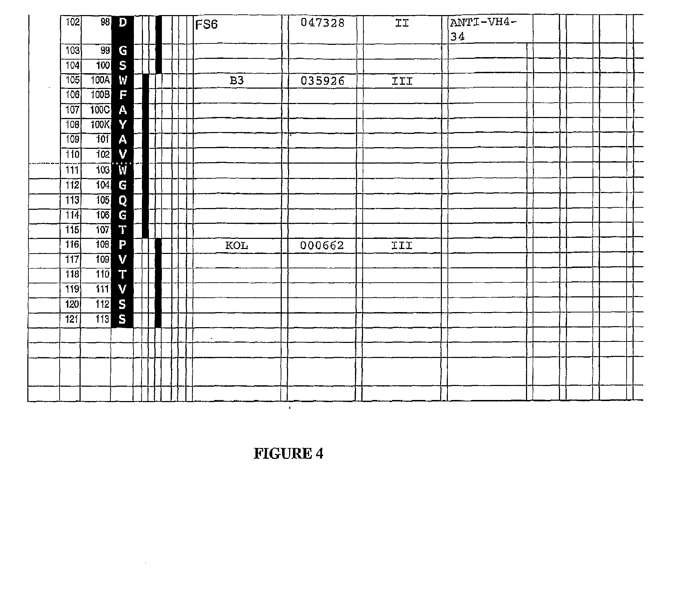

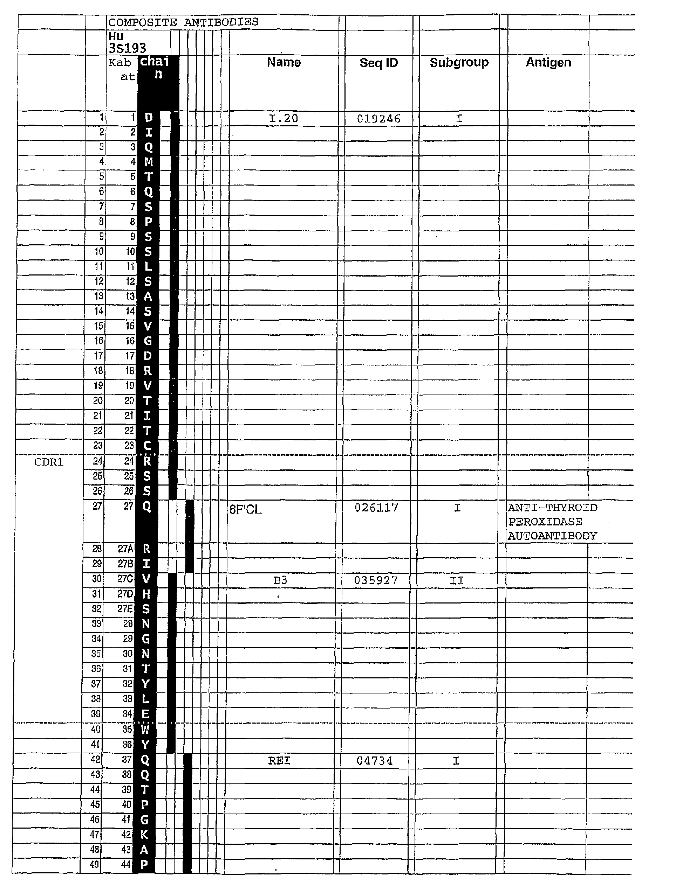

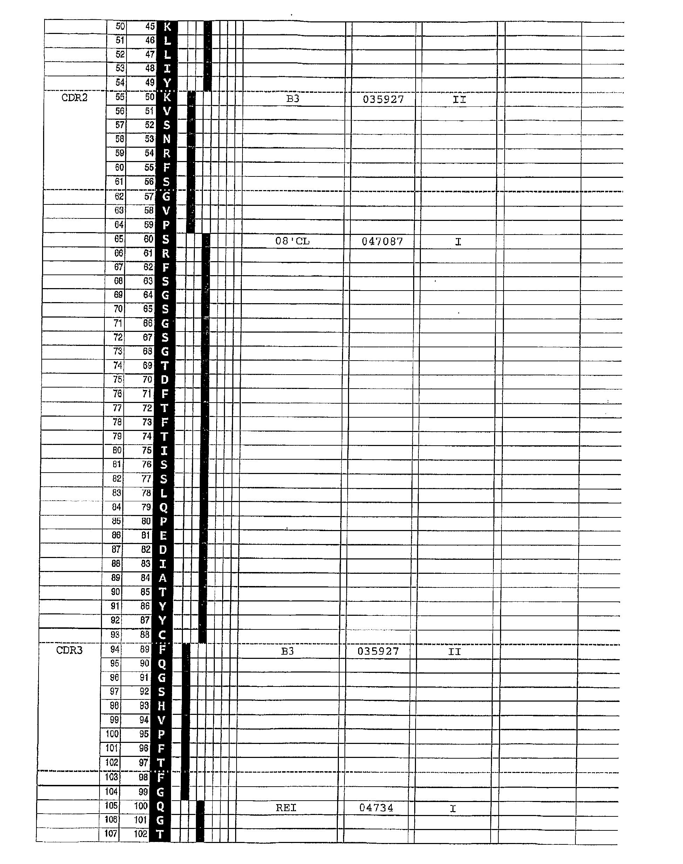

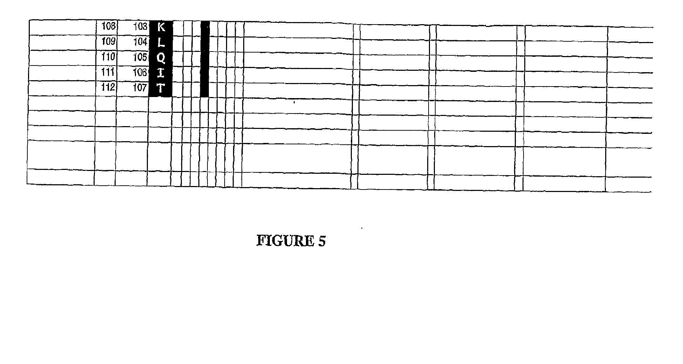

A Composite Human antibody specific for sialylated Lewis Y antigen was constructed as described in example 1 using, as the reference antibody variable region sequences, the humanised 3S193 antibody (Scott et al.; Cancer Res., 60 (2000) p3254-3261, US5874060). Segments from the in silico human variable region sequence library were selected for identity to the corresponding amino acids in the humanised 3S 193 variable region sequence and combined to produce the Composite Human VH and VL sequences as shown in figures 4 and 5 respectively. In parallel, a reference chimeric anti-Lewis Y antibody was made from the reference V region sequences. Human IgG1 (V_{H}) and Kappa (V_{K}) constant regions were used on both the Composite Human anti-Lewis Y antibody and the chimeric reference antibody and antibodies were tested by ELISA against synthetic Lewis Y-HSA conjugate as described in US5874060. The data showed a minimum concentration of 0.1ug/ml chimeric antibody to give a binding signal in the assay compared to 0.15ug/ml Composite Human antibody which is consistent with the data of US5874060.

### Example 4 - Construction of Composite Human Anti-IgE Antibody

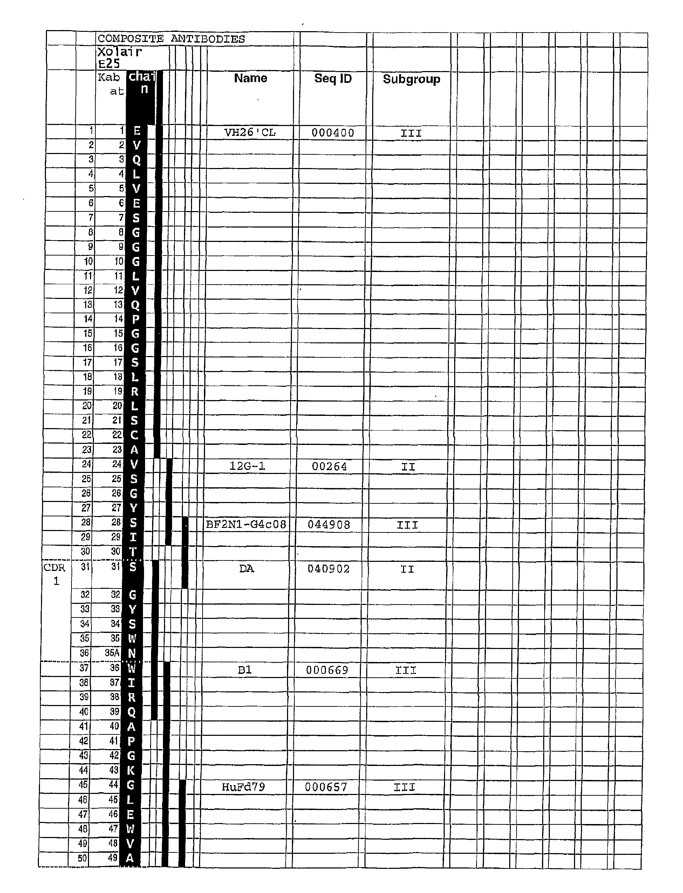

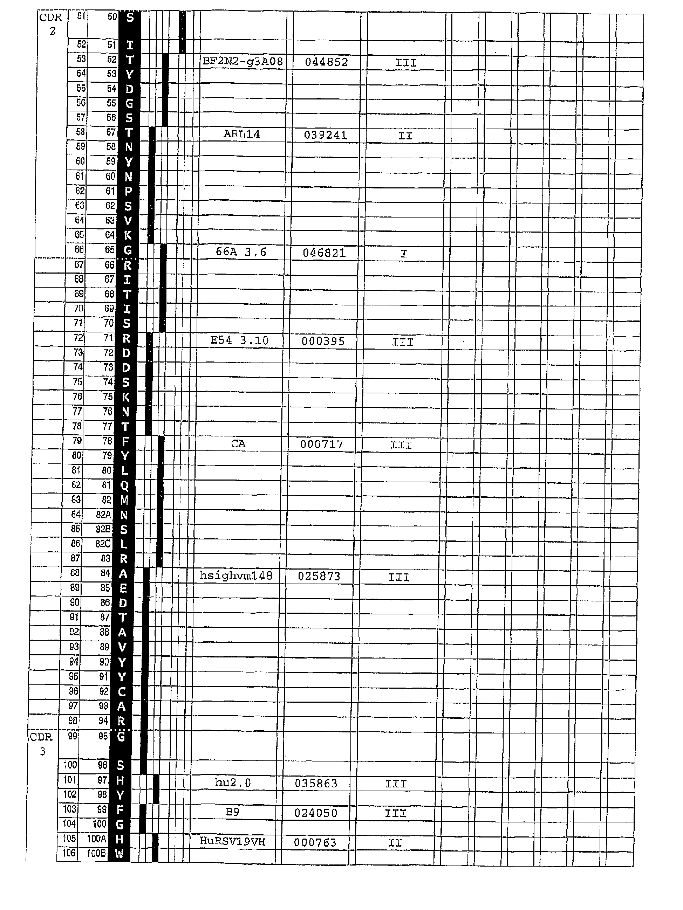

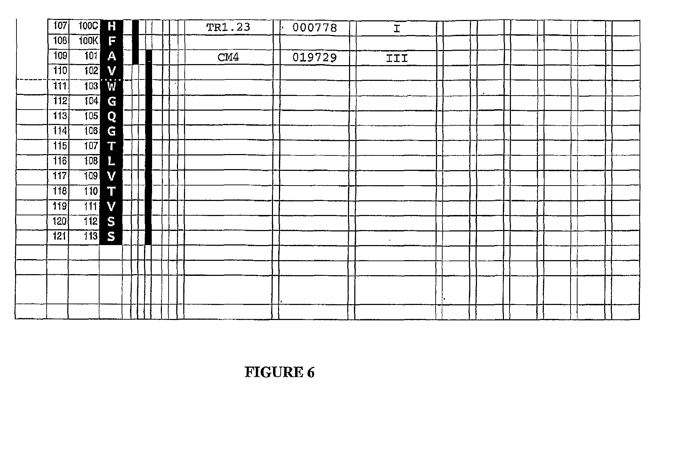

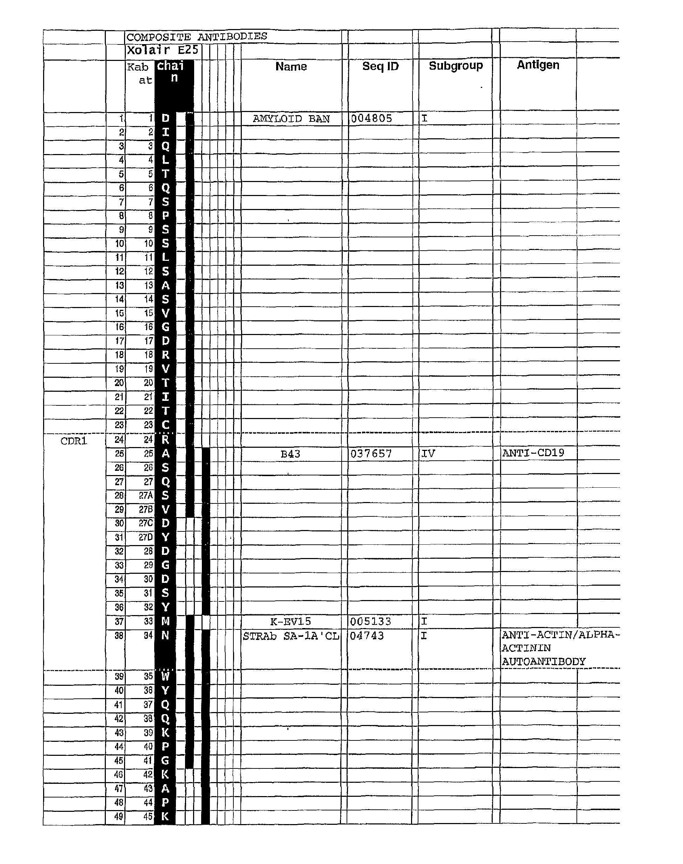

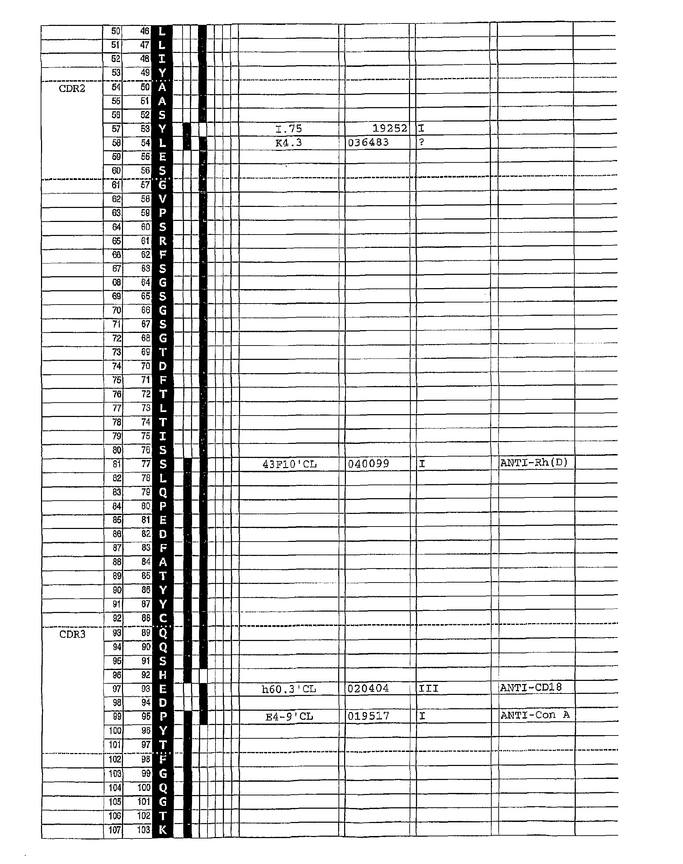

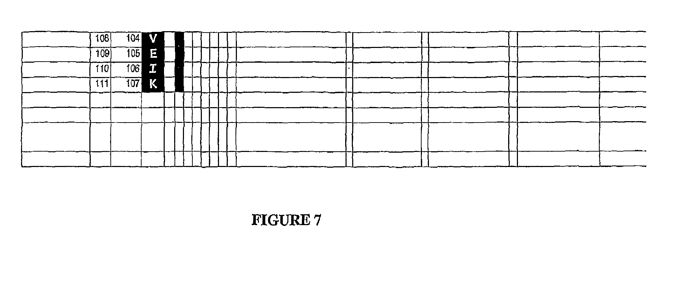

A Composite Human Anti-IgE antibody was constructed as described in example 1 using, as the reference antibody variable region sequences, the humanised anti-IgE antibody known as Xolair® (Presta et al., J. Immunol., 151(5) (1993) p2623-2632). Segments from the in silico human variable region sequence library were selected for identity to the corresponding amino acids in the Xolair® variable region sequence and combined to produce the Composite Human VH and VL sequences as shown in figures 6 and 7 respectively. In parallel, a reference chimeric anti-IgE antibody was made from the reference V region sequences. Human IgG1 (V_{H}) and Kappa (V_{K}) constant regions were used on both the Composite Human Anti-IgE antibody and the chimeric reference antibody.

The specificity of the Fabs was further characterized by surface plasmon resonance (BIAcore 2000, Biacore AB, Uppsala, Sweden). Recombinant human IgE Fab was produced as decscribed by Flicker et al., J. Immunol., 165 (2000) p3849-3859. Test antibodies were purified and immobilized onto flow cells of a CM chip using a NHS/EDC kit (Biacore) to obtain 2010 RU for chimeric anti-IgE and 2029 RU for Composite Human anti-IgE. 10 and 25nM recombinant human IgE Fab in Hepes-buffered saline (10 mM Hepes, 3.4 mM EDTA, 150 mM NaCl, 0.05% (v/v) surfactant P20, pH 7.4) was passed over the test antibodies at a flow rate of 5 µl/min for 10 minutes. The results showed that for both 10 and 25nM IgE Fab, an equivalent SPR (surface plasmon resonance) curve was detected for the chimeric anti-IgE and the Composite Human anti-IgE antibodies showing that the latter had successfully achieved binding efficiency equivalent to the reference anti-IgE antibody.

### Example 5 - Generation and Screening of Composite Human scFv Libraries

The strategy for initial construction of the human scFv (single-chain Fv) library was to construct seven consensus human VH and four consensus human VL (kappa) genes as detailed in Knappik et al., J. Mol. Biol., 296 (2000) 57-86 and to clone into these a large number of VH and VL CDR3 segments from databases of human variable regions. This list of CDR3s is shown in table 1 for VH CDR3s, table 2 for VL CDR3s of 8 amino acids and table 3 for VL CDR3s of 10 amino acids. For the master VH and VL construction, 6 overlapping synthetic oligonucleotides encoding VH and VL up to the end of framework 3 were synthesised as detailed by Knappik et al., ibid, and subjected to recursive PCR (Prodromou and Pearl, Protein Engineering, 5 (1992) 827-829). These were ligated into EcoRV digested pZero-1 vector (Invitrogen, Paisley, UK). For addition of CH1 and C kappa, both initially with 4D5 CDR3s (Carter et al, Bio/Technology, 10 (1992) 163-167), the protocol of Knappik et al., ibid, was followed except that the VH-CH1 SapI-EcoRI and VL-C kappa NsiI-SphI fragments were both blunt-end cloned into EcoRV digested pZero-1.

**Table 1**

| # Name | H3 | Length-H3 | Subgroup (H ) |
|---|---|---|---|
| MUC1-1'CL | DFLSGYLDY | 9 | I |
| ALL1-1'CL | VRGSGSFDY | 9 | III |
| ALL7-1'CL | DRGGNYFDY | 9 | III |
| L36'CL | MYNWNFFDY | 9 | I |
| 5.M13'CL | AGLGMIFDY | 9 | I |
| Au2.1'CL | RGFNGQLIF | 9 | I |
| M71'CL | ALTGDAFDI | 9 | II |
| VH6.N1'CL | TKLDWYFDL | 9 | II |
| E55 6.X'CL | RYGGFYFDY | 9 | II |
| E55 6.11'CL | GYSNEGMDV | 9 | II |
| VH6.A5'CL | SWDGYSYIY | 9 | II |
| VH6-EX8'CL | QMGAEYFQH | 9 | III |
| E54 4.2'CL | DMSLDAFDI | 9 | II |
| RF-SJ4'CL | GSVGATLGE | 9 | II |
| 3.A290'CL | YGDYHYFDY | 9 | III |
| A95 | GVGSSGWDH | 9 | III |
| 60P2'CL | KGSLYYFDY | 9 | III |
| E55 3.6'CL | PNWNDAFDI | 9 | III |
| E55 3.16'CL | RGIPHAFDI | 9 | III |
| 333'CL | PPEVESLRS | 9 | III |
| 1H1'CL | PPEVESLRS | 9 | III |
| 126'CL | PPEVESLRS | 9 | III |
| 1B11'CL | PPEVESLRS | 9 | III |
| 115'CL | PPEVESLRS | 9 | III |
| 112'CL | PPEVESLRS | 9 | III |
| 2C12'CL | PPEVESLRS | 9 | III |
| 2A12'CL | PPEVESLRS | 9 | III |
| BUT | DLAAARLF? | 9 | III |
| KOL-based | QGTIAGIRH | 9 | III |
| resh. | | | |
| CAMPATH-9 | | | |
| L2E8'CL | EDYYYGMDV | 9 | III |
| s5D4'CL | DPINWYFDL | 9 | III |
| ss4'CL | DRAAGDRDY | 9 | III |
| P2-57'CL | HQMYSNSDY | 9 | I |
| HuHMFG1'CL | SYDFAWFAY | 9 | I |
| NEW-based | QGTIAGIRH | 9 | II |
| resh. | | | |
| CAMPATH-9 | | | |
| TR1.10'CL | VLGIIAADH | 9 | I |
| L3B2'CL | DLTGDAFDI | 9 | I |
| DAW | SCGSQYFDY | 9 | II |
| ss7'CL | LWNWDAFDI | 9 | I |
| ss6'CL | DIMTWGFDY | 9 | I |
| s5A9'CL | SNWYWYFDL | 9 | III |
| NEWM | NLIAGCIDV | 9 | II |
| L2A12'CL | GGKGGEFDD | 9 | I |
| B5G10H'CL | DSGNYRIDY | 9 | II |
| E55 3.9'CL | DPRLDAFDI | 9 | III |
| SpA1-29'CL | GYSYPVWGR | 9 | III |
| AM28'CL | LVGNSWLDY | 9 | III |
| BM2'CL | DL?GLVVEY | 9 | III |
| CM29'CL | KVSLSAFDI | 9 | III |
| B-B10 M0'CL | RGDAMYFDV | 9 | I |
| HSVCBM8'CL | DPNPWYFDL | 9 | III |
| HSVCD53'CL | DYGDYAFDI | 9 | III |
| HSVCBG6'CL | SAHSDAFDM | 9 | III |
| MICA 4'CL | LEGLGWFDP | 9 | I |
| 1/11'CL | RSDYGAIDY | 9 | III |
| 5/8'CL | NLGFYHMDV | 9 | III |
| B6204'CL | EARGGGGEY | 9 | III |
| VH CLONE | EGWISALNG | 9 | III |
| 1'CL | | | |
| VH CLONE | EGEGEYFDY | 9 | III |
| 32'CL | | | |
| MG6-1'CL | ERTSGDFDF | 9 | III |
| MG6-3'CL | NSPGATFES | 9 | III |
| Daudi'CL | GNGQKCFDY | 9 | III |
| IE4'CL | RGSLQYLDY | 9 | I |
| IF10'CL | NNGSYYFDY | 9 | I |
| hsighvm148'C | GSDYSNFAY | 9 | III |
| L | | | |
| E3-MPO'CL | STHRSAFDV | 9 | II |
| rev9Fd'CL | EGVHKNFDH | 9 | III |
| NANUC-1'CL | LSRAGGFDI | 9 | III |
| Patient | RMPAVAFDY | 9 | II |
| 14'CL | | | |
| 14G1'CL | RMPAVAFDY | 9 | II |
| 14G2'CL | RMRAVAFDY | 9 | II |
| 14G3'CL | RMPAVAFDY | 9 | II |
| A15'CL | DYGGNPAEL | 9 | I |
| G15'CL | GPTCSGGSC | 9 | I |
| M11'CL | RKGAAHFDY | 9 | I |
| RF-DI1'CL | EEVGGYFQH | 9 | III |
| AC-18'CL | DFDGGSFDY | 9 | III |
| AC-29'CL | DFDGGSLDY | 9 | III |
| AC-40'CL | DFDGGSFDY | 9 | III |
| TR35'CL | KVPSHGMDY | 9 | III |
| TR36'CL | KVPSHGMDY | 9 | III |
| TR37'CL | KVPSHGMDY | 9 | III |
| TR38'CL | KVPSHGMDY | 9 | III |
| L34'CL | QPLARHFDP | 9 | III |
| L100'CL | GPLMRWFDD | 9 | III |
| WG1'CL | VAVAGGFDP | 9 | III |
| RF-ET5'CL | GVEVAGTAS | 9 | I |
| RF-ET10'CL | YYESSAGPP | 9 | III |
| EW-D1'CL | EIPRGGSCY | 9 | III |
| EW-D3'CL | EIPRGGSCY | 9 | III |
| KN-D6'CL | KEKWDSSRC | 9 | III |
| HH-M2'CL | GSAAAGTQG | 9 | III |
| AK-D8'CL | DFSWAGPHF | 9 | III |
| BALL-1'CL | GTHYYDIRV | 9 | III |
| YJ | DGSGSYEGN | 9 | III |
| K2.2 | GGAVAAFDY | 9 | III |
| E2.5 | KPVTGGEDY | 9 | III |
| MSL5 | DYDGAWFAY | 9 | I |
| Hb-2 | WDGRLLVDY | 9 | III |
| b4'CL | HKGLRYFDY | 9 | III |
| b3'CL | HKGLRYFDY | 9 | III |
| b2'CL | HKGLRYFDY | 9 | III |
| b5'CL | HKGLRYFDY | 9 | III |
| b17'CL | HKGLRYFDY | 9 | III |
| b19'CL | HKGLRYFDY | 9 | III |
| A3-H2'CL | YRGDTYDYS | 9 | III |
| A3-M9'CL | WVGATTSDY | 9 | III |
| Tmu69'CL | EDMDYGMDV | 9 | III |
| Amu1d4-3'CL | GGRDRYLVY | 9 | III |
| 1946'CL | VRVSYGMDV | 9 | V |
| GN901v1.0 | MRKGYAMDY | 9 | III |
| GN901v1.1 | MRKGYAMDY | 9 | III |
| N901H/KOL | MRKGYAMDY | 9 | III |
| N901H/G36005 | MRKGYAMDY | 9 | III |
| N901H/PLO123 | MRKGYAMDY | 9 | III |
| Patient | RMPAVAFDY | 9 | II |
| 14'CL | | | |
| 14G1(2)'CL | RMPAVAFDY | 9 | II |
| 14G2'CL | RMRAVAFDY | 9 | II |
| 14G3'CL | RMPAVAFDY | 9 | II |
| CLL-8'CL | TSIVRGFGP | 9 | II |
| BA-1F`CL | DFFRDYFDY | 9 | I |
| BA-2P'CL | DFFRDYFDY | 9 | III |
| L3055 4.6'CL | GGTQPFDIR | 9 | II |
| 15'CL | SQASGPFDY | 9 | I |
| CL-G'CL | GLYQLLFDY | 9 | III |
| CL-O'CL | AGGRTSFDP | 9 | I |
| BA3'CL | EGNTKAPDY | 9 | III |
| PS`CL | NGTSGDFDY | 9 | II |
| HNK20 hu7 | YGTSYWFPY | 9 | I |
| HNK20 hu10 | YGTSYWFPY | 9 | I |
| Amu1d4-3'CL | GGRDRYLVY | 9 | III |
| Amu1e10-3'CL | LRYQLLYNY | 9 | I |
| 1e8-3'CL | YIAYDAFDI | 9 | I |
| 1f7-3'CL | ITPRNAVDI | 9 | III |
| Agamma41- | DGLLAATDY | 9 | III |
| 3'CL | | | |
| Agamma8-3'CL | DRAYLDFWG | 9 | III |
| Amu10-3'CL | DKEPAYFDY | 9 | I |
| Amu2-1'CL | RGFNGQLIF | 9 | I |
| Amu40-2'CL | LSVVVPAAL | 9 | III |
| Amu70-1'CL | LADDDPEDF | 9 | I |
| Tmu69'CL | EDMDYGMDV | 9 | III |
| B7-g2B01'CL | SAGGSAWST | 9 | III |
| B8-g3C11'CL | DRSYYGMDV | 9 | III |
| B8-g3F05'CL | DKGTRYSDQ | 9 | III |
| BF1N- | WLVEGSFDY | 9 | III |
| g3C12'CL | | | |
| BF1N- | GYVGSSLDY | 9 | III |
| g3H05'CL | | | |
| BF1P- | WHQLRGPDY | 9 | III |
| g2A11'CL | | | |
| BF2P1- | ENSDYYFDY | 9 | III |
| g3D10'CL | | | |
| BF2P1- | DGTYGSGVR | 9 | III |
| g3E12'CL | | | |
| BF2P2- | GGSMVPFDY | 9 | III |
| g3C10'CL | | | |
| BF2P2- | RGWNYYFDS | 9 | III |
| g3D05'CL | | | |
| BF2P2- | DAYYYGLDV | 9 | III |
| g3D12'CL | | | |
| BF2P3- | DGRYDPIDY | 9 | III |
| g3C10'CL | | | |
| BF2P1- | VGSSGWYDY | 9 | I |
| g7B02'CL | | | |
| BF2N1- | DLYDYYDEP | 9 | I |
| g1C10'CL | | | |
| BF2N2- | DGAAASFDY | 9 | I |
| g1A11'CL | | | |
| BF2N2- | VVGADYFDY | 9 | I |
| g1E01'CL | | | |
| BF2N1- | DQNWGYFDY | 9 | III |
| g3F03'CL | | | |
| BF2N2- | GVLRDAFDI | 9 | III |
| g3B07'CL | | | |
| BF2N2- | ASDGYGMDV | 9 | III |
| g3C03'CL | | | |
| BF2N2- | GVLRHALDI | 9 | III |
| g3F07'CL | | | |
| BF2N1- | GGCGWYKNY | 9 | III |
| g4A03'CL | | | |
| BF2N1- | GSNYAKTGY | 9 | III |
| g4B10'CL | | | |
| BF2N1- | GKFQLLFDY | 9 | III |
| g4C11'CL | | | |
| BF2N1- | ALHGGGMDV | 9 | III |
| g6A07'CL | | | |
| BF2N1- | ALHGGGMDV | 9 | II |
| g6F07'CL | | | |
| BF2N2- | VYPPDAFDL | 9 | III |
| g6D09'CL | | | |
| mAbRWL1'CL | PWDYWFFDL | 9 | II |
| SV-10 | DRVAAAGDY | 9 | III |
| SV-7 | DKGTRYSDQ | 9 | III |
| SV-9 | DRVATIPDY | 9 | III |
| DN6'CL | ERGITLMDV | 9 | I |
| DN7'CL | ERGITLMDV | 9 | I |
| SC12'CL | LDWLLPIDY | 9 | I |
| SC13'CL | LDWLLPIDY | 9 | I |
| D11'CL | DDGDRAFGY | 9 | III |
| JON'CL | DPWPAAFDI | 9 | III |
| DEZ'CL | VRGSWSGDS | 9 | III |
| BAR | RHSSDWYPY | 9 | III |
| KC13H'CL | SSPYGALDY | 9 | III |
| clone 15'CL | GLDQYKTGH | 9 | II |
| B22'CL | GAGAAPHDY | 9 | III |
| P13'CL | GAGAAPHDY | 9 | III |
| PS'CL | NGTSGDFDY | 9 | II |
| Patient 2 | ALRPATFDF | 9 | III |

**Table 2**

| # Name | L3 | Length- L3 | Class |
|---|---|---|---|
| HIV-s8'CL | QQYADLIT | 8 | IGG1-KAPPA |
| FOG1-A4'CL | QQYYSTPT | 8 | -KAPPA |
| SA-4B'CL | QQYNTYPT | 8 | IGG-KAPPA |
| HIV-b5'CL | QQGNSFPK | 8 | IGG1-KAPPA |
| HIV-loop8'CL | QQYGYSLT | 8 | IGG1-KAPPA |
| Reg-A'CL | QQFGGSFT | 8 | KAPPA |
| 9F12Fab'CL | QQSSNTVT | 8 | KAPPA |
| GP68'CL | QQYNSLIT | 8 | IGG1-KAPPA |
| C471'CL | QQYNNWPT | 8 | IGM-KAPPA |
| B8807'CL | LQHNSYPF | 8 | IGM-KAPPA |
| B122'CL | QQYNSQYT | 8 | IGM-KAPPA |
| B6204'CL | QQYGSLWT | 8 | IGM-KAPPA |
| IM-9'CL | QHYNRPWT | 8 | IGG-KAPPA |
| T48.16-G8'CL | QQYGSRLT | 8 | KAPPA |
| 7F'CL | QHYGTPRT | 8 | IGG1-KAPPA |
| 1A6'CL | QQYNNWPT | 8 | IGM-KAPPA |
| 1.59'CL | MQATQFPT | 8 | IGM-KAPPA |
| antiTac | HQASTYPL | 8 | KAPPA |
| WE | QQYGRSPR | 8 | KAPPA |
| D1.1 | QQDDLPYT | 8 | KAPPA |
| K2.2 | QNDNLPLT | 8 | KAPPA |
| E1.1 | QQESLPLT | 8 | KAPPA |
| E2.4 | QQDNLPLT | 8 | KAPPA |
| E2.5 | QQESLPCG | 8 | KAPPA |
| E2.11 | QQDSLPLT | 8 | KAPPA |
| SSaPB | QQYGSSRS | 8 | IGM-KAPPA |
| SEGaBM | QQYGSSRT | 8 | IGM-KAPPA |
| SELcLN | QQYCGSLS | 8 | IGM-KAPPA |
| mAb5.G3'CL | QQSYSTLT | 8 | IGM |
| P7'CL | QLYGSSLT | 8 | KAPPA |
| PA | QQYNNLWT | 8 | KAPPA |
| CAR | QQYNTFFT | 8 | KAPPA |
| Taykv322'CL | QQYGSSPT | 8 | KAPPA |
| Taykv310'CL | QQYGSSLT | 8 | KAPPA |
| Taykv320'CL | QQYGSSLT | 8 | KAPPA |
| slkv22'CL | QQYGSSKT | 8 | KAPPA |
| LES | QQYNNWPP | 8 | KAPPA |
| RF-TMC1'CL | QHRNNWPP | 8 | IGM-KAPPA-III |
| slkv4'CL | QQRSNWPS | 8 | KAPPA |
| MD3.13'CL | QQYGSSPT | 8 | KAPPA |
| VJI'CL | QQYDTIPT | 8 | KAPPA |
| rsv13L'CL | QASINTPL | 8 | IGG1-KAPPA |
| II.2'CL | MQALQPWT | 8 | KAPPA |
| I.75'CL | QQGFSDRS | 8 | KAPPA |
| II.14'CL | MQATQFVT | 8 | KAPPA |
| III.7'CL | QRCKGMFS | 8 | KAPPA |
| SPA3-16'CL | QQYGGSPW | 8 | KAPPA |
| VL CLONE 52'CL | CRSHWPYT | 8 | KAPPA |
| 6F5-01'CL | QQYYSTPP | 8 | KAPPA |
| 6F7-42'CL | QQCNTNPP | 8 | KAPPA |
| 6F8-01'CL | QQYYSTPP | 8 | KAPPA |
| 6F9-31'CL | QQYYSVPP | 8 | KAPPA |
| D7'CL | QQYDSLVT | 8 | IGG1-KAPPA |
| HuVK'CL | HQYLSSWT | 8 | KAPPA |
| BC-26'CL | MQGIHLLT | 8 | KAPPA |
| VkLaE34'CL | QHYYGTPH | 8 | KAPPA |
| FL9-K | QQYNTYPT | 8 | KAPPA |
| HSC7 | QEFGDSGT | 8 | IGG |
| HSC28 | QQYGGSPW | 8 | IGG |
| SEGcPB | QQYGSSRT | 8 | IGM-KAPPA |
| P3'CL | QQYDSLPT | 8 | KAPPA |
| A5K3'CL | QQYGSVFT | 8 | IGM |
| BZ1K1'CL | QQYNSYCS | 8 | IGM |
| BZ2K1'CL | QQYYSTPL | 8 | IGM |
| D11K3'CL | QQYNDWPT | 8 | IGM |
| D17K2'CL | MQNIQFPT | 8 | IGM |
| F21K1'CL | QQYDNLPP | 8 | IGM |
| F22K3'CL | QLLR?LRT | 8 | IGM |
| SCFV198'CL | YQYNNGYT | 8 | KAPPA |
| KC25L'CL | QQRSNWPT | 8 | KAPPA |
| ASSYN13'CL | QQYGTSHT | 8 | KAPPA |
| BCPBL1'CL | QQYNHWPS | 8 | KAPPA |
| BCPBL4'CL | QQYGSLYT | 8 | KAPPA |
| BCPBL6'CL | QQNKDWPL | 8 | KAPPA |
| BCSYN6'CL | QQFGTSLT | 8 | KAPPA |
| ITPBL14'CL | QQRSNWWT | 8 | KAPPA |
| ITPBL2'CL | QQCSNWPT | 8 | KAPPA |
| SP10'CL | QQYGSSPT | 8 | KAPPA |

**Table 3**

| # Name | L3 | Length-L3 | Class |
|---|---|---|---|
| 8E10'CL | QQYGSSPSIT | 10 | IGM-KAPPA |
| III-2R'CL | QKYNSAPPST | 10 | IGM-KAPPA |
| II-1'CL | QEYNNWPLWT | 10 | KAPPA |
| 35G6'CL | QQYGGSPPWT | 10 | IGM-KAPPA |
| GF4/1.1'CL | HEYNGWPPWT | 10 | IGG3-KAPPA |
| RF-TS5'CL | QQYNSYSPLT | 10 | IGM-KAPPA |
| O-81'CL | MQHTHWSPIT | 10 | IGM-KAPPA |
| mAb114'CL | QHYNNWPPWT | 10 | IGM-KAPPA |
| HIV-B8'CL | QQSYNTPPWT | 10 | IGG1-KAPPA |
| HIV-b8'CL | QQSYNTPPWT | 10 | IGG1-KAPPA |
| TT117'CL | QHYGSSPPWT | 10 | IGG1-KAPPA |
| HIV- | QQHNNWPPLT | 10 | IGG1-KAPPA |
| loop13'CL | | | |
| HIV-s3'CL | QVYGQSPVFT | 10 | IGG1-KAPPA |
| 1-185-37'CL | QQYGSSPMYT | 10 | IGM-KAPPA |
| 1-187-29'CL | QQYGSSPMYT | 10 | IGM-KAPPA |
| HIV-s5'CL | QRFGTSPLYT | 10 | IGG1-KAPPA |
| HIV-b3'CL | QQYGDSPLYS | 10 | IGG1-KAPPA |
| GER | QQYDDWPPIT | 10 | IGG-KAPPA |
| BLI'CL | QQLNSYPPYT | 10 | IGM-KAPPA |
| 2A4'CL | QQSYSTPPDT | 10 | IGG |
| 0-16'CL | QHYNNWPPSS | 10 | KAPPA |
| mAb48'CL | QHYNRLPPWT | 10 | IGG3-KAPPA |
| 447.8H'CL | QQYDRSVPLT | 10 | KAPPA |
| GP13'CL | QQYYTTPTYT | 10 | IGG1-KAPPA |
| M37GO37'CL | QQYYTTPPLT | 10 | IGG-KAPPA |
| 9500'CL | QQLYSYPHLT | 10 | IGM-KAPPA |
| 9702'CL | CQQYGSSRWT | 10 | IGG-KAPPA |
| GSD2B5B10'CL | MQALQTPMST | 10 | KAPPA |
| MD2F4'CL | QQRSEWPPLT | 10 | KAPPA |
| GAN4B.5'CL | QQYDTSPAWT | 10 | KAPPA |
| NANUC-2'CL | QQYGSSQGFT | 10 | IgG1-kappa |
| SOL10'CL | MQSIQLPRWT | 10 | KAPPA |
| AB1/2'CL | QHYGLSPPIT | 10 | IGG1-KAPPA |
| AB4'CL | QEYGSSPPRT | 10 | IGG1-KAPPA |
| RH-14'CL | SSYRSSSTRV | 10 | IGG1-LAMBDA |
| AB1/2'CL | QHYGLSPPIT | 10 | IGG1-KAPPA |
| AB4'CL | QEYGSSPPRT | 10 | IGG1-KAPPA |
| L55-81'CL | QQYYTTLPLT | 10 | IGM-KAPPA |
| B3 | SSYSSTTRVV | 10 | IGG |
| HUL-mRF'CL | QQYGSSPQTF | 10 | IGM-KAPPA |
| 25C1'CL | FCQYNRYPYT | 10 | KAPPA |
| LC4aPB | LQRSNWGEVT | 10 | IGM-KAPPA |
| LC4bPB | QQRSNWGEVT | 10 | IGM-KAPPA |
| LC4cPB | QQRSNWGEVT | 10 | IGM-KAPPA |
| mAb3.B6'CL | QQYGSSPLFT | 10 | IGM |
| mAb1.C8'CL | CSYTSSSTLV | 10 | IGM |
| P9'CL | QQRSNWPPIT | 10 | KAPPA |
| 21H9'CL | QQSYNTLSLT | 10 | IGG1-KAPPA |
| 19A5'CL | QHYGNSPPYT | 10 | IGG1-KAPPA |
| 43F10'CL | QQSHKTLAWT | 10 | IGG1-KAPPA |
| FON'CL | MQGTYWPPYT | 10 | IGM-KAPPA |
| Hu PR1A3 | HQYYTYPLFT | 10 | KAPPA |
| hu PR1A3 | HQYYTYPLFT | 10 | KAPPA |
| CLL-412'CL | QQSYSTPPWT | 10 | IGG-KAPPA |
| MEV | QQSYTNPEVT | 10 | KAPPA |
| SON | QQYGSSPPYT | 10 | IGM-KAPPA |
| HEW''CL | QQYGSSPRYT | 10 | KAPPA |
| HEW'CL | QQYGSSPRYT | 10 | KAPPA |
| JH' | QQFGNSPPL? | 10 | IGG2-KAPPA |
| HG2B10K'CL | QQYAGSPPVT | 10 | IGG-KAPPA |
| CLL'CL | QQYNNWPPWT | 10 | IGM-KAPPA |
| slkv12'CL | QQYNNWPPWT | 10 | KAPPA |
| bkv6'CL | QQRSNCSGLT | 10 | KAPPA |
| slkv11'CL | QQYNNWPPWT | 10 | KAPPA |
| slkv13'CL | QQYNNWPPWT | 10 | KAPPA |
| bkv7'CL | QQYNNWPPCT | 10 | KAPPA |
| bkv22'CL | QQYNNWPPWT | 10 | KAPPA |
| bkv35'CL | QQRSFWPPLT | 10 | KAPPA |
| MD3.3'CL | QQRSNWPSIT | 10 | KAPPA |
| MD3.1'CL | QQRSNWPPLT | 10 | KAPPA |
| GA3.6'CL | QQRTNWPIFT | 10 | KAPPA |
| M3.5N'CL | QQRSNWPPGT | 10 | KAPPA |
| MD3.4'CL | QQYNNWPPLT | 10 | KAPPA |
| M3 . 1N ' CL | QQYNNWPTWT | 10 | KAPPA |
| GA3.4'CL | QQRMRWPPLT | 10 | KAPPA |
| MD3.7'CL | QQYGSSPKWT | 10 | KAPPA |
| MD3.9'CL | QQYGSSPQYT | 10 | KAPPA |
| GA3.1'CL | QQYGSSPPYT | 10 | KAPPA |
| bkv32'CL | QQYDRSLPRT | 10 | KAPPA |
| GA3.5'CL | QQYGNSPLFS | 10 | KAPPA |
| GA3 . 8 ' CL | QQYGGSPLFS | 10 | KAPPA |
| E29.1 | QQYNNWPTWT | 10 | IGM-KAPPA |
| KAPPA'CL | | | |
| R5A3K'CL | MQALQTLGLT | 10 | IGM-KAPPA |
| R1C8K'CL | MQALQTLGLT | 10 | IGG-KAPPA |
| I.24'CL | QQSHSAPPYT | 10 | KAPPA |
| III.12'CL | QQYGSSPLFT | 10 | KAPPA |
| III.S'CL | QQYNDWPPWT | 10 | KAPPA |
| I.18'CL | QQYNGNSPLT | 10 | KAPPA |
| I.67'CL | QQLNTYPPWT | 10 | KAPPA |
| III.6'CL | HKYGGSPPYT | 10 | KAPPA |
| II.65'CL | MQDTHWPPWT | 10 | KAPPA |
| III.14'CL | QHYGRSPPLT | 10 | KAPPA |
| 424.F6.24'CL | QQYGNSPPYT | 10 | KAPPA |
| T33-5'CL | QQYGSSPPYT | 10 | IGM-KAPPA |
| AL-MH | QQYFNVPPVT | 10 | KAPPA |
| AL-Es305 | QHYHNLPPTT | 10 | KAPPA |
| L47'CL | IQGTHWPQYT | 10 | IGM-KAPPA AND LAMBDA |
| F29'CL | QQYGSSRALT | 10 | IGM-KAPPA AND LAMBDA |
| G28'CL | QQYYSTPSYT | 10 | IGM-KAPPA AND LAMBDA |
| G21'CL | MQALQTLMCS | 10 | IGM-KAPPA AND LAMBDA |
| VL CLONE | QQSYSTPPLT | 10 | KAPPA |
| 45'CL | | | |
| VL CLONE | QQSYSTPPIT | 10 | KAPPA |
| 48'CL | | | |
| VL CLONE | QQYGGSLPIT | 10 | KAPPA |
| 56'CL | | | |
| C9'CL | QQYGSSTPLT | 10 | IGG1-KAPPA |
| ITC88'CL | QQRSSWPPLT | 10 | KAPPA |
| AC18'CL | QQRYSWPPLT | 10 | KAPPA |
| AC31'CL | QQRYNWPPLT | 10 | KAPPA |
| AC32'CL | QQRSNWPPLT | 10 | KAPPA |
| AC37'CL | QQRSSWPPLT | 10 | KAPPA |
| B'20 | QQYNNWPPWT | 10 | IgM-VkIIIa |
| (Humkv328- | | | |
| Jk1)'CL | | | |
| B9601 (Vg- | QQRSNWPPYT | 10 | IgM-VkIIIa |
| Jk2)'CL | | | |
| MF8 | QQYNNWPPWT | 10 | IgM-VkIIIa |
| (Humkv328- | | | |
| Jk1)'CL | | | |
| B'2 | QQYNNWPPWT | 10 | IgM-VkIIIa |
| (Humkv328- | | | |
| Jk1)'CL | | | |
| kappa1'CL | QQYGSSPPIT | 10 | IGG2-KAPPA |
| kappa2'CL | QQYNNWPPIT | 10 | IGG2-KAPPA |
| kappa3'CL | QQRSSWPPIT | 10 | IGG2-KAPPA |
| kappa4'CL | QQYGSSPRVT | 10 | IGG2-KAPPA |
| kappa5'CL | QQYNTNSPIS | 10 | IGG2-KAPPA |
| kappa7'CL | QNYGSSPRIT | 10 | IGG2-KAPPA |
| kappa8'CL | QQYGSSPPIT | 10 | IGG2-KAPPA |
| ToP218'CL | MQSIQLPRFT | 10 | KAPPA |
| ToP241'CL | MQSVQLPRFT | 10 | KAPPA |
| ToP309'CL | MQSVQLPRFT | 10 | KAPPA |
| L1235K3'CL | QQYDKWPPVT | 10 | KAPPA |
| SOL1'CL | MQSIQFPRWT | 10 | KAPPA |
| BC-2'CL | MQGIHLPPYI | 10 | KAPPA |
| P3'CL | NQGTQWLLYT | 10 | KAPPA |
| P5'CL | QQYNSYAPYT | 10 | KAPPA |
| AB1/2'CL | QHYGLSPPIT | 10 | IGG-KAPPA |
| AB4'CL | QEYGSSPPRT | 10 | IGG-KAPPA |
| MH | QQYFNVPPVT | 10 | KAPPA |
| FL6-K | QQLTSYPPWT | 10 | KAPPA |
| FL2-K | QQVNSYPGLT | 10 | KAPPA |
| FL4-K | QQVFSYPGIT | 10 | KAPPA |
| FL1-K | QQYTSLPGIT | 10 | KAPPA |
| MM4-K | QHSYSTLPLT | 10 | KAPPA |
| MM9-K | QQYYNIPYIT | 10 | KAPPA |
| HSC4 | QLYGSSPRVT | 10 | IGG |
| HSC11 | QQYANWPPIT | 10 | IGG |
| HSC13 | QQYNISPRDT | 10 | IGG |
| HSC23 | QQFGSSPLIT | 10 | IGG |
| HSC35 | QQYGDFPRVT | 10 | IGG |
| REV | QQYGDWPPYT | 10 | KAPPA |
| BLU | QQYYTTLSWT | 10 | KAPPA |
| BK2'CL | QQYNKWPPLT | 10 | KAPPA |
| GK6'CL | MQGTHWLPVT | 10 | IGG-KAPPA |
| L1236K3'CL | QQYDKWPPVT | 10 | KAPPA |
| P1'CL | QQYDNLPPIH | 10 | KAPPA |
| H01'CL | QQLNNYPPFT | 10 | KAPPA |
| I01'CL | QQSYSTPPYT | 10 | KAPPA |
| I10'CL | QQSYSTPPYS | 10 | KAPPA |
| I12'CL | QQSYSTPPYT | 10 | KAPPA |
| 126TP14K'CL | QQYNNWLPFT | 10 | IGG-KAPPA |
| L32'CL | AAWDDSLTLM | 10 | IGM-KAPPA |

For insertion of CDR3s, single oligonucleotides encoding each of the CDR3s of table H from the plus strand were synthesised with 12 homologous nucleotides added to each termini for annealing to the consensus VH and VL genes. In addition to these CDR sequences, CDRs from the antibody E25 (see example 4) were included. These primers were extended and secondary primers were added to introduce directly adjacent to the N and C termini of the VH and VL genes (without C regions) 5'Notl-3'Xbal sites for VH and 5'SpeI-3'BamHI for VL. Prior to cloning, a further pair of complimentary primers was used to insert the linker sequence (Gly4Ser)3 between VH and VL whilst maintaining XbaI and SpeI sites. Full-sized VH-linker-VL fragments were digested with NotI and BamHI and were cloned into NotI-BamHI digested pBluescript II KS(+/-) (Stratagene, Amsterdam, Netherlands).

Individual Bluescript clones were picked, plasmid DNA was purified and dispensed robotically into 96 well plates as described in WO99/11777. DNAs were then subjected to IVTT including tRNA-biotinyl-lysine and further robotically arrayed onto a streptavidin surface as described in WO99/11777. The immobilised initial scFv library of 10,000 independant clones was then screened by incubation with recombinant human IgE Fab (see example 4). Wells were blocked with PBS/3% BSA at room temperature for 1 hour, washed three times in PBS and treated with 5ug/ml human IgE Fab in PBS/3% BSA for 1 hour. Wells were then washed a further three times in PBS and treated with 5ug/ml alkaline phosphatase-labelled chimeric anti-IgE (example 4) in PBS/3% BSA for 1.5hrs. Wells were further washed five times in PBS and colour developed using the substrates 5-bromo-1-chloro-3-indolyl phosphate and nitro blue tetrazolium (Roche Molecular) for visualization. A strong signal observed at a frequency of 1 of 9600 wells was shown to derive from a VH and VL pair both containing E25 CDR3's.

### Example 6 - Construction of Composite Mouse Anti-TNFα Antibody

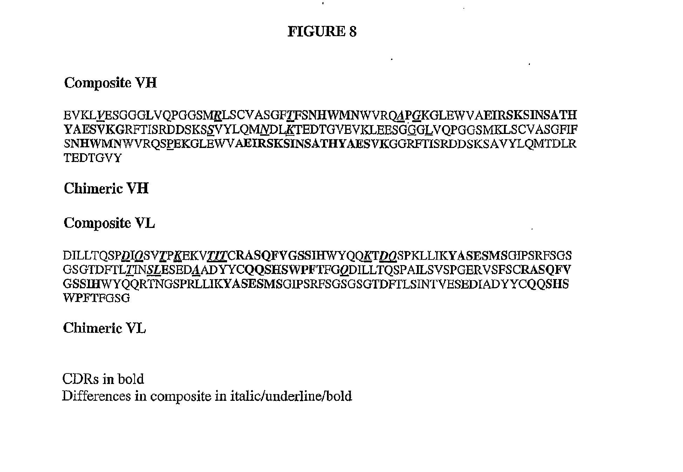

A mouse variable region sequence library was created as described in example 1 for the human library using NCBI Igblast, Kabat and Genbank databases. The reference antibody variable region sequences used was a chimeric anti-TNFα antibody known as Remicade® (Le et al., US6277969) using the variable regions of the mouse cA2 antibody. Segments from the in silico mouse variable region sequence library were selected partly corresponding amino acids in the Remicade® variable region but including variations designed to avoid human T cell epitopes in the sequence in the form of non-self human MHC class II binders measured as in example 1. Composite mouse VH and VL sequences compared to sequences used in the chimeric antibody are shown in figure 8 indicating differences of 9 and 16 amino acids in VH and VL respectively between the two antibodies as a result of segment selection for epitope avoidance in the Composite Mouse antibody.

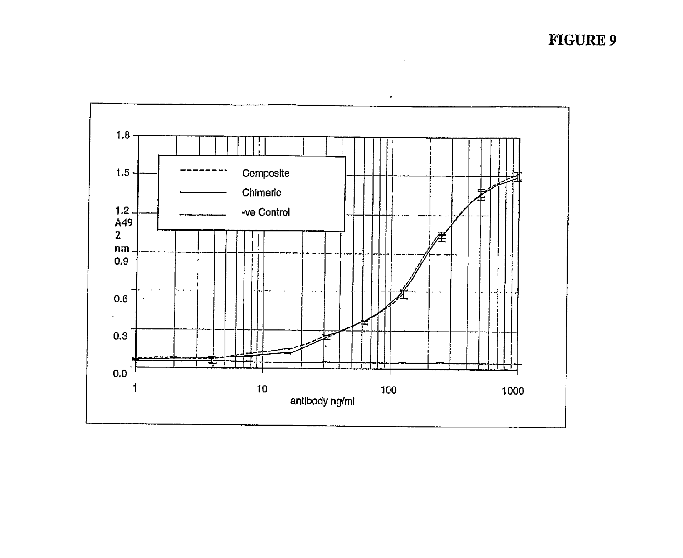

The Composite Mouse and chimeric anti-TNFα antibodies were generated as described in example 1. Comparison of purified antibodies for binding to immobilised human TNα in a standard ELISA (described in WO 03/042247A2) showed that the Composite Mouse antibody retained the full binding capacity of the chimeric anti-TNFα antibody (figure 9). The immunogenicity of these antibodies was then compared as described in example 2 using 24 HLA-DR typed human blood samples for T cell assays. The results showed that the chimaeric anti-TNFα antibody induced significant proliferative responses (SI greater than 2) in nine of the twenty four healthy donors tested (37.5%) compared to the Composite Mouse anti-TNFα antibody where none of the twenty four donors (0%) induced SI>2. These results indicated that a Composite Mouse antibody comprising segments of variable region sequence derived totally from mouse V regions with selection of such segments to avoid human T cell epitopes could remove the immunogenicity in human T cell assays displayed by the corresponding chimeric antibody without any epitope avoidance measures.

### Example 7: Construction of a Composite Human Anti-TNFα Antibody

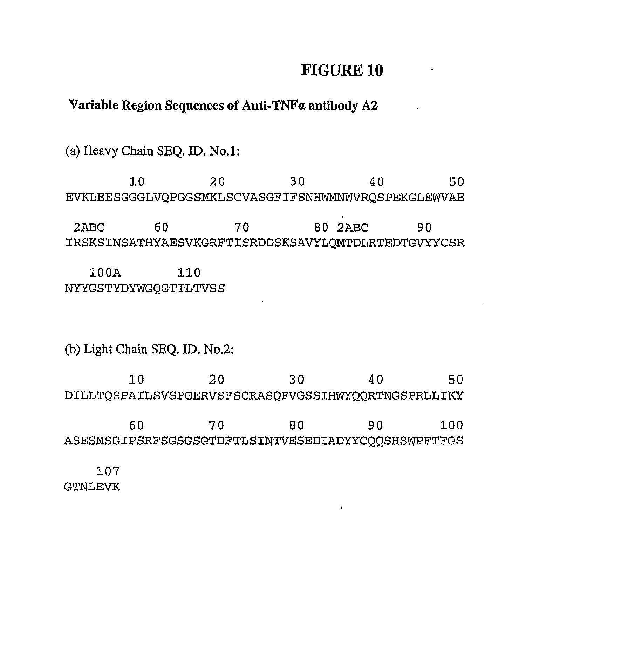

The reference mouse variable region heavy and light chain sequences of antibody A2 directed against human TNFα was obtained from US patent 5656272 (Fig. 10. SEQ. IDs No. 1 and No. 2 respectively). A structural model was made of the mouse reference variable regions and amino-acids critical for CDR conformation were identified based upon their distance from the CDRs (<3Ǻ) and their likely packing close to CDRs. Important, but less critical residues were identified based upon their distance from the CDRs (>3Å <6Å) and their likely influence on more critical residues packing closer to the CDRs. A further set of residues were identified based upon their frequency of occurrence in mouse antibody sequences i.e. amino-acids found at a particular location with a frequency of less than 1%.

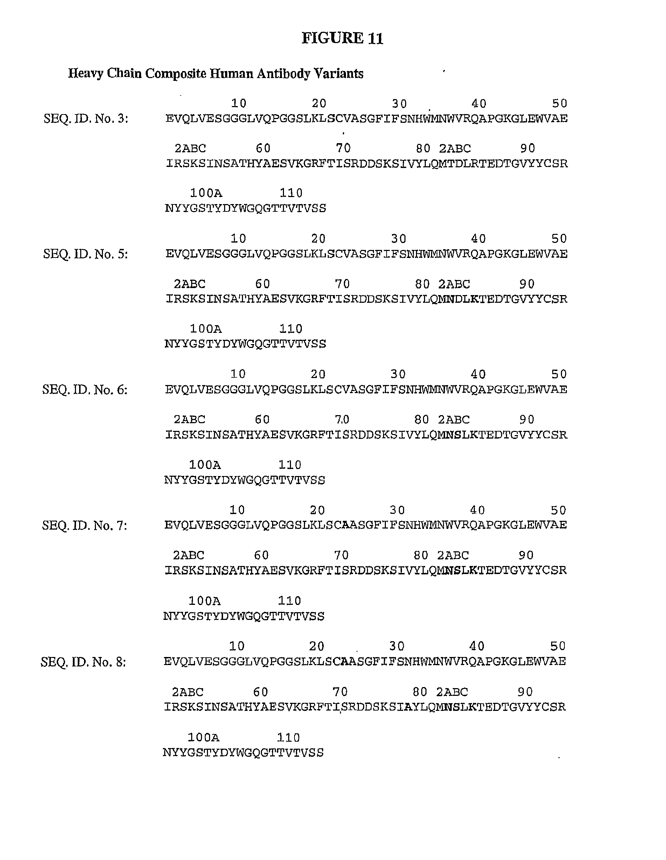

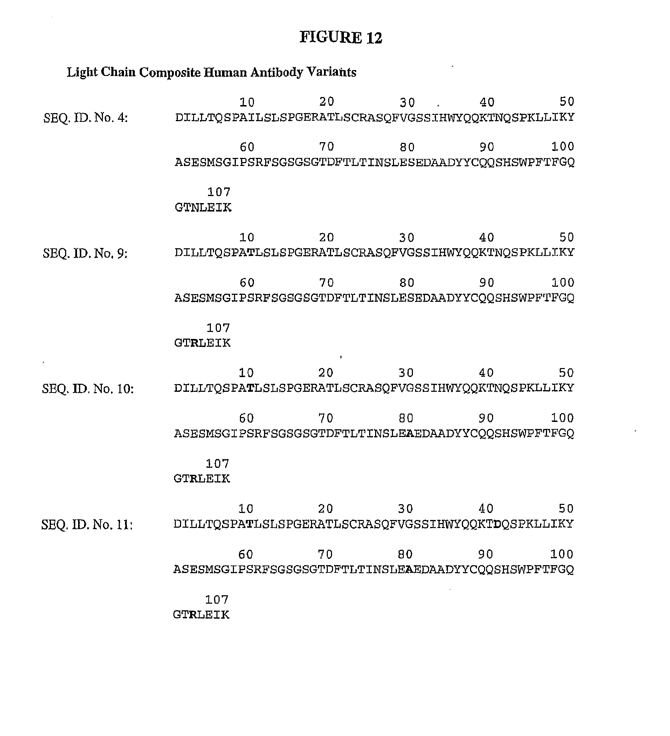

Human V region sequence segments that included as many of these residues as possible were selected (table 4) to create full-length VH and VL sequences. Alterations were made to these sequences to include all the identified structurally important residues to create sequences to serve as a template for epitope avoidance and Composite Human Antibody design. A preferred sequence for each composite VH and VL was designed to include important residues from the reference mouse antibody. These variable heavy and light chain amino acid sequences are shown in Figs. 11 and 12. SEQ IDs No. 3 and No. 4 respectively.

**Table 4: Human Antibody Database Derivation of Sequence Segments For Primary CHAB Variants**

| **(a) Heavy Chain** | |
|---|---|
| Genbank | |
| Accession No | Sequence segment |
| CAA61442 | EVQLVESGGGLVQPGGSLKLSC |
| CAD88676 | LSCVASGFIFS |
| CAB37182 | FSNHWM |
| AAS86088 | HMMNWVRQAPGKGLEWVA |
| CAC43592 | AEI |
| ABB54411 | IRSKS |
| AAL96548 | SIN |
| AAK51359 | NSA |
| CAA67405 | SAT |
| CAB87447 | ATHYA |
| AAD30769 | HYAESVKGRFTISRD |
| CAC15703 | RFTISRDDSKSI |
| AAQ05509 | IVYLQM |
| AAT96742 | YLQMTDLR |
| AAD20526 | LRTEDTGVYYC |
| CAB44788 | VYYCSRNY |
| AAO38724 | NYYGS |
| AAK14004 | GSTY |
| AAD20470 | TYDYWGQGT |
| AAB32435 | DYWGQGTTVTVSS |

| **(b) Light Chain** | |
|---|---|
| Genbank | |
| Accession No. | Sequence segment |
| CAC06686 | DILLTQ |
| AAX57564 | LTQSPAILSLSPGERATLSC |
| X72820 | LSLSPGERATLSCRASQ |
| AAC15439 | QFV |
| AAZ09058 | VGSS |
| Z84907 | SSI |
| AAL10835 | IHWYQQK |
| AAQ21835 | QQKPNQSPKLLIK |
| M27751 | LLIKYAS |
| AAY16512 | YASE |
| AAR89591 | ES |
| AAD 19534 | SM |
| AAV71416 | MSG |
| AAZ09098 | GIP |
| CAG27043 | PSRFSGSGSGTDFTLTINSLE |
| AAQ21937 | SLESEDAA |
| AAC41988 | ADYYCQQ |
| AAY33370 | YYCQQSHS |
| AAD19457 | HSWP |
| AAQ55271 | WPFTFGQGT |
| AAW69118 | TFGQGTNLEIK |

The composite heavy and light chain variable region sequences were scanned for the presence of potential T cell epitopes using a variety of *in silico* methods (e.g. Propred [http://imtech.res.in/raghava/propred/index.html], Peptide Threading [www.csd.abdn.ac.uk/~gjlk/MHC-thread], SYFPEITHI (www.syfpeithi.de), MHCpred (www.jenner.ac.uk/MHCPred/) and compared to homologous human germ-line framework region sequences in conjuction with reference mouse CDRs.

The following heavy chain variable region variants were made (see Fig 11):
SEQ. ID. No. 5 contains the following changes with respect to SEQ. ID. No. 3: T82aN + R83K.
SEQ. ID. No. 6 contains the following changes with respect to SEQ. ID. No. 3: T82aN + R83K + D82bS
SEQ. ID. No. 7 contains the following changes with respect to SEQ. ID. No. 3: T82aN + R83k + D82bS + V23A.
SEQ. ID. No. 8 contains the following changes with respect to SEQ. ID. No. 3: T82aN + R83K + D82bS + V23A + V78A

The following light chain variable region variants were made (see Fig. 12):
SEQ. ID. No. 9 contains the following changes with respect to SEQ. ID. No. 4: I10T + N103R.
SEQ. ID. No. 10 contains the following changes with respect to SEQ. ID. No. 4: I10T + N103R + S80A.
SEQ. ID. No. 11 contains the following changes with respect to SEQ. ID. No. 4: I10T + N103R + S80A + N41D.

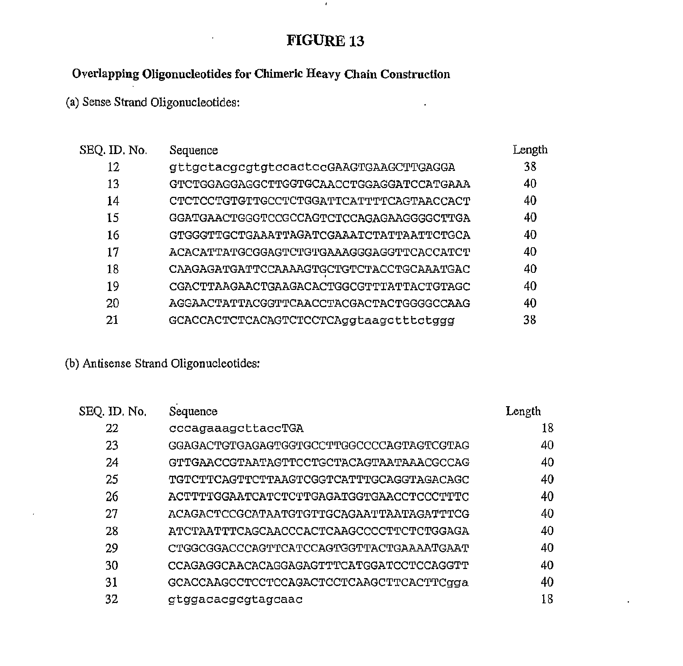

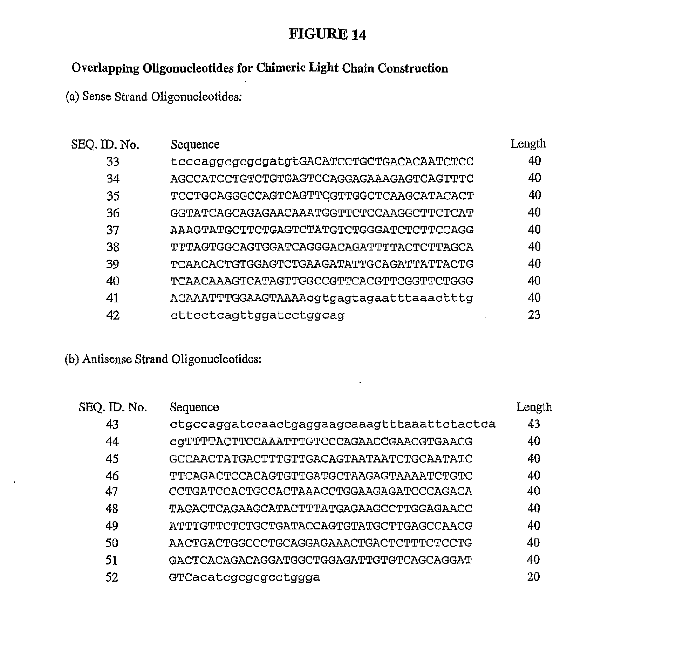

For construction of a control chimeric antibody, the nucleotide sequences that translate to give SEQ. IDs No. 1 and No. 2 were constructed using a series of overlapping 40mer synthetic oligonucleotides. The V region sequences were flanked by additional 5' and 3' sequences to facilitate cloning into mammalian expression vectors. The sequences of the oligonucleotides are shown in Figure 13 and Figure 14

Oligonucleotides were purchased from Sigma-Genosys (Poole, UK) and resuspended at a concentration of 100µM. 1µl of each of the heavy chain sense strand oligonucleotides, except the most 5' oligonucleotide, were mixed together and 1.5µl (approx. 1µg) of the mix was treated with Polynucleotide Kinase (PNK, Invitrogen, Paisley UK) in a 20µl reaction containing additionally: 2µl 10x PNK buffer, 2µl 10mM ATP, 14µl H₂O, 0.5µl (5 units) PNK. The reaction was incubated at 37°C for 30 min and the enzyme inactivated by heating at 70°C for 20 min. The heavy chain antisense, light chain sense and antisense oligonucleotides were similarly phosphorylated. The 5' oligonucleotide from each set was diluted 1 in 9 with H₂O and 1.5µl added to the appropriate reaction mix. Each reaction was then diluted to 0.5ml and spin dialyzed in an Amicon microcon YM3 concentrator for 90 min at 8000 rpm until the volume was not more than 44µl.

The sense and antisense mixes for the heavy chain, and those for the light chain, were combined and made up to 88µl with H₂O. 10µl 10x Ligase Chain Reaction (LCR) buffer and 2µl Pfu ligase (8 units, Stratagene, Cambridge UK) were added to each reaction and incubated as follows in a programmable heating block: 94°C for 4 min, then 60°C for 3min for 1 cycle followed by 20 cycles of 94°C for 39 sec. then 60°C for 2 min. Finally the reactions were incubated for 5 min at 60°C. 10µl of each LCR was run through a 1% agarose gel stained with ethidium bromide and compared to 1Kb ladder markers (Invitrogen). A smear of ligated DNA was observed in each lane, surrounding a faint specific band of approximately 400bp.

The heavy and light chain LCRs were amplified via PCR using as primers SEQ. ID. No.s 12 and 22 for the heavy chain and SEQ. ID. No.s 33 and 43 for the light chain. The following were included in each reaction: 5µl LCR, 5µl 10x Expand HiFi buffer (Roche, Lewes UK), 1µl 10mM NTP mix, 0.25µl each primer (from 100µM stocks), 0.5µl Expand HiFi polymerase (3 units, Roche) and 38µl H₂O. The reactions were cycled as follows: 94°C 2 min followed by 20 cycles of 94°C for 30 sec, 60°C for 30 sec and 72°C for 30 sec. Finally the reaction was incubated for 5 min at 72°C. The yield and specificity of the reaction was confirmed by agarose gel electrophoresis, as above. Specific, sharp bands at approximately 400bp were observed for each reaction.

The reaction products were purified using a Qiagen PCR purification kit and each eluted in 30µl H₂O. The heavy chain product was digested in a standard reaction with restriction enzymes *Mlu* I and *Hind* III and the light chain product was digested with *Bss*H II and *Bam*H I. The reaction products were again purified using a Qiagen PCR purification kit and each eluted in 30µl H₂O.

The light chain expression vector pANT08 was based upon a pAT153 backbone and contains in the following order: CMV immediate/early enhancer promoter -590 to +7, a 30nt 5' UTR derived from a highly expressed mouse antibody light chain RNA, a mouse consensus light chain signal sequence incorporating a *Bss*H II restriction site near the variable region start codon, a short linker (in place of a variable region) to a human composite intron contining 33 nt from the variable region splice site to a *Bam*H I restriction site followed by a fragment of human genomic DNA containing 343 nt of the intron preceding the human constant Kappa (CK) region gene, the CK gene and CK polyA.

The heavy chain expression vector pANT09 was similar to pANT08 through the promoter region, which is followed by: a 62nt 5' UTR derived from the heavy chain counterpart of that described above, a mouse heavy chain consensus signal sequence that incorporates a *Mlu* I restriction site near the variable region start codon, a short linker (in place of a variable region) to the variable region splice site immediately followed by a fragment of human genomic DNA from a *Hind* III restriction site located in the intron 211 nt upstream of the CH1 gene, to the end of the CH region poly A site. This fragment includes the CH1, hinge, CH2 and CH3 introns and exons of human IgG1. This vector also included a gene for dihydrofolate reductase, controlled by an SV40 promoter and polyA signal, for resistance to methotrexate.

2µg each vector was digested with the relevant restriction enzymes in standard reactions in a total volume of 30µl. Each reaction was run through a 1% agarose gel, as above, and the vector specific bands (6.0Kbp heavy chain and 4.2Kbp light chain) were excised from the gel and purified using a Qiagen gel extract kit and eluted in 30µl H₂O.

1µl each digested vector was ligated to 3µl of the corresponding digested variable gene PCR product using a Ligafast kit (Promega, Southampton UK). 2.5µl each ligation reaction was transformed into sub-cloning efficiency competent XL1-blue (Stratagene), as instructed by the manufacturer, and plated onto LB agar plates containing 100µg/ml ampicillin and incubated overnight at 37°C. Ten bacterial colonies from each ligation were inoculated into 10ml 2x YT broth containing 100µg/ml ampicillin and grown overnight at 37°C with shaking. Plasmid was purified from 1.5ml each overnight culture using a Qiagen plasmid preparation kit and each eluted in 50µl H₂O. The plasmids were sent to a contract sequencing facility and sequenced with a standard CMV promoter primer and clones with the correct V region gene sequence identified.

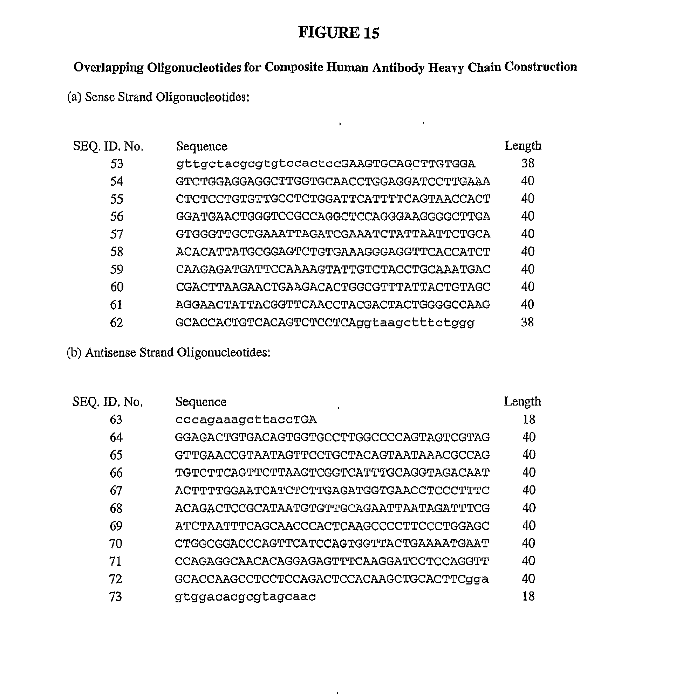

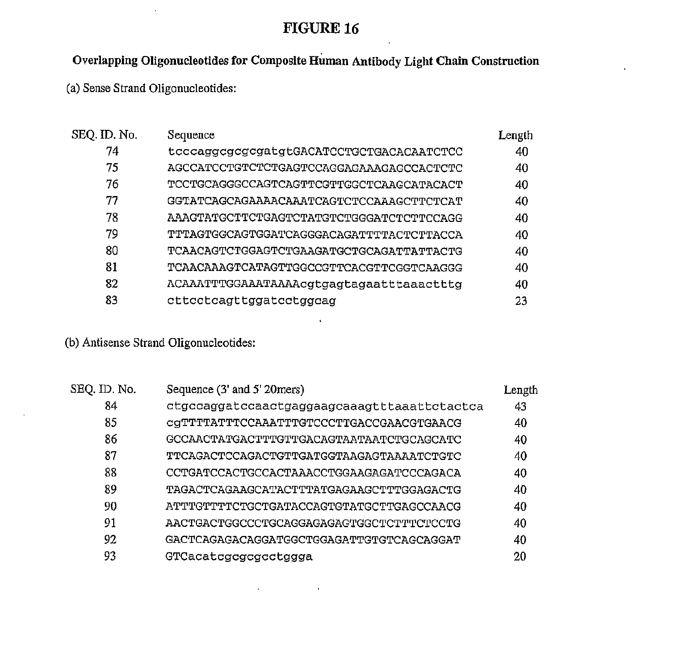

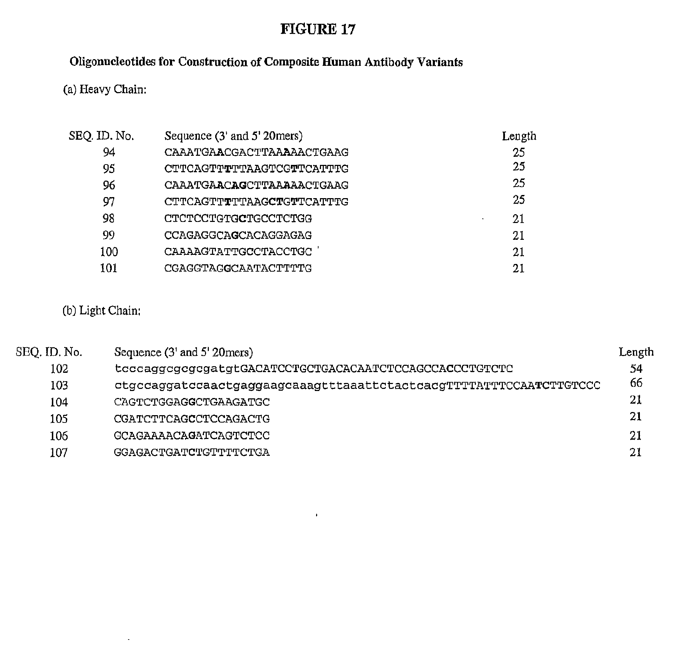

For construction of Compsoite Human Antibodies, the nucleotide sequences that translate to give SEQ. IDs No. 3 and No. 4 were constructed using a series of overlapping 40mer synthetic oligonucleotides. The sequences of the oligonucleotides are shown in Figure 15 and Figure 16. The nucleotide sequence that translates to give SEQ. ID. No. 5 was constructed via overlap PCR using oligonucleotide primers SEQ. ID. No.s 94 and 95 (Figure 17) together with oligonucleotides SEQ. ID. No.s 53 and 63 and the plasmid DNA of the primary Composite Human Antibody heavy chain variant as template. Two PCR reactions were done using as primer pairs either SEQ. ID. No.s 53 and 95, or SEQ. ID. No.s 94 and 63. The following were included in each reaction: 1µl (100ng) plasmid template, 5µl 10x Expand HiFi buffer (Roche), 1µl 10mM NTP mix, 0.25µl each primer (from 100µM stocks), 0.5µl Expand HiFi polymerase (3 units, Roche) and 42µl H₂O. The reactions were cycled as follows: 94°C 2 min followed by 20 cycles of 94°C for 30 sec, 60°C for 30 sec and 72°C for 30 sec. Finally the reaction was incubated for 5 min at 72°C. The entire reactions were electrophoresed through a 1% agarose gel and the specific bands of 295bp and 126bp were excised and purified using a Qiagen gel extraction kit. The DNAs were eluted in 30µl H₂O.

The two purified fragments were joined in a PCR reaction using oligonucleotide primers SEQ. ID. No.s 53 and 63 using PCR conditions as described above, except that the template used was 1µl 295bp product and 1µl 126bp product, hence the amount of H₂O was reduced to 41µl. The joined PCR product of 396bp was purified using a Qiagen PCR purification kit and was eluted in 30µl H₂O.

The nucleotide sequence that translates to give SEQ. ID. No. 6 was constructed via overlap PCR using oligonucleotide primers SEQ. ID. No.s 96 and 97 (Figure 17) together with oligonucleotides SEQ. ID. No.s 53 and 63 and the plasmid DNA of the primary Composite Human Antibody heavy chain variant as template. Two PCR reactions were done using as primer pairs either SEQ. ID. No.s 53 and 97, or SEQ. ID. No.s 96 and 63. The first stage PCRs were done as described above and yielded fragments of 295bp and 126bp. These fragments were purified, joined together and repurified, also as described above.

The nucleotide sequence that translates to give SEQ. ID. No. 7 was constructed via overlap PCR using oligonucleotide primers SEQ. ID. No.s 98 and 99 (Figure 17) together with oligonucleotides SEQ. ID. No.s 53 and 63 and the PCR product for SEQ. ID. No. 6 as template. Two PCR reactions were done using as primer pairs either SEQ. ID. No.s 53 and 99, or SEQ. ID. No.s 98 and 63. The first stage PCRs were done as described above and yielded fragments of 98bp and 318bp. These fragments were purified, joined together and repurified, also as described above.

The nucleotide sequence that translates to give SEQ. ID. No. 8 was constructed via overlap PCR using oligonucleotide primers SEQ. ID. No.s 100 and 101 (Figure 17) together with oligonucleotides SEQ. ID. No.s 53 and 63 and the PCR product for SEQ. ID. No. 7 as template. Two PCR reactions were done using as primer pairs either SEQ. ID. No.s 53 and 101, or SEQ. ID. No.s 100 and 63. The first stage PCRs were done as described above and yielded fragments of 270bp and 155bp. These fragments were purified, joined together and repurified, also as described above.

Each of the above PCR products was digested with *Mlu* I and *Hind* III and ligated into similarly digested pANT09. The ligations were transformed and plated, colonies picked, plasmids prepared and sequenced all as described above.

The nucleotide sequence that translates to give SEQ. ID. No. 9 was constructed via PCR using oligonucleotide primers SEQ. ID. No.s 102 and 103 (Figure 17) and the plasmid DNA of the primary Composite Human Antibody light chain variant as template.A single PCR reaction was done, as described for the heavy chain variants, that yielded a product of 383bp. The entire reaction was electrophoresed through a 1% agarose gel and the specific band was excised and purified using a Qiagen gel extraction kit. The DNA was eluted in 30µl H₂O.

The nucleotide sequence that translates to give SEQ. ID. No. 10 was constructed via overlap PCR using oligonucleotide primers SEQ. ID. No.s 104 and 105 (Figure 17) together with oligonucleotides SEQ. ID. No.s 74 and 84 and the PCR product for SEQ. ID. No. 9 as template. Two PCR reactions were done using as primer pairs either SEQ. ID. No.s 74 and 105, or SEQ. ID. No.s 104 and 84. The first stage PCRs were done as described above for the heavy chain variants and yielded fragments of 265bp and 139bp. These fragments were purified, joined together to create a product of 383bp and repurified, also as described above for the heavy chain variants.

The nucleotide sequence that translates to give SEQ. ID. No. 11 was constructed via overlap PCR using oligonucleotide primers SEQ. ID. No.s 106 and 107 (Figure 17) together with oligonucleotides SEQ. ID. No.s 74 and 84 and the PCR product for SEQ. ID. No. 10 as template. Two PCR reactions were done using as primer pairs either SEQ. ID. No.s 74 and 107, or SEQ. ID. No.s 106 and 84. The first stage PCRs were done as described above for the heavy chain variants and yielded fragments of 148bp and 256bp. These fragments were purified, joined together to create a product of 383bp and repurified, also as described above for the heavy chain variants.

Each of the above PCR products was digested with *Bss*H II and *Bam*H I and ligated into similarly digested pANT08. The ligations were transformed and plated, colonies picked, plasmid prepared and sequenced all as described above.

CHO-K1 cells (ATCC# CCL-61) were propagated in high glucose DMEM containing 10% FCS, L-glutamine, sodium pyruvate and L-proline. Near confluent cultures were harvested for transfection using Lipofectamine 2000 as instructed by the manufacturer (Invitrogen). Transfections were done in 48 well plates seeded with 200µl cells at 3x10⁵ cells/ml using a total of 0.5µg plasmid DNA comprising 0.3µg heavy chain construct and 0.2µg light chain construct.

Transfections were incubated at 37°C/5% CO₂ for 48 to 72h before harvesting the supernatants. Antibody expression was quantified by ELISA using: a mouse monoclonal anti-human IgG capture antibody, human IgG1/Kappa standards and HRP conjugated goat anti-human Kappa light chains as detection antibody (all reagents from Sigma).

All combinations of heavy and light chains were transfected (i.e. 6 heavy chains x 5 light chains = 30 transfections). Antibody expression levels were generally in the range of 0.5 to 2.0 µg/ml, however no expression was observed with heavy chain SEQ. ID. No. 8.

The expressed antibodies were tested for their ability to neutralize the activity of human TNFα using TNF-sensitive WEHI-164 cells (Espevik et al., J. Immunol. Methods 1986, 95, 99-105). Cells were plated in 1 µg/ml actinomycin D at 5x10⁴ cells per well in 96-well microtiter plates for 3-4 hours. Cells were exposed to 40 pM human TNFα and varying concentrations of the chimeric antibody (range Ing/ml to 500ng/ml) to create a standard curve. The various combinations of heavy and light chains were tested at a single concentration point of 25ng/ml that had previously been determined as the ED₅₀ of the chimeric antibody. All assays were done in triplicate. The mixtures were incubated overnight at 37°C. Cell viability was determined by adding 3-[4,5-dimethyl-thiazol-2-yl]-2,5-diphenyltetrazaliumbromide dye (MTT) to a final concentration of 0.5 mg/ml, incubating for 4 hours at 37°C, lysing the cells in 0.1M HCl, 0.1% SDS and measuring the optical density at 550 nm wavelength.

The optical densities from the heavy and light chain combinations were used to calculate the apparent antibody concentrations from the standard curve. The apparent concentration of the chimeric was divided by that of each of the variant combinations to give a fold difference value. Values lower than that for the chimeric indicated that those combinations were more effective at protecting the cells from TNFα cytotoxicity, whereas higher values indicated that they were less effective. The values for all combinations are shown in Table 5.

**Table 5: Ratio of Activities of Composite Human Antibody Variants compared to Chimeric Antibody**

| **SEQ. ID.No.** | **1** | **3** | **5** | **6** | **7** | **8** |
|---|---|---|---|---|---|---|
| **2** | 1.00 | 1.38 | 1.24 | 1.20 | 1.02 | ND |
| **4** | 1.51 | 2.28 | 1.28 | 1.38 | 1.05 | ND |
| **9** | 1.28 | 2.14 | 1.32 | 1.77 | 0.95 | ND |
| **10** | 1.31 | 2.51 | 1.17 | 1.63 | 0.98 | ND |
| **11** | 16.90 | 15.15 | 196.49 | 134.08 | 105.61 | ND |

The following Composite Human Antibody heavy and light chain combinations gave fold differences close to 1.0: SEQ. ID. No.s 5/10, SEQ. ID. No.s 7/4, SEQ. ID. No.s 7/9, SEQ. ID. No.s 7/10. These combinations were selected for further study.

The expression plasmids carrying the sequences selected above were transfected into NS0 cells (ECACC No. 85110503). The cells were grown in high glucose DMEM containing L-glutamine, sodium pyruvate, 5% ultra low IgG FCS and pen/strep. Cells were harvested during log phase of growth, spun down and resuspended at 5x10⁶ cells/ml in fresh growth media. 750µl cells were mixed with a total of 30µg of each plasmid pair, which had been linearised with *Ssp* I, in 50µl H₂O. The cell/plasmid mixture was transferred to a 4mm gap cuvette and electroporated using an Equibio Easyject Plus at 250V, 1500µF, infinite resistance. The electroporate was immediately transferred to 25ml pre-warmed growth media and plated out in 5x 96 well flat bottomed plates at 100µl/well. The plates were incubated at 37°C/5% CO₂. 48h post-electroporation, 50µl media containing 300nM methotrexate was added to each well to give a final concentration of 100nM. 7 days post-electroporation a further 50µl of media containing 100nM methotrexate was added to each well.

Approximately 2 week post-electroporation, the media in some wells began to turn yellow, indicating transfected colony growth. Media from these wells were tested for antibody expression using the anti-human IgG Fc capture/ anti-human Ig Kappa light chain HRP conjugate detection ELISA. The test samples were compared to a human IgG1/Kappa standard and antibody expression levels estimated. Colonies expressing useful amounts of antibody were expanded in media containing 200nM methotrexate.

Antibodies were purified from 500ml culture media via protein A affinity chromarography followed by size exclusion chromatography using Sephacryl S200. The purified antibodies were quantified by UV absorbance at 280nm, assuming that OD₂₈₀ 1= 1.4mg/ml.

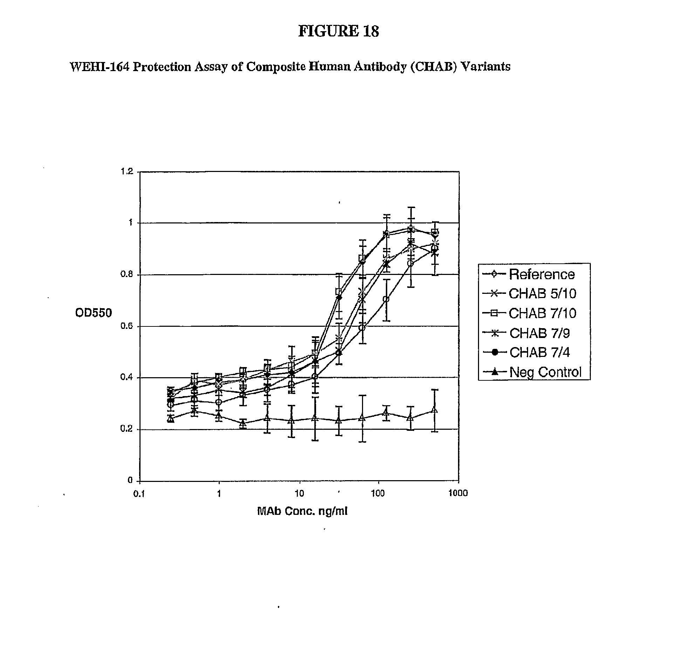

Purified chimeric and composite antibodies were tested for activity via the WEHI-164 protection assay described in example 4 above. Each antibody was tested over the full concentration range previously used to create the standard curve (see Figure 18). Composite Human Antibody 7/10 (i.e. containing SEQ. ID. No.s 7 and 10) was found to be the most active variant and had the same activity as the chimeric antibody. Composite Human Antibodies 7/9 and 5/10 had similar activity that was slightly reduced compared to the chimeric, and Composite Human Antibody 7/4 was the least active.

Therefore since Composite Human Antibody 7/10 was predicted to have the most favourable MHC class II binding profile and was the most active variant, this was selected for testing in a time course T cell proliferation assay. Human PBMCs were prepared from buffy coats derived from human blood donations via two rounds of Ficoll density centrifugation. The prepared PBMC were resuspended at a density of 3x10⁷ cells/ml in 1ml aliquots in 90% human AB serum/10% DMSO, and stored under liquid nitrogen. PBMC were tissue typed using a Dynal Allset® PCR typing kit.

The lead Composite Human Antibody was compared to the chimeric antibody in whole protein T cell assays using human PBMC from 20 healthy donors. PBMC from each donor were thawed, washed and resuspended in AIM V serum free lymphocyte growth media. On day 1, 50µg protein was added to 2ml bulk cultures of 4x10⁶ PBMC in 24 well plates, and triplicate 100µl aliquots were removed and transferred to 96 well plates on days 6 to 9. Each aliquot was pulsed with 75µl media containing 1µCi tritiated thymidine for 24h, before harvesting and measuring incorporation of radioactivity. Results were normalised by calculation of the Stimulation Index (SI). Coverage of a wide range of HLA DR allotypes was achieved by selecting donors according to individual MHC haplotypes.

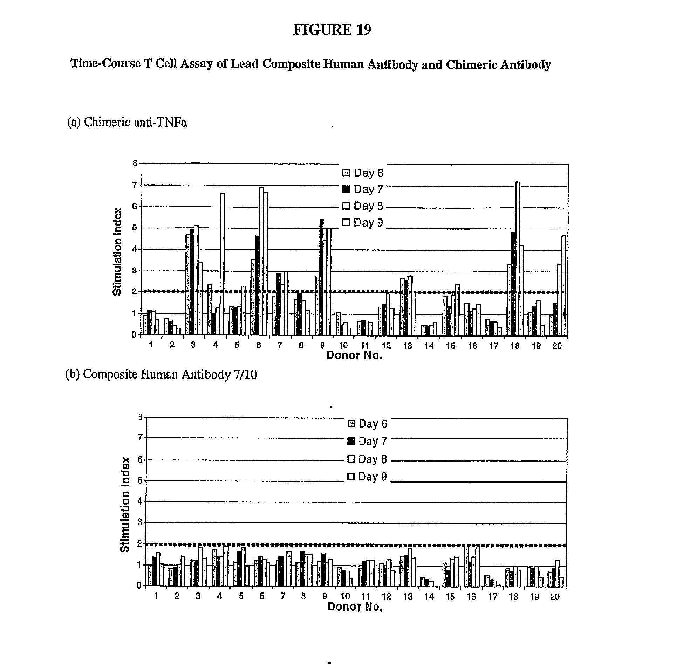

The results of the time-course assay are shown in Figure 19 and demonstrated that the chimeric antibody (Figure 19(a)) elicits a T cell response (SI >= 2) on at least one day in 10 of the 20 donors. In contrast, Composite Human Antibody (Figure 19(b)) failed to elicit a response in any of the donors at any time point. Therefore a non-immunogenic Composite Human Antibody was successfully constructed from segments of human antibodies using a mouse anti-TNFα antibody (A2) as reference.

### Example 8: Construction of a Composite Type I Ribosome Inhibitory Protein

Composite variants of the plant type I Ribosome Inhibitory Protein (RIP) bouganin (derived from *Bougainvillea spectabilis*) were generated using methods described in WO2005090579. The location of T cell epitopes in bouganin was tested by analysis of overlapping 15mer peptides as in W02005090579 and the peptides of SEQ ID 11-13 in table 6 (corresponding to residues 121-135, 130-144 and 148-162) were identified as epitopes. Bouganin was cloned from leaf tissue from a *Bougainvillea spectabilis* plant. mRNA was extracted using a polyA Tract System 1000 kit (Promega) from 100mg tissue as instructed by the manufacturer. cDNA was synthesised from the mRNA template using an AccessQuick RT-PCR system (Promega) with the following primers: ATGTACAACACTGTGTCATTTAAC and TTATTTGGAGCTTTTAAACTTAAGGATACC. The first primer additionally contains an ATG start codon and the second primer additionally contains a TAA stop codon. The PCR product was cloned using a T/A cloning system (pGEM T Easy, Promega) and several clones were sequenced to identify a correct clone orientated with the transcription direction of the T7 promoter contained within the vector.

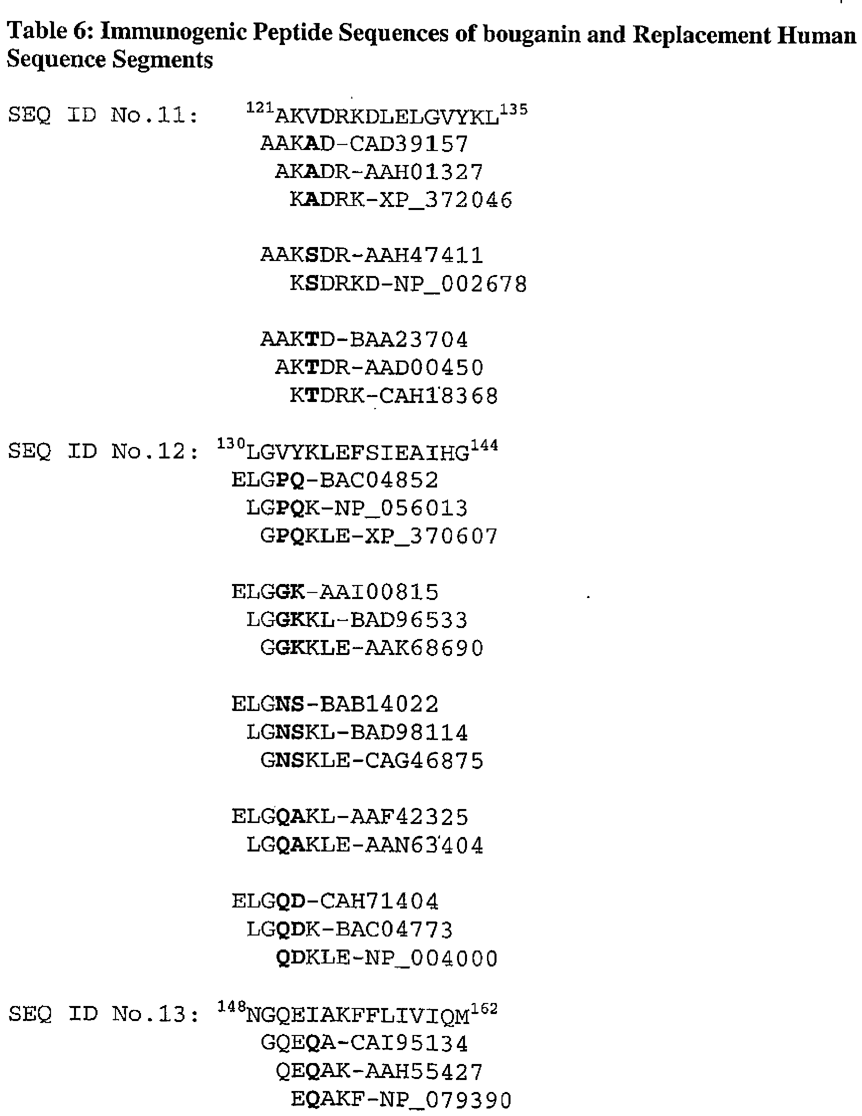

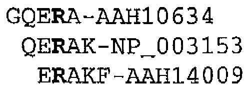

A series of variants were made containing the human sequence segments identified as shown in table 6. These were constructed using overlap PCR with a high fidelity polymerase (Expand Hi-Fi, Roche). The 5' and 3' primers were as above and the PCR products were cloned into the T/A cloning vector, as above, and correct clones identified that were orientated with the transcription direction of the T7 promoter. Clones were assayed for activity in a coupled transcription and translation reaction that included a control DNA expressing a luciferase gene (Luciferase T7 Control, Promega). Since bouganin is a ribosome inactivating protein, it significantly reduces the levels of translation of the luciferase gene and this reduction is conveniently assayed using a luciferase detection system such as Steady-Glo (Promega). Purified wild type or mutant bouganin plasmids were linearised with *Not* I and diluted to 10ng/µl. Luciferase T7 Control DNA was diluted to 125ng/µl. 1µl each DNA was mixed with 10µl TnT mix (Promega), 0.25µl Methionine and 0.25µl nuclease free water (supplied in TnT kit). Controls were wt bouganin and Luciferase T7 Control only. Reactions were undertaken in triplicate and incubated for 1 hour at 30°C. 5µl each reaction was transferred to a black walled 96 well luminometer plate and mixed with 45µl water and 50µl Steady-Glo reagent. Luminescence was read in a Wallac Microbeta Trilux luminometer. Activity was expressed as a percentage of the luminescence observed from the Luciferase T7 Control DNA alone.

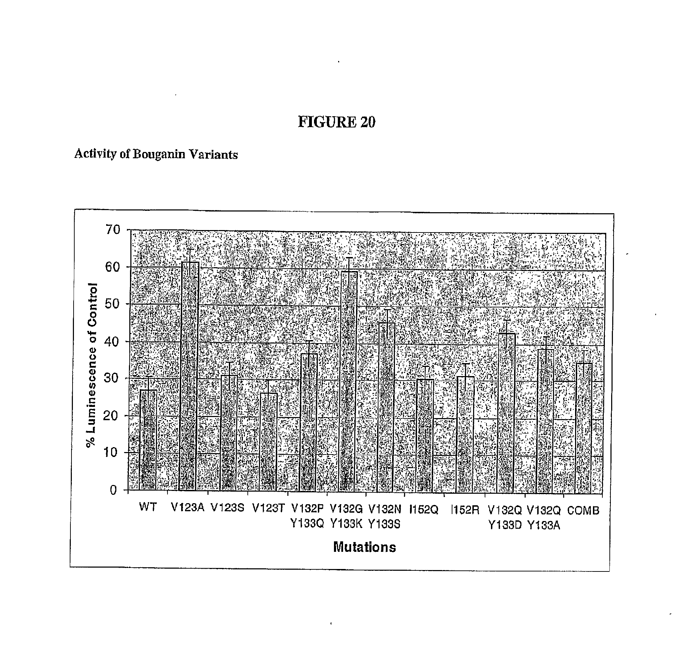

Figure 20 illustrates the activity profile of a number of different variants. This shows that the most active variants are: V123T in peptide 41; V132P/Y133Q in peptide 44; 1152Q in peptide 50. A combined mutant was made containing these 4 mutations and re-tested in the activity assay. vThe activity of this mutant is indicated by COMB in figure 20 and retains approximately 75% of the activity of the wt protein.

Peptides containing the human sequence segments within the active COMB variant corresponding to residues 121-135, 130-144 and 148-162 were synthesised and compared to the corresponding wild type peptides in a time-course T cell assay with human PBMCs from 20 healthy donors as described in example 7. The results showed that peptides containing human sequence segments induced no T cell proliferation in any donor at any time point whilst each of the wild type peptides induced proliferation with SI>2 in >10% of all donors for at least one time point.

### Example 9: Construction of a Composite Hirudin

Composite variants of the thrombin inhibitor hirudin (derived from *Hirudo medicinalis*) were generated using methods described in WO2004113386 using the protein with SEQ ID No 14 in table 7 as wild type. The location of T cell epitopes in hirudin was tested by analysis of overlapping 15mer peptides as in WO200411336 and the peptide 27-41 CILGSDGEKNQCVTG was shown to give a significant T cell response with human PBMCs from 20 healthy donors. The human sequence segment KCRH from human melanoma-associated antigen (AAN40505.1) was used to replace the hirudin residues at 26-29 using overlap PCR as in example 8 resulting in a variant hirudin molecule with 28/29IL changed to 28/29RH which retained full activity of the wild type hirudin using assays described in W02004113386. The modified peptide 27-41 CRHGSDGEKNQCVTG was tested together with the wild type peptide 27-41 CILGSDGEKNQCVTG in T cell assays as in example 8 demonstrating the loss of T cell epitope activity in the modified peptide.

### Example 10: Construction of Composite Human Anti-IgE Antibody with Tr Epitopes

VH and VL genes from the Composite Human Anti-IgE antibody of example 4 were cloned according to standard polymerase chain reaction (PCR) methods from Orlandi et al., *ibid* into separate plasmid vectors as templates for a VL- and VH-specific PCR using oligonucleotide primer pairs. Overlapping complementary sequences were introduced into the PCR products that combined during the subsequent fusion PCR to form the coding sequence either of a 20 amino acid (G₄S₁)₄ linker or, alternatively, a 20 amino acid sequence GGSNNLSCLTIPASANNGGS containing a 10 amino acid Tr epitope from the hepatitis C core protein (P19, MacDonald et al., Journal of Infectious Diseases, 185 (2002) p720-727) flanked each side by two asparagines residues and a GGS triplet. This final amplification step was performed with primer pairs for subsequent cleavage with the restriction enzymes *Eco*RV and *Bsp*E1 and cloning into the bluescript KS vector (Stratagene). Dimeric forms of the Composite Human anti-IgE single chain antibodies (scFvs) were then constructed by the method of Mack et al., Proc Natl Acad Sci U S A., 92 (1995) p7021-7025. The dimeric VL-linker-VH-VL-linker-VH fragment was subcloned into the *Eco*R1/*Sal*1 sites of the expression vector pEF-DHFR (Mack et al., ibid) and transfected into DHFR-deficient Chinese hamster ovary (CHO) cells by electroporation. Selection, gene amplification, and protein production were performed as described by Mach et al., *ibid*)*.* The dimeric scFv's were purified via the C-terminal histidine tails by affinity chromatography on a nickel-nitrilotriacetic acid (Ni-NTA) column (Qiagen) to give dimeric Fvs designated CHABIgEG4S1x4 ((G₄S₁)₄ linker between VL and VH) and CHABIgEHCVP19 (HCV Tr epitope between VL and VH).

Dimeric scFvs were subsequently tested in human T cell assays at 50µg/ml exactly as described by Hall et al., Blood 100 (2002) p4529-4536 using PBMCs from 20 healthy donors. The results showed no significant proliferation of T cell for either CHABIgEG4S1x4 or CHABIgEHCVP19 but showed a significant level of IL-10 production (SI>2) from 4 out of 20 donors stimulated with CHABIgEHCVP19 but not with CHABIgEG4S1x4 (SI>2 in 0 of 20 donors). This demonstrates the effect of a Tr epitope included within the antibody molecule for the induction of the immunosuppressive cytokine IL-10.

## Claims

1. An antibody variable region or antigen-binding fragment thereof, wherein the variable region comprises two or more segments of amino acid sequence from one or more other antibodies or antigen-binding fragments thereof, and wherein the two or more segments are neither whole CDRs nor whole framework regions.

2. An antibody variable region or antigen-binding fragment thereof as claimed in claim 1 wherein two or more segments of amino acid sequences are derived from human antibodies.

3. An antibody variable region or antigen-binding fragment thereof as claimed in claim 1 or claim 2, wherein the final antibody or antigen-binding molecule is devoid of helper T cell epitopes.

4. An antibody or antigen-binding molecule which comprises an antibody variable region or antigen-binding fragment thereof as claimed in any one of claims 1 to 3 wherein the final antibody or antigen-binding molecule contains additional amino acid sequences which provide for one or more regulatory T cell epitopes.

5. A pharmaceutical composition comprising one or more antibody variable regions or antigen-binding fragments thereof as claimed in any one of claims 1 to 3 or one or more antibodies or antigen-binding molecules as claimed in claim 4.

6. A method for generating an antibody variable region or antigen-binding fragment, wherein the method comprises the following steps;
(a) Generating a library of genes encoding antibody variable regions or antigen-binding fragments derived from two or more segments of amino acid sequence from other antibodies or other antigen-binding fragments wherein the two or more segments are neither whole CDRs nor whole framework regions;
(b) Expressing and screening the library of antibody variable regions or antigen-binding fragments for binding to one or more antigens of interest.

7. A method as claimed in claim 6 wherein the two or more segments of amino acid sequences used to generate the library are derived from human antibodies.

8. A method as claimed in claim 6 or claim 7 wherein the combined antibody variable region or antigen-binding fragments are screened to avoid helper T cell epitopes in the final antibody variable regions or antigen-binding fragments.

9. A method as claimed in any one of claims 6 to 8 wherein additional amino acid sequences which provide for one or more regulatory T cell epitopes are included in the final antibody variable regions or antigen-binding fragments.

10. A pharmaceutical composition as claimed in claim 5 which binds to human HER-2.

11. A pharmaceutical composition as claimed in claim 5 which binds to human Lewis Y antigen.

12. A pharmaceutical composition as claimed in claim 5 which binds to human IgE.

13. A pharmaceutical composition as claimed in claim 5 which binds to human TNFalpha.


SEQUENCE LISTING

<110>  ANTITOPE LIMITED
       BAKER, Matthew Paul
       JONES, Timothy David

<120>  Human Antibodies and Proteins

<130>  P40089EP1/PWC

<140>
<141>  2006-02-03

<150>  GB0502201.7
<151>  2005-02-03

<150>  GB0506945.5
<151>  2005-04-05

<150>  GB0503190.1
<151>  2005-02-16

<160>  600

<170>  PatentIn version 3.2

<210>  1
<211>  120
<212>  PRT
<213>  Homo sapiens

<400>  1

Glu Val Lys Leu Glu Glu Ser Gly Gly Gly Leu Val Gln Pro Gly Gly
1               5                   10                  15

Ser Met Lys Leu Ser Cys Val Ala Ser Gly Phe Ile Phe Ser Asn His
            20                  25                  30

Trp Met Asn Trp Val Arg Gln Ser Pro Glu Lys Gly Leu Glu Trp Val
        35                  40                  45

Ala Glu Ile Arg Ser Lys Ser Ile Asn Ser Ala Thr His Tyr Ala Glu
    50                  55                  60

Ser Val Lys Gly Arg Phe Thr Ile Ser Arg Asp Asp Ser Lys Ser Ala
65                  70                  75                  80

Val Tyr Leu Gln Met Thr Asp Leu Arg Thr Glu Asp Thr Gly Val Tyr
                85                  90                  95

Tyr Cys Ser Arg Asn Tyr Tyr Gly Ser Thr Tyr Asp Tyr Trp Gly Gln
            100                 105                 110

Gly Thr Thr Leu Thr Val Ser Ser
        115                 120


<210>  2
<211>  107
<212>  PRT

<213>  Homo sapiens

<400>  2

Asp Ile Leu Leu Thr Gln Ser Pro Ala Ile Leu Ser Val Ser Pro Gly
1               5                   10                  15

Glu Arg Val Ser Phe Ser Cys Arg Ala Ser Gln Phe Val Gly Ser Ser
            20                  25                  30

Ile His Trp Tyr Gln Gln Arg Thr Asn Gly Ser Pro Arg Leu Leu Ile
        35                  40                  45

Lys Tyr Ala Ser Glu Ser Met Ser Gly Ile Pro Ser Arg Phe Ser Gly
    50                  55                  60

Ser Gly Ser Gly Thr Asp Phe Thr Leu Ser Ile Asn Thr Val Glu Ser
65                  70                  75                  80

Glu Asp Ile Ala Asp Tyr Tyr Cys Gln Gln Ser His Ser Trp Pro Phe
                85                  90                  95

Thr Phe Gly Ser Gly Thr Asn Leu Glu Val Lys
            100                 105


<210>  3
<211>  120
<212>  PRT
<213>  Homo sapiens

<400>  3

Glu Val Gln Leu Val Glu Ser Gly Gly Gly Leu Val Gln Pro Gly Gly
1               5                   10                  15

Ser Leu Lys Leu Ser Cys Val Ala Ser Gly Phe Ile Phe Ser Asn His
            20                  25                  30

Trp Met Asn Trp Val Arg Gln Ala Pro Gly Lys Gly Leu Glu Trp Val
        35                  40                  45

Ala Glu Ile Arg Ser Lys Ser Ile Asn Ser Ala Thr His Tyr Ala Glu
    50                  55                  60

Ser Val Lys Gly Arg Phe Thr Ile Ser Arg Asp Asp Ser Lys Ser Ile
65                  70                  75                  80

Val Tyr Leu Gln Met Thr Asp Leu Arg Thr Glu Asp Thr Gly Val Tyr
                85                  90                  95

Tyr Cys Ser Arg Asn Tyr Tyr Gly Ser Thr Tyr Asp Tyr Trp Gly Gln
            100                 105                 110

Gly Thr Thr Val Thr Val Ser Ser

115                 120


<210>  4
<211>  107
<212>  PRT
<213>  Homo sapiens

<400>  4

Asp Ile Leu Leu Thr Gln Ser Pro Ala Ile Leu Ser Leu Ser Pro Gly
1               5                   10                  15


Glu Arg Ala Thr Leu Ser Cys Arg Ala Ser Gln Phe Val Gly Ser Ser
            20                  25                  30


Ile His Trp Tyr Gln Gln Lys Thr Asn Gln Ser Pro Lys Leu Leu Ile
        35                  40                  45


Lys Tyr Ala Ser Glu Ser Met Ser Gly Ile Pro Ser Arg Phe Ser Gly
    50                  55                  60


Ser Gly Ser Gly Thr Asp Phe Thr Leu Thr Ile Asn Ser Leu Glu Ser
65                  70                  75                  80


Glu Asp Ala Ala Asp Tyr Tyr Cys Gln Gln Ser His Ser Trp Pro Phe
                85                  90                  95


Thr Phe Gly Gln Gly Thr Asn Leu Glu Ile Lys
            100                 105


<210>  5
<211>  120
<212>  PRT
<213>  Homo sapiens

<400>  5

Glu Val Gln Leu Val Glu Ser Gly Gly Gly Leu Val Gln Pro Gly Gly
1               5                   10                  15


Ser Leu Lys Leu Ser Cys Val Ala Ser Gly Phe Ile Phe Ser Asn His
            20                  25                  30


Trp Met Asn Trp Val Arg Gln Ala Pro Gly Lys Gly Leu Glu Trp Val
        35                  40                  45


Ala Glu Ile Arg Ser Lys Ser Ile Asn Ser Ala Thr His Tyr Ala Glu
    50                  55                  60


Ser Val Lys Gly Arg Phe Thr Ile Ser Arg Asp Asp Ser Lys Ser Ile
65                  70                  75                  80


Val Tyr Leu Gln Met Asn Asp Leu Lys Thr Glu Asp Thr Gly Val Tyr
                85                  90                  95

Tyr Cys Ser Arg Asn Tyr Tyr Gly Ser Thr Tyr Asp Tyr Trp Gly Gln
            100                 105                 110

Gly Thr Thr Val Thr Val Ser Ser
        115                 120

<210>  6
<211>  120
<212>  PRT
<213>  Homo sapiens

<400>  6

Glu Val Gln Leu Val Glu Ser Gly Gly Gly Leu Val Gln Pro Gly Gly
1               5                   10                  15

Ser Leu Lys Leu Ser Cys Val Ala Ser Gly Phe Ile Phe Ser Asn His
            20                  25                  30

Trp Met Asn Trp Val Arg Gln Ala Pro Gly Lys Gly Leu Glu Trp Val
        35                  40                  45

Ala Glu Ile Arg Ser Lys Ser Ile Asn Ser Ala Thr His Tyr Ala Glu
    50                  55                  60

Ser Val Lys Gly Arg Phe Thr Ile Ser Arg Asp Asp Ser Lys Ser Ile
65                  70                  75                  80

Val Tyr Leu Gln Met Asn Ser Leu Lys Thr Glu Asp Thr Gly Val Tyr
                85                  90                  95

Tyr Cys Ser Arg Asn Tyr Tyr Gly Ser Thr Tyr Asp Tyr Trp Gly Gln
            100                 105                 110

Gly Thr Thr Val Thr Val Ser Ser
        115                 120

<210>  7
<211>  120
<212>  PRT
<213>  Homo sapiens

<400>  7

Glu Val Gln Leu Val Glu Ser Gly Gly Gly Leu Val Gln Pro Gly Gly
1               5                   10                  15

Ser Leu Lys Leu Ser Cys Ala Ala Ser Gly Phe Ile Phe Ser Asn His
            20                  25                  30

Trp Met Asn Trp Val Arg Gln Ala Pro Gly Lys Gly Leu Glu Trp Val
        35                  40                  45

Ala Glu Ile Arg Ser Lys Ser Ile Asn Ser Ala Thr His Tyr Ala Glu

50                  55                  60

Ser Val Lys Gly Arg Phe Thr Ile Ser Arg Asp Asp Ser Lys Ser Ile
65                  70                  75                  80

Val Tyr Leu Gln Met Asn Ser Leu Lys Thr Glu Asp Thr Gly Val Tyr
                85                  90                  95

Tyr Cys Ser Arg Asn Tyr Tyr Gly Ser Thr Tyr Asp Tyr Trp Gly Gln
            100                 105                 110

Gly Thr Thr Val Thr Val Ser Ser
        115                 120

<210>  8
<211>  120
<212>  PRT
<213>  Homo sapiens

<400>  8

Glu Val Gln Leu Val Glu Ser Gly Gly Gly Leu Val Gln Pro Gly Gly
1               5                   10                  15

Ser Leu Lys Leu Ser Cys Ala Ala Ser Gly Phe Ile Phe Ser Asn His
            20                  25                  30

Trp Met Asn Trp Val Arg Gln Ala Pro Gly Lys Gly Leu Glu Trp Val
        35                  40                  45

Ala Glu Ile Arg Ser Lys Ser Ile Asn Ser Ala Thr His Tyr Ala Glu
    50                  55                  60

Ser Val Lys Gly Arg Phe Thr Ile Ser Arg Asp Asp Ser Lys Ser Ile
65                  70                  75                  80

Ala Tyr Leu Gln Met Asn Ser Leu Lys Thr Glu Asp Thr Gly Val Tyr
                85                  90                  95

Tyr Cys Ser Arg Asn Tyr Tyr Gly Ser Thr Tyr Asp Tyr Trp Gly Gln
            100                 105                 110

Gly Thr Thr Val Thr Val Ser Ser
        115                 120

<210>  9
<211>  107
<212>  PRT
<213>  Homo sapiens

<400>  9

Asp Ile Leu Leu Thr Gln Ser Pro Ala Thr Leu Ser Leu Ser Pro Gly
1               5                   10                  15

Glu Arg Ala Thr Leu Ser Cys Arg Ala Ser Gln Phe Val Gly Ser Ser
            20                  25                  30

Ile His Trp Tyr Gln Gln Lys Thr Asn Gln Ser Pro Lys Leu Leu Ile
        35                  40                  45

Lys Tyr Ala Ser Glu Ser Met Ser Gly Ile Pro Ser Arg Phe Ser Gly
    50                  55                  60

Ser Gly Ser Gly Thr Asp Phe Thr Leu Thr Ile Asn Ser Leu Glu Ser
65                  70                  75                  80

Glu Asp Ala Ala Asp Tyr Tyr Cys Gln Gln Ser His Ser Trp Pro Phe
                85                  90                  95

Thr Phe Gly Gln Gly Thr Arg Leu Glu Ile Lys
            100                 105

<210>  10
<211>  107
<212>  PRT
<213>  Homo sapiens

<400>  10

Asp Ile Leu Leu Thr Gln Ser Pro Ala Thr Leu Ser Leu Ser Pro Gly
1               5                   10                  15

Glu Arg Ala Thr Leu Ser Cys Arg Ala Ser Gln Phe Val Gly Ser Ser
            20                  25                  30

Ile His Trp Tyr Gln Gln Lys Thr Asn Gln Ser Pro Lys Leu Leu Ile
        35                  40                  45

Lys Tyr Ala Ser Glu Ser Met Ser Gly Ile Pro Ser Arg Phe Ser Gly
    50                  55                  60

Ser Gly Ser Gly Thr Asp Phe Thr Leu Thr Ile Asn Ser Leu Glu Ala
65                  70                  75                  80

Glu Asp Ala Ala Asp Tyr Tyr Cys Gln Gln Ser His Ser Trp Pro Phe
                85                  90                  95

Thr Phe Gly Gln Gly Thr Arg Leu Glu Ile Lys
            100                 105

<210>  11
<211>  107
<212>  PRT
<213>  Homo sapiens

<400>  11

Asp Ile Leu Leu Thr Gln Ser Pro Ala Thr Leu Ser Leu Ser Pro Gly

1               5                   10                  15

Glu Arg Ala Thr Leu Ser Cys Arg Ala Ser Gln Phe Val Gly Ser Ser
            20                  25                  30

Ile His Trp Tyr Gln Gln Lys Thr Asp Gln Ser Pro Lys Leu Leu Ile
        35                  40                  45

Lys Tyr Ala Ser Glu Ser Met Ser Gly Ile Pro Ser Arg Phe Ser Gly
    50                  55                  60

Ser Gly Ser Gly Thr Asp Phe Thr Leu Thr Ile Asn Ser Leu Glu Ala
65                  70                  75                  80

Glu Asp Ala Ala Asp Tyr Tyr Cys Gln Gln Ser His Ser Trp Pro Phe
                85                  90                  95

Thr Phe Gly Gln Gly Thr Arg Leu Glu Ile Lys
            100                 105

<210>  12
<211>  38
<212>  DNA
<213>  Homo sapiens

<400>  12
gttgctacgc gtgtccactc cgaagtgaag cttgagga                              38

<210>  13
<211>  40
<212>  DNA
<213>  Homo sapiens

<400>  13
gtctggagga ggcttggtgc aacctggagg atccatgaaa                            40

<210>  14
<211>  40
<212>  DNA
<213>  Homo sapiens

<400>  14
ctctcctgtg ttgcctctgg attcattttc agtaaccact                            40

<210>  15
<211>  40
<212>  DNA
<213>  Homo sapiens

<400>  15
ggatgaactg ggtccgccag tctccagaga aggggcttga                            40

<210>  16
<211>  40
<212>  DNA
<213>  Homo sapiens

<400>  16
gtgggttgct gaaattagat cgaaatctat taattctgca                              40


<210>  17
<211>  40
<212>  DNA
<213>  Homo sapiens

<400>  17
acacattatg cggagtctgt gaaagggagg ttcaccatct                              40


<210>  18
<211>  40
<212>  DNA
<213>  Homo sapiens

<400>  18
caagagatga ttccaaaagt gctgtctacc tgcaaatgac                              40


<210>  19
<211>  40
<212>  DNA
<213>  Homo sapiens

<400>  19
cgacttaaga actgaagaca ctggcgttta ttactgtagc                              40


<210>  20
<211>  40
<212>  DNA
<213>  Homo sapiens

<400>  20
aggaactatt acggttcaac ctacgactac tggggccaag                              40


<210>  21
<211>  38
<212>  DNA
<213>  Homo sapiens

<400>  21
gcaccactct cacagtctcc tcaggtaagc tttctggg                                38


<210>  22
<211>  18
<212>  DNA
<213>  Homo sapiens

<400>  22
cccagaaagc ttacctga                                                      18


<210>  23
<211>  40
<212>  DNA
<213>  Homo sapiens

<400>  23
ggagactgtg agagtggtgc cttggcccca gtagtcgtag                              40


<210>  24

<211>  40
<212>  DNA
<213>  Homo sapiens

<400>  24
gttgaaccgt aatagttcct gctacagtaa taaacgccag                              40


<210>  25
<211>  40
<212>  DNA
<213>  Homo sapiens

<400>  25
tgtcttcagt tcttaagtcg gtcatttgca ggtagacagc                              40


<210>  26
<211>  40
<212>  DNA
<213>  Homo sapiens

<400>  26
acttttggaa tcatctcttg agatggtgaa cctccctttc                              40


<210>  27
<211>  40
<212>  DNA
<213>  Homo sapiens

<400>  27
acagactccg cataatgtgt tgcagaatta atagatttcg                              40


<210>  28
<211>  40
<212>  DNA
<213>  Homo sapiens

<400>  28
atctaatttc agcaacccac tcaagcccct tctctggaga                              40


<210>  29
<211>  40
<212>  DNA
<213>  Homo sapiens

<400>  29
ctggcggacc cagttcatcc agtggttact gaaaatgaat                              40


<210>  30
<211>  40
<212>  DNA
<213>  Homo sapiens

<400>  30
ccagaggcaa cacaggagag tttcatggat cctccaggtt                              40


<210>  31
<211>  40
<212>  DNA
<213>  Homo sapiens

<400>  31

gcaccaagcc tcctccagac tcctcaagct tcacttcgga                                40

<210>  32
<211>  18
<212>  DNA
<213>  Homo sapiens

<400>  32
gtggacacgc gtagcaac                                                        18

<210>  33
<211>  40
<212>  DNA
<213>  Homo sapiens

<400>  33
tcccaggcgc gcgatgtgac atcctgctga cacaatctcc                                40

<210>  34
<211>  40
<212>  DNA
<213>  Homo sapiens

<400>  34
agccatcctg tctgtgagtc caggagaaag agtcagtttc                                40

<210>  35
<211>  40
<212>  DNA
<213>  Homo sapiens

<400>  35
tcctgcaggg ccagtcagtt cgttggctca agcatacact                                40

<210>  36
<211>  40
<212>  DNA
<213>  Homo sapiens

<400>  36
ggtatcagca gagaacaaat ggttctccaa ggcttctcat                                40

<210>  37
<211>  40
<212>  DNA
<213>  Homo sapiens

<400>  37
aaagtatgct tctgagtcta tgtctgggat ctcttccagg                                40

<210>  38
<211>  40
<212>  DNA
<213>  Homo sapiens

<400>  38
tttagtggca gtggatcagg gacagatttt actcttagca                                40

<210>  39
<211>  40

<212>  DNA
<213>  Homo sapiens

<400>  39
tcaacactgt ggagtctgaa gatattgcag attattactg                              40


<210>  40
<211>  40
<212>  DNA
<213>  Homo sapiens

<400>  40
tcaacaaagt catagttggc cgttcacgtt cggttctggg                              40


<210>  41
<211>  40
<212>  DNA
<213>  Homo sapiens

<400>  41
acaaatttgg aagtaaaacg tgagtagaat ttaaactttg                              40


<210>  42
<211>  23
<212>  DNA
<213>  Homo sapiens

<400>  42
cttcctcagt tggatcctgg cag                                                23


<210>  43
<211>  43
<212>  DNA
<213>  Homo sapiens

<400>  43
ctgccaggat ccaactgagg aagcaaagtt taaattctac tca                          43


<210>  44
<211>  40
<212>  DNA
<213>  Homo sapiens

<400>  44
cgttttactt ccaaatttgt cccagaaccg aacgtgaacg                              40


<210>  45
<211>  40
<212>  DNA
<213>  Homo sapiens

<400>  45
gccaactatg actttgttga cagtaataat ctgcaatatc                              40


<210>  46
<211>  40
<212>  DNA
<213>  Homo sapiens

<400>  46
ttcagactcc acagtgttga tgctaagagt aaaatctgtc                              40

<210>  47
<211>  40
<212>  DNA
<213>  Homo sapiens

<400>  47
cctgatccac tgccactaaa cctggaagag atcccagaca                              40


<210>  48
<211>  40
<212>  DNA
<213>  Homo sapiens

<400>  48
tagactcaga agcatacttt atgagaagcc ttggagaacc                              40


<210>  49
<211>  40
<212>  DNA
<213>  Homo sapiens

<400>  49
atttgttctc tgctgatacc agtgtatgct tgagccaacg                              40


<210>  50
<211>  40
<212>  DNA
<213>  Homo sapiens

<400>  50
aactgactgg ccctgcagga gaaactgact ctttctcctg                              40


<210>  51
<211>  40
<212>  DNA
<213>  Homo sapiens

<400>  51
gactcacaga caggatggct ggagattgtg tcagcaggat                              40


<210>  52
<211>  20
<212>  DNA
<213>  Homo sapiens

<400>  52
gtcacatcgc gcgcctggga                                                    20


<210>  53
<211>  38
<212>  DNA
<213>  Homo sapiens

<400>  53
gttgctacgc gtgtccactc cgaagtgcag cttgtgga                                38


<210>  54
<211>  40
<212>  DNA

<213>  Homo sapiens

<400>  54
gtctggagga ggcttggtgc aacctggagg atccttgaaa                              40


<210>  55
<211>  40
<212>  DNA
<213>  Homo sapiens

<400>  55
ctctcctgtg ttgcctctgg attcattttc agtaaccact                              40


<210>  56
<211>  40
<212>  DNA
<213>  Homo sapiens

<400>  56
ggatgaactg ggtccgccag gctccaggga aggggcttga                              40


<210>  57
<211>  40
<212>  DNA
<213>  Homo sapiens

<400>  57
gtgggttgct gaaattagat cgaaatctat taattctgca                              40


<210>  58
<211>  40
<212>  DNA
<213>  Homo sapiens

<400>  58
acacattatg cggagtctgt gaaagggagg ttcaccatct                              40


<210>  59
<211>  40
<212>  DNA
<213>  Homo sapiens

<400>  59
caagagatga ttccaaaagt attgtctacc tgcaaatgac                              40


<210>  60
<211>  40
<212>  DNA
<213>  Homo sapiens

<400>  60
cgacttaaga actgaagaca ctggcgttta ttactgtagc                              40


<210>  61
<211>  40
<212>  DNA
<213>  Homo sapiens

<400>  61
aggaactatt acggttcaac ctacgactac tggggccaag                              40

<210>  62
<211>  38
<212>  DNA
<213>  Homo sapiens

<400>  62
gcaccactgt cacagtctcc tcaggtaagc tttctggg                              38


<210>  63
<211>  18
<212>  DNA
<213>  Homo sapiens

<400>  63
cccagaaagc ttacctga                                                    18


<210>  64
<211>  40
<212>  DNA
<213>  Homo sapiens

<400>  64
ggagactgtg acagtggtgc cttggcccca gtagtcgtag                            40


<210>  65
<211>  40
<212>  DNA
<213>  Homo sapiens

<400>  65
gttgaaccgt aatagttcct gctacagtaa taaacgccag                            40


<210>  66
<211>  40
<212>  DNA
<213>  Homo sapiens

<400>  66
tgtcttcagt tcttaagtcg gtcatttgca ggtagacaat                            40


<210>  67
<211>  40
<212>  DNA
<213>  Homo sapiens

<400>  67
cacttttgga atcatctctt gagatggtga acctcctttc                            40


<210>  68
<211>  40
<212>  DNA
<213>  Homo sapiens

<400>  68
acagactccg cataatgtgt tgcagaatta atagatttcg                            40


<210>  69
<211>  40
<212>  DNA
<213>  Homo sapiens

<400>  69
atctaatttc agcaacccac tcaagcccct tccctggagc                              40


<210>  70
<211>  40
<212>  DNA
<213>  Homo sapiens

<400>  70
ctggcggacc cagttcatcc agtggttact gaaaatgaat                              40


<210>  71
<211>  40
<212>  DNA
<213>  Homo sapiens

<400>  71
ccagaggcaa cacaggagag tttcaaggat cctccaggtt                              40


<210>  72
<211>  40
<212>  DNA
<213>  Homo sapiens

<400>  72
gcaccaagcc tcctccagac tccacaagct gcacttcgga                              40


<210>  73
<211>  18
<212>  DNA
<213>  Homo sapiens

<400>  73
gtggacacgc gtagcaac                                                      18


<210>  74
<211>  40
<212>  DNA
<213>  Homo sapiens

<400>  74
tcccaggcgc gcgatgtgac atcctgctga cacaatctcc                              40


<210>  75
<211>  40
<212>  DNA
<213>  Homo sapiens

<400>  75
agccatcctg tctctgagtc caggagaaag agccactctc                              40


<210>  76
<211>  40
<212>  DNA
<213>  Homo sapiens

<400>  76
tcctgcaggg ccagtcagtt cgttggctca agcatacact                              40

<210>  77
<211>  40
<212>  DNA
<213>  Homo sapiens

<400>  77
ggtatcagca gaaaacaaat cagtctccaa agcttctcat                              40


<210>  78
<211>  40
<212>  DNA
<213>  Homo sapiens

<400>  78
aaagtatgct tctgagtcta tgtctgggat ctcttccagg                              40


<210>  79
<211>  40
<212>  DNA
<213>  Homo sapiens

<400>  79
tttagtggca gtggatcagg gacagatttt actcttacca                              40


<210>  80
<211>  40
<212>  DNA
<213>  Homo sapiens

<400>  80
tcaacagtct ggagtctgaa gatgctgcag attattactg                              40


<210>  81
<211>  40
<212>  DNA
<213>  Homo sapiens

<400>  81
tcaacaaagt catagttggc cgttcacgtt cggtcaaggg                              40


<210>  82
<211>  40
<212>  DNA
<213>  Homo sapiens

<400>  82
acaaatttgg aaataaaacg tgagtagaat ttaaactttg                              40


<210>  83
<211>  23
<212>  DNA
<213>  Homo sapiens

<400>  83
cttcctcagt tggatcctgg cag                                                23


<210>  84
<211>  43
<212>  DNA
<213>  Homo sapiens

<400>  84
ctgccaggat ccaactgagg aagcaaagtt taaattctac tca                          43


<210>  85
<211>  40
<212>  DNA
<213>  Homo sapiens

<400>  85
cgttttattt ccaaatttgt cccttgaccg aacgtgaacg                              40


<210>  86
<211>  40
<212>  DNA
<213>  Homo sapiens

<400>  86
gccaactatg actttgttga cagtaataat ctgcagcatc                              40


<210>  87
<211>  40
<212>  DNA
<213>  Homo sapiens

<400>  87
ttcagactcc agactgttga tggtaagagt aaaatctgtc                              40


<210>  88
<211>  40
<212>  DNA
<213>  Homo sapiens

<400>  88
cctgatccac tgccactaaa cctggaagag atcccagaca                              40


<210>  89
<211>  40
<212>  DNA
<213>  Homo sapiens

<400>  89
tagactcaga agcatacttt atgagaagct ttggagactg                              40


<210>  90
<211>  40
<212>  DNA
<213>  Homo sapiens

<400>  90
atttgttttc tgctgatacc agtgtatgct tgagccaacg                              40


<210>  91
<211>  40
<212>  DNA
<213>  Homo sapiens

<400>  91
aactgactgg ccctgcagga gagagtggct ctttctcctg                              40


<210>  92

<211>  40
<212>  DNA
<213>  Homo sapiens

<400>  92
gactcagaga caggatggct ggagattgtg tcagcaggat                              40


<210>  93
<211>  20
<212>  DNA
<213>  Homo sapiens

<400>  93
gtcacatcgc gcgcctggga                                                    20


<210>  94
<211>  25
<212>  DNA
<213>  Homo sapiens

<400>  94
caaatgaacg acttaaaaac tgaag                                              25


<210>  95
<211>  25
<212>  DNA
<213>  Homo sapiens

<400>  95
cttcagtttt taagtcgttc atttg                                              25


<210>  96
<211>  25
<212>  DNA
<213>  Homo sapiens

<400>  96
caaatgaaca gcttaaaaac tgaag                                              25


<210>  97
<211>  25
<212>  DNA
<213>  Homo sapiens

<400>  97
cttcagtttt taagctgttc atttg                                              25


<210>  98
<211>  20
<212>  DNA
<213>  Homo sapiens

<400>  98
ctctcctgtg ctgcctctgg                                                    20


<210>  99
<211>  20
<212>  DNA
<213>  Homo sapiens

<400>  99

ccagaggcag cacaggagag                                                20

<210>  100
<211>  20
<212>  DNA
<213>  Homo sapiens

<400>  100
caaaagtatt gcctacctgc                                                20

<210>  101
<211>  20
<212>  DNA
<213>  Homo sapiens

<400>  101
cgaggtaggc aatacttttg                                                20

<210>  102
<211>  54
<212>  DNA
<213>  Homo sapiens

<400>  102
tcccaggcgc gcgatgtgac atcctgctga cacaatctcc agccaccctg tctc          54

<210>  103
<211>  66
<212>  DNA
<213>  Homo sapiens

<400>  103
ctgccaggat ccaactgagg aagcaaagtt taaattctac tcacgtttta tttccaatct    60

tgtccc                                                               66

<210>  104
<211>  21
<212>  DNA
<213>  Homo sapiens

<400>  104
cagtctggag gctgaagatg c                                              21

<210>  105
<211>  21
<212>  DNA
<213>  Homo sapiens

<400>  105
cgatcttcag cctccagact g                                              21

<210>  106
<211>  21
<212>  DNA
<213>  Homo sapiens

<400>  106
gcagaaaaca gatcagtctc c                                              21

<210>  107
<211>  21
<212>  DNA
<213>  Homo sapiens

<400>  107
ggagactgat ctgttttctg a                                                21


<210>  108
<211>  9
<212>  PRT
<213>  Homo sapiens

<400>  108

Asp Phe Leu Ser Gly Tyr Leu Asp Tyr
1               5


<210>  109
<211>  9
<212>  PRT
<213>  Homo sapiens

<400>  109

Val Arg Gly Ser Gly Ser Phe Asp Tyr
1               5


<210>  110
<211>  9
<212>  PRT
<213>  Homo sapiens

<400>  110

Asp Arg Gly Gly Asn Tyr Phe Asp Tyr
1               5


<210>  111
<211>  9
<212>  PRT
<213>  Homo sapiens

<400>  111

Met Tyr Asn Trp Asn Phe Phe Asp Tyr
1               5


<210>  112
<211>  9
<212>  PRT
<213>  Homo sapiens

<400>  112

Ala Gly Leu Gly Met Ile Phe Asp Tyr
1               5


<210>  113
<211>  9
<212>  PRT
<213>  Homo sapiens

<400>  113

Arg Gly Phe Asn Gly Gln Leu Ile Phe
1               5


<210>  114
<211>  9
<212>  PRT
<213>  Homo sapiens

<400>  114

Ala Leu Thr Gly Asp Ala Phe Asp Ile
1               5


<210>  115
<211>  9
<212>  PRT
<213>  Homo sapiens

<400>  115

Thr Lys Leu Asp Trp Tyr Phe Asp Leu
1               5


<210>  116
<211>  9
<212>  PRT
<213>  Homo sapiens

<400>  116

Arg Tyr Gly Gly Phe Tyr Phe Asp Tyr
1               5


<210>  117
<211>  9
<212>  PRT
<213>  Homo sapiens

<400>  117

Gly Tyr Ser Asn Glu Gly Met Asp Val
1               5


<210>  118
<211>  9
<212>  PRT
<213>  Homo sapiens

<400>  118

Ser Trp Asp Gly Tyr Ser Tyr Ile Tyr
1               5


<210>  119
<211>  9
<212>  PRT
<213>  Homo sapiens

<400>  119

Gln Met Gly Ala Glu Tyr Phe Gln His
1               5

<210>  120
<211>  9
<212>  PRT
<213>  Homo sapiens

<400>  120

Asp Met Ser Leu Asp Ala Phe Asp Ile
1               5

<210>  121
<211>  9
<212>  PRT
<213>  Homo sapiens

<400>  121

Gly Ser Val Gly Ala Thr Leu Gly Glu
1               5

<210>  122
<211>  9
<212>  PRT
<213>  Homo sapiens

<400>  122

Tyr Gly Asp Tyr His Tyr Phe Asp Tyr
1               5

<210>  123
<211>  9
<212>  PRT
<213>  Homo sapiens

<400>  123

Gly Val Gly Ser Ser Gly Trp Asp His
1               5

<210>  124
<211>  9
<212>  PRT
<213>  Homo sapiens

<400>  124

Lys Gly Ser Leu Tyr Tyr Phe Asp Tyr
1               5

<210>  125
<211>  9
<212>  PRT
<213>  Homo sapiens

<400>  125

Pro Asn Trp Asn Asp Ala Phe Asp Ile

1                   5


<210>  126
<211>  9
<212>  PRT
<213>  Homo sapiens

<400>  126

Arg Gly Ile Pro His Ala Phe Asp Ile
1                   5


<210>  127
<211>  9
<212>  PRT
<213>  Homo sapiens

<400>  127

Pro Pro Glu Val Glu Ser Leu Arg Ser
1                   5


<210>  128
<211>  9
<212>  PRT
<213>  Homo sapiens

<400>  128

Pro Pro Glu Val Glu Ser Leu Arg Ser
1                   5


<210>  129
<211>  9
<212>  PRT
<213>  Homo sapiens

<400>  129

Pro Pro Glu Val Glu Ser Leu Arg Ser
1                   5


<210>  130
<211>  9
<212>  PRT
<213>  Homo sapiens

<400>  130

Pro Pro Glu Val Glu Ser Leu Arg Ser
1                   5


<210>  131
<211>  9
<212>  PRT
<213>  Homo sapiens

<400>  131

Pro Pro Glu Val Glu Ser Leu Arg Ser
1                   5

<210>  132
<211>  9
<212>  PRT
<213>  Homo sapiens

<400>  132

Pro Pro Glu Val Glu Ser Leu Arg Ser
1               5


<210>  133
<211>  9
<212>  PRT
<213>  Homo sapiens

<400>  133

Pro Pro Glu Val Glu Ser Leu Arg Ser
1               5


<210>  134
<211>  9
<212>  PRT
<213>  Homo sapiens

<400>  134

Pro Pro Glu Val Glu Ser Leu Arg Ser
1               5


<210>  135
<211>  8
<212>  PRT
<213>  Homo sapiens

<400>  135

Asp Leu Ala Ala Ala Arg Leu Phe
1               5


<210>  136
<211>  9
<212>  PRT
<213>  Homo sapiens

<400>  136

Gln Gly Thr Ile Ala Gly Ile Arg His
1               5


<210>  137
<211>  9
<212>  PRT
<213>  Homo sapiens

<400>  137

Glu Asp Tyr Tyr Tyr Gly Met Asp Val
1               5


<210>  138

<211>  9
<212>  PRT
<213>  Homo sapiens

<400>  138

Asp Pro Ile Asn Trp Tyr Phe Asp Leu
1               5


<210>  139
<211>  9
<212>  PRT
<213>  Homo sapiens

<400>  139

Asp Arg Ala Ala Gly Asp Arg Asp Tyr
1               5


<210>  140
<211>  9
<212>  PRT
<213>  Homo sapiens

<400>  140

His Gln Met Tyr Ser Asn Ser Asp Tyr
1               5


<210>  141
<211>  9
<212>  PRT
<213>  Homo sapiens

<400>  141

Ser Tyr Asp Phe Ala Trp Phe Ala Tyr
1               5


<210>  142
<211>  9
<212>  PRT
<213>  Homo sapiens

<400>  142

Gln Gly Thr Ile Ala Gly Ile Arg His
1               5


<210>  143
<211>  9
<212>  PRT
<213>  Homo sapiens

<400>  143

Val Leu Gly Ile Ile Ala Ala Asp His
1               5


<210>  144
<211>  9
<212>  PRT

<213>  Homo sapiens

<400>  144

Asp Leu Thr Gly Asp Ala Phe Asp Ile
1               5


<210>  145
<211>  9
<212>  PRT
<213>  Homo sapiens

<400>  145

Ser Cys Gly Ser Gln Tyr Phe Asp Tyr
1               5


<210>  146
<211>  9
<212>  PRT
<213>  Homo sapiens

<400>  146

Leu Trp Asn Trp Asp Ala Phe Asp Ile
1               5


<210>  147
<211>  9
<212>  PRT
<213>  Homo sapiens

<400>  147

Asp Ile Met Thr Trp Gly Phe Asp Tyr
1               5


<210>  148
<211>  9
<212>  PRT
<213>  Homo sapiens

<400>  148

Ser Asn Trp Tyr Trp Tyr Phe Asp Leu
1               5


<210>  149
<211>  9
<212>  PRT
<213>  Homo sapiens

<400>  149

Asn Leu Ile Ala Gly Cys Ile Asp Val
1               5


<210>  150
<211>  9
<212>  PRT
<213>  Homo sapiens

<400>  150

Gly Gly Lys Gly Gly Glu Phe Asp Asp
1               5


<210>  151
<211>  9
<212>  PRT
<213>  Homo sapiens

<400>  151

Asp Ser Gly Asn Tyr Arg Ile Asp Tyr
1               5


<210>  152
<211>  9
<212>  PRT
<213>  Homo sapiens

<400>  152

Asp Pro Arg Leu Asp Ala Phe Asp Ile
1               5


<210>  153
<211>  9
<212>  PRT
<213>  Homo sapiens

<400>  153

Gly Tyr Ser Tyr Pro Val Trp Gly Arg
1               5


<210>  154
<211>  9
<212>  PRT
<213>  Homo sapiens

<400>  154

Leu Val Gly Asn Ser Trp Leu Asp Tyr
1               5


<210>  155
<211>  9
<212>  PRT
<213>  Homo sapiens


<220>
<221>  misc_feature
<222>  (3)..(3)
<223>  Xaa can be any naturally occurring amino acid

<400>  155

Asp Leu Xaa Gly Leu Val Val Glu Tyr
1               5


<210>  156

<211>  9
<212>  PRT
<213>  Homo sapiens

<400>  156

Lys Val Ser Leu Ser Ala Phe Asp Ile
1               5


<210>  157
<211>  9
<212>  PRT
<213>  Homo sapiens

<400>  157

Arg Gly Asp Ala Met Tyr Phe Asp Val
1               5


<210>  158
<211>  9
<212>  PRT
<213>  Homo sapiens

<400>  158

Asp Pro Asn Pro Trp Tyr Phe Asp Leu
1               5


<210>  159
<211>  9
<212>  PRT
<213>  Homo sapiens

<400>  159

Asp Tyr Gly Asp Tyr Ala Phe Asp Ile
1               5


<210>  160
<211>  9
<212>  PRT
<213>  Homo sapiens

<400>  160

Ser Ala His Ser Asp Ala Phe Asp Met
1               5


<210>  161
<211>  9
<212>  PRT
<213>  Homo sapiens

<400>  161

Leu Glu Gly Leu Gly Trp Phe Asp Pro
1               5


<210>  162
<211>  9
<212>  PRT

<213>  Homo sapiens

<400>  162

Arg Ser Asp Tyr Gly Ala Ile Asp Tyr
1               5


<210>  163
<211>  9
<212>  PRT
<213>  Homo sapiens

<400>  163

Asn Leu Gly Phe Tyr His Met Asp Val
1               5


<210>  164
<211>  9
<212>  PRT
<213>  Homo sapiens

<400>  164

Glu Ala Arg Gly Gly Gly Gly Glu Tyr
1               5


<210>  165
<211>  9
<212>  PRT
<213>  Homo sapiens

<400>  165

Glu Gly Trp Ile Ser Ala Leu Asn Gly
1               5


<210>  166
<211>  9
<212>  PRT
<213>  Homo sapiens

<400>  166

Glu Gly Glu Gly Glu Tyr Phe Asp Tyr
1               5


<210>  167
<211>  9
<212>  PRT
<213>  Homo sapiens

<400>  167

Glu Arg Thr Ser Gly Asp Phe Asp Phe
1               5


<210>  168
<211>  9
<212>  PRT
<213>  Homo sapiens

<400>  168

Asn Ser Pro Gly Ala Thr Phe Glu Ser
1               5


<210>  169
<211>  9
<212>  PRT
<213>  Homo sapiens

<400>  169

Gly Asn Gly Gln Lys Cys Phe Asp Tyr
1               5


<210>  170
<211>  9
<212>  PRT
<213>  Homo sapiens

<400>  170

Arg Gly Ser Leu Gln Tyr Leu Asp Tyr
1               5


<210>  171
<211>  9
<212>  PRT
<213>  Homo sapiens

<400>  171

Asn Asn Gly Ser Tyr Tyr Phe Asp Tyr
1               5


<210>  172
<211>  9
<212>  PRT
<213>  Homo sapiens

<400>  172

Gly Ser Asp Tyr Ser Asn Phe Ala Tyr
1               5


<210>  173
<211>  9
<212>  PRT
<213>  Homo sapiens

<400>  173

Ser Thr His Arg Ser Ala Phe Asp Val
1               5


<210>  174
<211>  9
<212>  PRT
<213>  Homo sapiens

<400>  174

Glu Gly Val His Lys Asn Phe Asp His
1               5

<210>  175
<211>  9
<212>  PRT
<213>  Homo sapiens

<400>  175

Leu Ser Arg Ala Gly Gly Phe Asp Ile
1               5

<210>  176
<211>  9
<212>  PRT
<213>  Homo sapiens

<400>  176

Arg Met Pro Ala Val Ala Phe Asp Tyr
1               5

<210>  177
<211>  9
<212>  PRT
<213>  Homo sapiens

<400>  177

Arg Met Pro Ala Val Ala Phe Asp Tyr
1               5

<210>  178
<211>  9
<212>  PRT
<213>  Homo sapiens

<400>  178

Arg Met Arg Ala Val Ala Phe Asp Tyr
1               5

<210>  179
<211>  9
<212>  PRT
<213>  Homo sapiens

<400>  179

Arg Met Pro Ala Val Ala Phe Asp Tyr
1               5

<210>  180
<211>  9
<212>  PRT
<213>  Homo sapiens

<400>  180

Asp Tyr Gly Gly Asn Pro Ala Glu Leu
1               5

<210>  181
<211>  9
<212>  PRT
<213>  Homo sapiens

<400>  181

Gly Pro Thr Cys Ser Gly Gly Ser Cys
1               5


<210>  182
<211>  9
<212>  PRT
<213>  Homo sapiens

<400>  182

Arg Lys Gly Ala Ala His Phe Asp Tyr
1               5


<210>  183
<211>  9
<212>  PRT
<213>  Homo sapiens

<400>  183

Glu Glu Val Gly Gly Tyr Phe Gln His
1               5


<210>  184
<211>  9
<212>  PRT
<213>  Homo sapiens

<400>  184

Asp Phe Asp Gly Gly Ser Phe Asp Tyr
1               5


<210>  185
<211>  9
<212>  PRT
<213>  Homo sapiens

<400>  185

Asp Phe Asp Gly Gly Ser Leu Asp Tyr
1               5


<210>  186
<211>  9
<212>  PRT
<213>  Homo sapiens

<400>  186

Asp Phe Asp Gly Gly Ser Phe Asp Tyr
1               5

<210>  187
<211>  9
<212>  PRT
<213>  Homo sapiens

<400>  187

Lys Val Pro Ser His Gly Met Asp Tyr
1               5


<210>  188
<211>  9
<212>  PRT
<213>  Homo sapiens

<400>  188

Lys Val Pro Ser His Gly Met Asp Tyr
1               5


<210>  189
<211>  9
<212>  PRT
<213>  Homo sapiens

<400>  189

Lys Val Pro Ser His Gly Met Asp Tyr
1               5


<210>  190
<211>  9
<212>  PRT
<213>  Homo sapiens

<400>  190

Lys Val Pro Ser His Gly Met Asp Tyr
1               5


<210>  191
<211>  9
<212>  PRT
<213>  Homo sapiens

<400>  191

Gln Pro Leu Ala Arg His Phe Asp Pro
1               5


<210>  192
<211>  9
<212>  PRT
<213>  Homo sapiens

<400>  192

Gly Pro Leu Met Arg Trp Phe Asp Asp
1               5


<210>  193
<211>  9

<212>  PRT
<213>  Homo sapiens

<400>  193

Val Ala Val Ala Gly Gly Phe Asp Pro
1               5


<210>  194
<211>  9
<212>  PRT
<213>  Homo sapiens

<400>  194

Gly Val Glu Val Ala Gly Thr Ala Ser
1               5


<210>  195
<211>  9
<212>  PRT
<213>  Homo sapiens

<400>  195

Tyr Tyr Glu Ser Ser Ala Gly Pro Pro
1               5


<210>  196
<211>  9
<212>  PRT
<213>  Homo sapiens

<400>  196

Glu Ile Pro Arg Gly Gly Ser Cys Tyr
1               5


<210>  197
<211>  9
<212>  PRT
<213>  Homo sapiens

<400>  197

Glu Ile Pro Arg Gly Gly Ser Cys Tyr
1               5


<210>  198
<211>  9
<212>  PRT
<213>  Homo sapiens

<400>  198

Lys Glu Lys Trp Asp Ser Ser Arg Cys
1               5


<210>  199
<211>  9
<212>  PRT
<213>  Homo sapiens

<400>  199

Gly Ser Ala Ala Ala Gly Thr Gln Gly
1               5

<210>  200
<211>  9
<212>  PRT
<213>  Homo sapiens

<400>  200

Asp Phe Ser Trp Ala Gly Pro His Phe
1               5

<210>  201
<211>  9
<212>  PRT
<213>  Homo sapiens

<400>  201

Gly Thr His Tyr Tyr Asp Ile Arg Val
1               5

<210>  202
<211>  9
<212>  PRT
<213>  Homo sapiens

<400>  202

Asp Gly Ser Gly Ser Tyr Glu Gly Asn
1               5

<210>  203
<211>  9
<212>  PRT
<213>  Homo sapiens

<400>  203

Gly Gly Ala Val Ala Ala Phe Asp Tyr
1               5

<210>  204
<211>  9
<212>  PRT
<213>  Homo sapiens

<400>  204

Lys Pro Val Thr Gly Gly Glu Asp Tyr
1               5

<210>  205
<211>  9
<212>  PRT
<213>  Homo sapiens

<400>  205

Asp Tyr Asp Gly Ala Trp Phe Ala Tyr
1               5

<210>  206
<211>  9
<212>  PRT
<213>  Homo sapiens

<400>  206

Trp Asp Gly Arg Leu Leu Val Asp Tyr
1               5

<210>  207
<211>  9
<212>  PRT
<213>  Homo sapiens

<400>  207

His Lys Gly Leu Arg Tyr Phe Asp Tyr
1               5

<210>  208
<211>  9
<212>  PRT
<213>  Homo sapiens

<400>  208

His Lys Gly Leu Arg Tyr Phe Asp Tyr
1               5

<210>  209
<211>  9
<212>  PRT
<213>  Homo sapiens

<400>  209

His Lys Gly Leu Arg Tyr Phe Asp Tyr
1               5

<210>  210
<211>  9
<212>  PRT
<213>  Homo sapiens

<400>  210

His Lys Gly Leu Arg Tyr Phe Asp Tyr
1               5

<210>  211
<211>  9
<212>  PRT
<213>  Homo sapiens

<400>  211

His Lys Gly Leu Arg Tyr Phe Asp Tyr

1               5


<210>  212
<211>  9
<212>  PRT
<213>  Homo sapiens

<400>  212

His Lys Gly Leu Arg Tyr Phe Asp Tyr
1               5


<210>  213
<211>  9
<212>  PRT
<213>  Homo sapiens

<400>  213

Tyr Arg Gly Asp Thr Tyr Asp Tyr Ser
1               5


<210>  214
<211>  9
<212>  PRT
<213>  Homo sapiens

<400>  214

Trp Val Gly Ala Thr Thr Ser Asp Tyr
1               5


<210>  215
<211>  9
<212>  PRT
<213>  Homo sapiens

<400>  215

Glu Asp Met Asp Tyr Gly Met Asp Val
1               5


<210>  216
<211>  9
<212>  PRT
<213>  Homo sapiens

<400>  216

Gly Gly Arg Asp Arg Tyr Leu Val Tyr
1               5


<210>  217
<211>  9
<212>  PRT
<213>  Homo sapiens

<400>  217

Val Arg Val Ser Tyr Gly Met Asp Val
1               5

<210>  218
<211>  9
<212>  PRT
<213>  Homo sapiens

<400>  218

Met Arg Lys Gly Tyr Ala Met Asp Tyr
1               5


<210>  219
<211>  9
<212>  PRT
<213>  Homo sapiens

<400>  219

Met Arg Lys Gly Tyr Ala Met Asp Tyr
1               5


<210>  220
<211>  9
<212>  PRT
<213>  Homo sapiens

<400>  220

Met Arg Lys Gly Tyr Ala Met Asp Tyr
1               5


<210>  221
<211>  9
<212>  PRT
<213>  Homo sapiens

<400>  221

Met Arg Lys Gly Tyr Ala Met Asp Tyr
1               5


<210>  222
<211>  9
<212>  PRT
<213>  Homo sapiens

<400>  222

Met Arg Lys Gly Tyr Ala Met Asp Tyr
1               5


<210>  223
<211>  9
<212>  PRT
<213>  Homo sapiens

<400>  223

Arg Met Pro Ala Val Ala Phe Asp Tyr
1               5


<210>  224

<211>  9
<212>  PRT
<213>  Homo sapiens

<400>  224

Arg Met Pro Ala Val Ala Phe Asp Tyr
1               5


<210>  225
<211>  9
<212>  PRT
<213>  Homo sapiens

<400>  225

Arg Met Arg Ala Val Ala Phe Asp Tyr
1               5


<210>  226
<211>  9
<212>  PRT
<213>  Homo sapiens

<400>  226

Arg Met Pro Ala Val Ala Phe Asp Tyr
1               5


<210>  227
<211>  9
<212>  PRT
<213>  Homo sapiens

<400>  227

Thr Ser Ile Val Arg Gly Phe Gly Pro
1               5


<210>  228
<211>  9
<212>  PRT
<213>  Homo sapiens

<400>  228

Asp Phe Phe Arg Asp Tyr Phe Asp Tyr
1               5


<210>  229
<211>  9
<212>  PRT
<213>  Homo sapiens

<400>  229

Asp Phe Phe Arg Asp Tyr Phe Asp Tyr
1               5


<210>  230
<211>  9
<212>  PRT

<213>  Homo sapiens

<400>  230

Gly Gly Thr Gln Pro Phe Asp Ile Arg
1               5


<210>  231
<211>  9
<212>  PRT
<213>  Homo sapiens

<400>  231

Ser Gln Ala Ser Gly Pro Phe Asp Tyr
1               5


<210>  232
<211>  9
<212>  PRT
<213>  Homo sapiens

<400>  232

Gly Leu Tyr Gln Leu Leu Phe Asp Tyr
1               5


<210>  233
<211>  9
<212>  PRT
<213>  Homo sapiens

<400>  233

Ala Gly Gly Arg Thr Ser Phe Asp Pro
1               5


<210>  234
<211>  9
<212>  PRT
<213>  Homo sapiens

<400>  234

Glu Gly Asn Thr Lys Ala Pro Asp Tyr
1               5


<210>  235
<211>  9
<212>  PRT
<213>  Homo sapiens

<400>  235

Asn Gly Thr Ser Gly Asp Phe Asp Tyr
1               5


<210>  236
<211>  9
<212>  PRT
<213>  Homo sapiens

<400>  236

Tyr Gly Thr Ser Tyr Trp Phe Pro Tyr
1               5


<210>  237
<211>  9
<212>  PRT
<213>  Homo sapiens

<400>  237

Tyr Gly Thr Ser Tyr Trp Phe Pro Tyr
1               5


<210>  238
<211>  9
<212>  PRT
<213>  Homo sapiens

<400>  238

Gly Gly Arg Asp Arg Tyr Leu Val Tyr
1               5


<210>  239
<211>  9
<212>  PRT
<213>  Homo sapiens

<400>  239

Leu Arg Tyr Gln Leu Leu Tyr Asn Tyr
1               5


<210>  240
<211>  9
<212>  PRT
<213>  Homo sapiens

<400>  240

Tyr Ile Ala Tyr Asp Ala Phe Asp Ile
1               5


<210>  241
<211>  9
<212>  PRT
<213>  Homo sapiens

<400>  241

Ile Thr Pro Arg Asn Ala Val Asp Ile
1               5


<210>  242
<211>  9
<212>  PRT
<213>  Homo sapiens

<400>  242

Asp Gly Leu Leu Ala Ala Thr Asp Tyr
1               5

<210>  243
<211>  9
<212>  PRT
<213>  Homo sapiens

<400>  243

Asp Arg Ala Tyr Leu Asp Phe Trp Gly
1               5

<210>  244
<211>  9
<212>  PRT
<213>  Homo sapiens

<400>  244

Asp Lys Glu Pro Ala Tyr Phe Asp Tyr
1               5

<210>  245
<211>  9
<212>  PRT
<213>  Homo sapiens

<400>  245

Arg Gly Phe Asn Gly Gln Leu Ile Phe
1               5

<210>  246
<211>  9
<212>  PRT
<213>  Homo sapiens

<400>  246

Leu Ser Val Val Val Pro Ala Ala Leu
1               5

<210>  247
<211>  9
<212>  PRT
<213>  Homo sapiens

<400>  247

Leu Ala Asp Asp Asp Pro Glu Asp Phe
1               5

<210>  248
<211>  9
<212>  PRT
<213>  Homo sapiens

<400>  248

Glu Asp Met Asp Tyr Gly Met Asp Val
1               5

<210>  249
<211>  9
<212>  PRT
<213>  Homo sapiens

<400>  249

Ser Ala Gly Gly Ser Ala Trp Ser Thr
1               5


<210>  250
<211>  9
<212>  PRT
<213>  Homo sapiens

<400>  250

Asp Arg Ser Tyr Tyr Gly Met Asp Val
1               5


<210>  251
<211>  9
<212>  PRT
<213>  Homo sapiens

<400>  251

Asp Lys Gly Thr Arg Tyr Ser Asp Gln
1               5


<210>  252
<211>  9
<212>  PRT
<213>  Homo sapiens

<400>  252

Trp Leu Val Glu Gly Ser Phe Asp Tyr
1               5


<210>  253
<211>  9
<212>  PRT
<213>  Homo sapiens

<400>  253

Gly Tyr Val Gly Ser Ser Leu Asp Tyr
1               5


<210>  254
<211>  9
<212>  PRT
<213>  Homo sapiens

<400>  254

Trp His Gln Leu Arg Gly Pro Asp Tyr
1               5

<210> 255
<211> 9
<212> PRT
<213> Homo sapiens

<400> 255

Glu Asn Ser Asp Tyr Tyr Phe Asp Tyr
1               5


<210> 256
<211> 9
<212> PRT
<213> Homo sapiens

<400> 256

Asp Gly Thr Tyr Gly Ser Gly Val Arg
1               5


<210> 257
<211> 9
<212> PRT
<213> Homo sapiens

<400> 257

Gly Gly Ser Met Val Pro Phe Asp Tyr
1               5


<210> 258
<211> 9
<212> PRT
<213> Homo sapiens

<400> 258

Arg Gly Trp Asn Tyr Tyr Phe Asp Ser
1               5


<210> 259
<211> 9
<212> PRT
<213> Homo sapiens

<400> 259

Asp Ala Tyr Tyr Tyr Gly Leu Asp Val
1               5


<210> 260
<211> 9
<212> PRT
<213> Homo sapiens

<400> 260

Asp Gly Arg Tyr Asp Pro Ile Asp Tyr
1               5


<210> 261
<211> 9

<212>  PRT
<213>  Homo sapiens

<400>  261

Val Gly Ser Ser Gly Trp Tyr Asp Tyr
1               5


<210>  262
<211>  9
<212>  PRT
<213>  Homo sapiens

<400>  262

Asp Leu Tyr Asp Tyr Tyr Asp Glu Pro
1               5


<210>  263
<211>  9
<212>  PRT
<213>  Homo sapiens

<400>  263

Asp Gly Ala Ala Ala Ser Phe Asp Tyr
1               5


<210>  264
<211>  9
<212>  PRT
<213>  Homo sapiens

<400>  264

Val Val Gly Ala Asp Tyr Phe Asp Tyr
1               5


<210>  265
<211>  9
<212>  PRT
<213>  Homo sapiens

<400>  265

Asp Gln Asn Trp Gly Tyr Phe Asp Tyr
1               5


<210>  266
<211>  9
<212>  PRT
<213>  Homo sapiens

<400>  266

Gly Val Leu Arg Asp Ala Phe Asp Ile
1               5


<210>  267
<211>  9
<212>  PRT
<213>  Homo sapiens

<400>  267

Ala Ser Asp Gly Tyr Gly Met Asp Val
1               5


<210>  268
<211>  9
<212>  PRT
<213>  Homo sapiens

<400>  268

Gly Val Leu Arg His Ala Leu Asp Ile
1               5


<210>  269
<211>  9
<212>  PRT
<213>  Homo sapiens

<400>  269

Gly Gly Cys Gly Trp Tyr Lys Asn Tyr
1               5


<210>  270
<211>  9
<212>  PRT
<213>  Homo sapiens

<400>  270

Gly Ser Asn Tyr Ala Lys Thr Gly Tyr
1               5


<210>  271
<211>  9
<212>  PRT
<213>  Homo sapiens

<400>  271

Gly Lys Phe Gln Leu Leu Phe Asp Tyr
1               5


<210>  272
<211>  9
<212>  PRT
<213>  Homo sapiens

<400>  272

Ala Leu His Gly Gly Gly Met Asp Val
1               5


<210>  273
<211>  9
<212>  PRT
<213>  Homo sapiens

<400>  273

Ala Leu His Gly Gly Gly Met Asp Val
1               5

<210>  274
<211>  9
<212>  PRT
<213>  Homo sapiens

<400>  274

Val Tyr Pro Pro Asp Ala Phe Asp Leu
1               5

<210>  275
<211>  9
<212>  PRT
<213>  Homo sapiens

<400>  275

Pro Trp Asp Tyr Trp Phe Phe Asp Leu
1               5

<210>  276
<211>  9
<212>  PRT
<213>  Homo sapiens

<400>  276

Asp Arg Val Ala Ala Ala Gly Asp Tyr
1               5

<210>  277
<211>  9
<212>  PRT
<213>  Homo sapiens

<400>  277

Asp Lys Gly Thr Arg Tyr Ser Asp Gln
1               5

<210>  278
<211>  9
<212>  PRT
<213>  Homo sapiens

<400>  278

Asp Arg Val Ala Thr Ile Pro Asp Tyr
1               5

<210>  279
<211>  9
<212>  PRT
<213>  Homo sapiens

<400>  279

Glu Arg Gly Ile Thr Leu Met Asp Val

1               5


<210>  280
<211>  9
<212>  PRT
<213>  Homo sapiens

<400>  280

Glu Arg Gly Ile Thr Leu Met Asp Val
1               5


<210>  281
<211>  9
<212>  PRT
<213>  Homo sapiens

<400>  281

Leu Asp Trp Leu Leu Pro Ile Asp Tyr
1               5


<210>  282
<211>  9
<212>  PRT
<213>  Homo sapiens

<400>  282

Leu Asp Trp Leu Leu Pro Ile Asp Tyr
1               5


<210>  283
<211>  9
<212>  PRT
<213>  Homo sapiens

<400>  283

Asp Asp Gly Asp Arg Ala Phe Gly Tyr
1               5


<210>  284
<211>  9
<212>  PRT
<213>  Homo sapiens

<400>  284

Asp Pro Trp Pro Ala Ala Phe Asp Ile
1               5


<210>  285
<211>  9
<212>  PRT
<213>  Homo sapiens

<400>  285

Val Arg Gly Ser Trp Ser Gly Asp Ser
1               5

<210>  286
<211>  9
<212>  PRT
<213>  Homo sapiens

<400>  286

Arg His Ser Ser Asp Trp Tyr Pro Tyr
1               5


<210>  287
<211>  9
<212>  PRT
<213>  Homo sapiens

<400>  287

Ser Ser Pro Tyr Gly Ala Leu Asp Tyr
1               5


<210>  288
<211>  9
<212>  PRT
<213>  Homo sapiens

<400>  288

Gly Leu Asp Gln Tyr Lys Thr Gly His
1               5


<210>  289
<211>  9
<212>  PRT
<213>  Homo sapiens

<400>  289

Gly Ala Gly Ala Ala Pro His Asp Tyr
1               5


<210>  290
<211>  9
<212>  PRT
<213>  Homo sapiens

<400>  290

Gly Ala Gly Ala Ala Pro His Asp Tyr
1               5


<210>  291
<211>  9
<212>  PRT
<213>  Homo sapiens

<400>  291

Asn Gly Thr Ser Gly Asp Phe Asp Tyr
1               5


<210>  292

<211>  9
<212>  PRT
<213>  Homo sapiens

<400>  292

Ala Leu Arg Pro Ala Thr Phe Asp Phe
1               5


<210>  293
<211>  8
<212>  PRT
<213>  Homo sapiens

<400>  293

Gln Gln Tyr Ala Asp Leu Ile Thr
1               5


<210>  294
<211>  8
<212>  PRT
<213>  Homo sapiens

<400>  294

Gln Gln Tyr Tyr Ser Thr Pro Thr
1               5


<210>  295
<211>  8
<212>  PRT
<213>  Homo sapiens

<400>  295

Gln Gln Tyr Asn Thr Tyr Pro Thr
1               5


<210>  296
<211>  8
<212>  PRT
<213>  Homo sapiens

<400>  296

Gln Gln Gly Asn Ser Phe Pro Lys
1               5


<210>  297
<211>  8
<212>  PRT
<213>  Homo sapiens

<400>  297

Gln Gln Tyr Gly Tyr Ser Leu Thr
1               5


<210>  298
<211>  8
<212>  PRT

<213>  Homo sapiens

<400>  298

Gln Gln Phe Gly Gly Ser Phe Thr
1               5


<210>  299
<211>  8
<212>  PRT
<213>  Homo sapiens

<400>  299

Gln Gln Ser Ser Asn Thr Val Thr
1               5


<210>  300
<211>  8
<212>  PRT
<213>  Homo sapiens

<400>  300

Gln Gln Tyr Asn Ser Leu Ile Thr
1               5


<210>  301
<211>  8
<212>  PRT
<213>  Homo sapiens

<400>  301

Gln Gln Tyr Asn Asn Trp Pro Thr
1               5


<210>  302
<211>  8
<212>  PRT
<213>  Homo sapiens

<400>  302

Leu Gln His Asn Ser Tyr Pro Phe
1               5


<210>  303
<211>  8
<212>  PRT
<213>  Homo sapiens

<400>  303

Gln Gln Tyr Asn Ser Gln Tyr Thr
1               5


<210>  304
<211>  8
<212>  PRT
<213>  Homo sapiens

<400>  304

Gln Gln Tyr Gly Ser Leu Trp Thr
1               5


<210>  305
<211>  8
<212>  PRT
<213>  Homo sapiens

<400>  305

Gln His Tyr Asn Arg Pro Trp Thr
1               5


<210>  306
<211>  8
<212>  PRT
<213>  Homo sapiens

<400>  306

Gln Gln Tyr Gly Ser Arg Leu Thr
1               5


<210>  307
<211>  8
<212>  PRT
<213>  Homo sapiens

<400>  307

Gln His Tyr Gly Thr Pro Arg Thr
1               5


<210>  308
<211>  8
<212>  PRT
<213>  Homo sapiens

<400>  308

Gln Gln Tyr Asn Asn Trp Pro Thr
1               5


<210>  309
<211>  8
<212>  PRT
<213>  Homo sapiens

<400>  309

Met Gln Ala Thr Gln Phe Pro Thr
1               5


<210>  310
<211>  8
<212>  PRT
<213>  Homo sapiens

<400>  310

His Gln Ala Ser Thr Tyr Pro Leu
1               5

<210>  311
<211>  8
<212>  PRT
<213>  Homo sapiens

<400>  311

Gln Gln Tyr Gly Arg Ser Pro Arg
1               5

<210>  312
<211>  8
<212>  PRT
<213>  Homo sapiens

<400>  312

Gln Gln Asp Asp Leu Pro Tyr Thr
1               5

<210>  313
<211>  8
<212>  PRT
<213>  Homo sapiens

<400>  313

Gln Asn Asp Asn Leu Pro Leu Thr
1               5

<210>  314
<211>  8
<212>  PRT
<213>  Homo sapiens

<400>  314

Gln Gln Glu Ser Leu Pro Leu Thr
1               5

<210>  315
<211>  8
<212>  PRT
<213>  Homo sapiens

<400>  315

Gln Gln Asp Asn Leu Pro Leu Thr
1               5

<210>  316
<211>  8
<212>  PRT
<213>  Homo sapiens

<400>  316

Gln Gln Glu Ser Leu Pro Cys Gly
1               5

<210>  317
<211>  8
<212>  PRT
<213>  Homo sapiens

<400>  317

Gln Gln Asp Ser Leu Pro Leu Thr
1               5


<210>  318
<211>  8
<212>  PRT
<213>  Homo sapiens

<400>  318

Gln Gln Tyr Gly Ser Ser Arg Ser
1               5


<210>  319
<211>  8
<212>  PRT
<213>  Homo sapiens

<400>  319

Gln Gln Tyr Gly Ser Ser Arg Thr
1               5


<210>  320
<211>  8
<212>  PRT
<213>  Homo sapiens

<400>  320

Gln Gln Tyr Cys Gly Ser Leu Ser
1               5


<210>  321
<211>  8
<212>  PRT
<213>  Homo sapiens

<400>  321

Gln Gln Ser Tyr Ser Thr Leu Thr
1               5


<210>  322
<211>  8
<212>  PRT
<213>  Homo sapiens

<400>  322

Gln Leu Tyr Gly Ser Ser Leu Thr
1               5

<210>  323
<211>  8
<212>  PRT
<213>  Homo sapiens

<400>  323

Gln Gln Tyr Asn Asn Leu Trp Thr
1               5


<210>  324
<211>  8
<212>  PRT
<213>  Homo sapiens

<400>  324

Gln Gln Tyr Asn Thr Phe Phe Thr
1               5


<210>  325
<211>  8
<212>  PRT
<213>  Homo sapiens

<400>  325

Gln Gln Tyr Gly Ser Ser Pro Thr
1               5


<210>  326
<211>  8
<212>  PRT
<213>  Homo sapiens

<400>  326

Gln Gln Tyr Gly Ser Ser Leu Thr
1               5


<210>  327
<211>  8
<212>  PRT
<213>  Homo sapiens

<400>  327

Gln Gln Tyr Gly Ser Ser Leu Thr
1               5


<210>  328
<211>  8
<212>  PRT
<213>  Homo sapiens

<400>  328

Gln Gln Tyr Gly Ser Ser Lys Thr
1               5


<210>  329
<211>  8

<212>  PRT
<213>  Homo sapiens

<400>  329

Gln Gln Tyr Asn Asn Trp Pro Pro
1               5


<210>  330
<211>  8
<212>  PRT
<213>  Homo sapiens

<400>  330

Gln His Arg Asn Asn Trp Pro Pro
1               5


<210>  331
<211>  8
<212>  PRT
<213>  Homo sapiens

<400>  331

Gln Gln Arg Ser Asn Trp Pro Ser
1               5


<210>  332
<211>  8
<212>  PRT
<213>  Homo sapiens

<400>  332

Gln Gln Tyr Gly Ser Ser Pro Thr
1               5


<210>  333
<211>  8
<212>  PRT
<213>  Homo sapiens

<400>  333

Gln Gln Tyr Asp Thr Ile Pro Thr
1               5


<210>  334
<211>  8
<212>  PRT
<213>  Homo sapiens

<400>  334

Gln Ala Ser Ile Asn Thr Pro Leu
1               5


<210>  335
<211>  8
<212>  PRT
<213>  Homo sapiens

<400>  335

Met Gln Ala Leu Gln Pro Trp Thr
1               5


<210>  336
<211>  8
<212>  PRT
<213>  Homo sapiens

<400>  336

Gln Gln Gly Phe Ser Asp Arg Ser
1               5


<210>  337
<211>  8
<212>  PRT
<213>  Homo sapiens

<400>  337

Met Gln Ala Thr Gln Phe Val Thr
1               5


<210>  338
<211>  8
<212>  PRT
<213>  Homo sapiens

<400>  338

Gln Arg Cys Lys Gly Met Phe Ser
1               5


<210>  339
<211>  8
<212>  PRT
<213>  Homo sapiens

<400>  339

Gln Gln Tyr Gly Gly Ser Pro Trp
1               5


<210>  340
<211>  8
<212>  PRT
<213>  Homo sapiens

<400>  340

Cys Arg Ser His Trp Pro Tyr Thr
1               5


<210>  341
<211>  8
<212>  PRT
<213>  Homo sapiens

<400>  341

Gln Gln Tyr Tyr Ser Thr Pro Pro
1               5


<210>  342
<211>  8
<212>  PRT
<213>  Homo sapiens

<400>  342

Gln Gln Cys Asn Thr Asn Pro Pro
1               5


<210>  343
<211>  8
<212>  PRT
<213>  Homo sapiens

<400>  343

Gln Gln Tyr Tyr Ser Thr Pro Pro
1               5


<210>  344
<211>  8
<212>  PRT
<213>  Homo sapiens

<400>  344

Gln Gln Tyr Tyr Ser Val Pro Pro
1               5


<210>  345
<211>  8
<212>  PRT
<213>  Homo sapiens

<400>  345

Gln Gln Tyr Asp Ser Leu Val Thr
1               5


<210>  346
<211>  8
<212>  PRT
<213>  Homo sapiens

<400>  346

His Gln Tyr Leu Ser Ser Trp Thr
1               5


<210>  347
<211>  8
<212>  PRT
<213>  Homo sapiens

<400>  347

Met Gln Gly Ile His Leu Leu Thr

1               5


<210>  348
<211>  8
<212>  PRT
<213>  Homo sapiens

<400>  348

Gln His Tyr Tyr Gly Thr Pro His
1               5


<210>  349
<211>  8
<212>  PRT
<213>  Homo sapiens

<400>  349

Gln Gln Tyr Asn Thr Tyr Pro Thr
1               5


<210>  350
<211>  8
<212>  PRT
<213>  Homo sapiens

<400>  350

Gln Glu Phe Gly Asp Ser Gly Thr
1               5


<210>  351
<211>  8
<212>  PRT
<213>  Homo sapiens

<400>  351

Gln Gln Tyr Gly Gly Ser Pro Trp
1               5


<210>  352
<211>  8
<212>  PRT
<213>  Homo sapiens

<400>  352

Gln Gln Tyr Gly Ser Ser Arg Thr
1               5


<210>  353
<211>  8
<212>  PRT
<213>  Homo sapiens

<400>  353

Gln Gln Tyr Asp Ser Leu Pro Thr
1               5

<210>  354
<211>  8
<212>  PRT
<213>  Homo sapiens

<400>  354

Gln Gln Tyr Gly Ser Val Phe Thr
1               5


<210>  355
<211>  8
<212>  PRT
<213>  Homo sapiens

<400>  355

Gln Gln Tyr Asn Ser Tyr Cys Ser
1               5


<210>  356
<211>  8
<212>  PRT
<213>  Homo sapiens

<400>  356

Gln Gln Tyr Tyr Ser Thr Pro Leu
1               5


<210>  357
<211>  8
<212>  PRT
<213>  Homo sapiens

<400>  357

Gln Gln Tyr Asn Asp Trp Pro Thr
1               5


<210>  358
<211>  8
<212>  PRT
<213>  Homo sapiens

<400>  358

Met Gln Asn Ile Gln Phe Pro Thr
1               5


<210>  359
<211>  8
<212>  PRT
<213>  Homo sapiens

<400>  359

Gln Gln Tyr Asp Asn Leu Pro Pro
1               5


<210>  360

<211>  8
<212>  PRT
<213>  Homo sapiens

<220>
<221>  misc_feature
<222>  (5)..(5)
<223>  Xaa can be any naturally occurring amino acid

<400>  360

Gln Leu Leu Arg Xaa Leu Arg Thr
1               5

<210>  361
<211>  8
<212>  PRT
<213>  Homo sapiens

<400>  361

Tyr Gln Tyr Asn Asn Gly Tyr Thr
1               5

<210>  362
<211>  8
<212>  PRT
<213>  Homo sapiens

<400>  362

Gln Gln Arg Ser Asn Trp Pro Thr
1               5

<210>  363
<211>  8
<212>  PRT
<213>  Homo sapiens

<400>  363

Gln Gln Tyr Gly Thr Ser His Thr
1               5

<210>  364
<211>  8
<212>  PRT
<213>  Homo sapiens

<400>  364

Gln Gln Tyr Asn His Trp Pro Ser
1               5

<210>  365
<211>  8
<212>  PRT
<213>  Homo sapiens

<400>  365

Gln Gln Tyr Gly Ser Leu Tyr Thr
1               5


<210>  366
<211>  8
<212>  PRT
<213>  Homo sapiens

<400>  366

Gln Gln Asn Lys Asp Trp Pro Leu
1               5


<210>  367
<211>  8
<212>  PRT
<213>  Homo sapiens

<400>  367

Gln Gln Phe Gly Thr Ser Leu Thr
1               5


<210>  368
<211>  8
<212>  PRT
<213>  Homo sapiens

<400>  368

Gln Gln Arg Ser Asn Trp Trp Thr
1               5


<210>  369
<211>  8
<212>  PRT
<213>  Homo sapiens

<400>  369

Gln Gln Cys Ser Asn Trp Pro Thr
1               5


<210>  370
<211>  8
<212>  PRT
<213>  Homo sapiens

<400>  370

Gln Gln Tyr Gly Ser Ser Pro Thr
1               5


<210>  371
<211>  10
<212>  PRT
<213>  Homo sapiens

<400>  371

Gln Gln Tyr Gly Ser Ser Pro Ser Ile Thr
1               5                   10

<210>  372
<211>  10
<212>  PRT
<213>  Homo sapiens

<400>  372

Gln Lys Tyr Asn Ser Ala Pro Pro Ser Thr
1               5                   10


<210>  373
<211>  10
<212>  PRT
<213>  Homo sapiens

<400>  373

Gln Glu Tyr Asn Asn Trp Pro Leu Trp Thr
1               5                   10


<210>  374
<211>  10
<212>  PRT
<213>  Homo sapiens

<400>  374

Gln Gln Tyr Gly Gly Ser Pro Pro Trp Thr
1               5                   10


<210>  375
<211>  10
<212>  PRT
<213>  Homo sapiens

<400>  375

His Glu Tyr Asn Gly Trp Pro Pro Trp Thr
1               5                   10


<210>  376
<211>  10
<212>  PRT
<213>  Homo sapiens

<400>  376

Gln Gln Tyr Asn Ser Tyr Ser Pro Leu Thr
1               5                   10


<210>  377
<211>  10
<212>  PRT
<213>  Homo sapiens

<400>  377

Met Gln His Thr His Trp Ser Pro Ile Thr
1               5                   10

<210>  378
<211>  10
<212>  PRT
<213>  Homo sapiens

<400>  378

Gln His Tyr Asn Asn Trp Pro Pro Trp Thr
1               5                   10


<210>  379
<211>  10
<212>  PRT
<213>  Homo sapiens

<400>  379

Gln Gln Ser Tyr Asn Thr Pro Pro Trp Thr
1               5                   10


<210>  380
<211>  10
<212>  PRT
<213>  Homo sapiens

<400>  380

Gln Gln Ser Tyr Asn Thr Pro Pro Trp Thr
1               5                   10


<210>  381
<211>  10
<212>  PRT
<213>  Homo sapiens

<400>  381

Gln His Tyr Gly Ser Ser Pro Pro Trp Thr
1               5                   10


<210>  382
<211>  10
<212>  PRT
<213>  Homo sapiens

<400>  382

Gln Gln His Asn Asn Trp Pro Pro Leu Thr
1               5                   10


<210>  383
<211>  10
<212>  PRT
<213>  Homo sapiens

<400>  383

Gln Val Tyr Gly Gln Ser Pro Val Phe Thr
1               5                   10


<210>  384
<211>  10

<212>  PRT
<213>  Homo sapiens

<400>  384

Gln Gln Tyr Gly Ser Ser Pro Met Tyr Thr
1               5                   10


<210>  385
<211>  10
<212>  PRT
<213>  Homo sapiens

<400>  385

Gln Gln Tyr Gly Ser Ser Pro Met Tyr Thr
1               5                   10


<210>  386
<211>  10
<212>  PRT
<213>  Homo sapiens

<400>  386

Gln Arg Phe Gly Thr Ser Pro Leu Tyr Thr
1               5                   10


<210>  387
<211>  10
<212>  PRT
<213>  Homo sapiens

<400>  387

Gln Gln Tyr Gly Asp Ser Pro Leu Tyr Ser
1               5                   10


<210>  388
<211>  10
<212>  PRT
<213>  Homo sapiens

<400>  388

Gln Gln Tyr Asp Asp Trp Pro Pro Ile Thr
1               5                   10


<210>  389
<211>  10
<212>  PRT
<213>  Homo sapiens

<400>  389

Gln Gln Leu Asn Ser Tyr Pro Pro Tyr Thr
1               5                   10


<210>  390
<211>  10
<212>  PRT
<213>  Homo sapiens

<400>  390

Gln Gln Ser Tyr Ser Thr Pro Pro Asp Thr
1               5                   10


<210>  391
<211>  10
<212>  PRT
<213>  Homo sapiens

<400>  391

Gln His Tyr Asn Asn Trp Pro Pro Ser Ser
1               5                   10


<210>  392
<211>  10
<212>  PRT
<213>  Homo sapiens

<400>  392

Gln His Tyr Asn Arg Leu Pro Pro Trp Thr
1               5                   10


<210>  393
<211>  10
<212>  PRT
<213>  Homo sapiens

<400>  393

Gln Gln Tyr Asp Arg Ser Val Pro Leu Thr
1               5                   10


<210>  394
<211>  10
<212>  PRT
<213>  Homo sapiens

<400>  394

Gln Gln Tyr Tyr Thr Thr Pro Thr Tyr Thr
1               5                   10


<210>  395
<211>  10
<212>  PRT
<213>  Homo sapiens

<400>  395

Gln Gln Tyr Tyr Thr Thr Pro Pro Leu Thr
1               5                   10


<210>  396
<211>  10
<212>  PRT
<213>  Homo sapiens

<400>  396

Gln Gln Leu Tyr Ser Tyr Pro His Leu Thr
1               5                   10

<210>  397
<211>  10
<212>  PRT
<213>  Homo sapiens

<400>  397

Cys Gln Gln Tyr Gly Ser Ser Arg Trp Thr
1               5                   10

<210>  398
<211>  10
<212>  PRT
<213>  Homo sapiens

<400>  398

Met Gln Ala Leu Gln Thr Pro Met Ser Thr
1               5                   10

<210>  399
<211>  10
<212>  PRT
<213>  Homo sapiens

<400>  399

Gln Gln Arg Ser Glu Trp Pro Pro Leu Thr
1               5                   10

<210>  400
<211>  10
<212>  PRT
<213>  Homo sapiens

<400>  400

Gln Gln Tyr Asp Thr Ser Pro Ala Trp Thr
1               5                   10

<210>  401
<211>  10
<212>  PRT
<213>  Homo sapiens

<400>  401

Gln Gln Tyr Gly Ser Ser Gln Gly Phe Thr
1               5                   10

<210>  402
<211>  10
<212>  PRT
<213>  Homo sapiens

<400>  402

Met Gln Ser Ile Gln Leu Pro Arg Trp Thr

1               5                   10


<210>  403
<211>  10
<212>  PRT
<213>  Homo sapiens

<400>  403

Gln His Tyr Gly Leu Ser Pro Pro Ile Thr
1               5                   10


<210>  404
<211>  10
<212>  PRT
<213>  Homo sapiens

<400>  404

Gln Glu Tyr Gly Ser Ser Pro Pro Arg Thr
1               5                   10


<210>  405
<211>  10
<212>  PRT
<213>  Homo sapiens

<400>  405

Ser Ser Tyr Arg Ser Ser Ser Thr Arg Val
1               5                   10


<210>  406
<211>  10
<212>  PRT
<213>  Homo sapiens

<400>  406

Gln His Tyr Gly Leu Ser Pro Pro Ile Thr
1               5                   10


<210>  407
<211>  10
<212>  PRT
<213>  Homo sapiens

<400>  407

Gln Glu Tyr Gly Ser Ser Pro Pro Arg Thr
1               5                   10


<210>  408
<211>  10
<212>  PRT
<213>  Homo sapiens

<400>  408

Gln Gln Tyr Tyr Thr Thr Leu Pro Leu Thr
1               5                   10

<210>  409
<211>  10
<212>  PRT
<213>  Homo sapiens

<400>  409

Ser Ser Tyr Ser Ser Thr Thr Arg Val Val
1               5                   10


<210>  410
<211>  10
<212>  PRT
<213>  Homo sapiens

<400>  410

Gln Gln Tyr Gly Ser Ser Pro Gln Thr Phe
1               5                   10


<210>  411
<211>  10
<212>  PRT
<213>  Homo sapiens

<400>  411

Phe Cys Gln Tyr Asn Arg Tyr Pro Tyr Thr
1               5                   10


<210>  412
<211>  10
<212>  PRT
<213>  Homo sapiens

<400>  412

Leu Gln Arg Ser Asn Trp Gly Glu Val Thr
1               5                   10


<210>  413
<211>  10
<212>  PRT
<213>  Homo sapiens

<400>  413

Gln Gln Arg Ser Asn Trp Gly Glu Val Thr
1               5                   10


<210>  414
<211>  10
<212>  PRT
<213>  Homo sapiens

<400>  414

Gln Gln Arg Ser Asn Trp Gly Glu Val Thr
1               5                   10


<210>  415

<211>  10
<212>  PRT
<213>  Homo sapiens

<400>  415

Gln Gln Tyr Gly Ser Ser Pro Leu Phe Thr
1               5                   10


<210>  416
<211>  10
<212>  PRT
<213>  Homo sapiens

<400>  416

Cys Ser Tyr Thr Ser Ser Ser Thr Leu Val
1               5                   10


<210>  417
<211>  10
<212>  PRT
<213>  Homo sapiens

<400>  417

Gln Gln Arg Ser Asn Trp Pro Pro Ile Thr
1               5                   10


<210>  418
<211>  10
<212>  PRT
<213>  Homo sapiens

<400>  418

Gln Gln Ser Tyr Asn Thr Leu Ser Leu Thr
1               5                   10


<210>  419
<211>  10
<212>  PRT
<213>  Homo sapiens

<400>  419

Gln His Tyr Gly Asn Ser Pro Pro Tyr Thr
1               5                   10


<210>  420
<211>  10
<212>  PRT
<213>  Homo sapiens

<400>  420

Gln Gln Ser His Lys Thr Leu Ala Trp Thr
1               5                   10


<210>  421
<211>  10
<212>  PRT

<213>  Homo sapiens

<400>  421

Met Gln Gly Thr Tyr Trp Pro Pro Tyr Thr
1               5                   10


<210>  422
<211>  10
<212>  PRT
<213>  Homo sapiens

<400>  422

His Gln Tyr Tyr Thr Tyr Pro Leu Phe Thr
1               5                   10


<210>  423
<211>  10
<212>  PRT
<213>  Homo sapiens

<400>  423

His Gln Tyr Tyr Thr Tyr Pro Leu Phe Thr
1               5                   10


<210>  424
<211>  10
<212>  PRT
<213>  Homo sapiens

<400>  424

Gln Gln Ser Tyr Ser Thr Pro Pro Trp Thr
1               5                   10


<210>  425
<211>  10
<212>  PRT
<213>  Homo sapiens

<400>  425

Gln Gln Ser Tyr Thr Asn Pro Glu Val Thr
1               5                   10


<210>  426
<211>  10
<212>  PRT
<213>  Homo sapiens

<400>  426

Gln Gln Tyr Gly Ser Ser Pro Pro Tyr Thr
1               5                   10


<210>  427
<211>  10
<212>  PRT
<213>  Homo sapiens

<400>  427

Gln Gln Tyr Gly Ser Ser Pro Arg Tyr Thr
1               5                   10


<210>  428
<211>  10
<212>  PRT
<213>  Homo sapiens

<400>  428

Gln Gln Tyr Gly Ser Ser Pro Arg Tyr Thr
1               5                   10


<210>  429
<211>  10
<212>  PRT
<213>  Homo sapiens



<220>
<221>  misc_feature
<222>  (10)..(10)
<223>  Xaa can be any naturally occurring amino acid

<400>  429

Gln Gln Phe Gly Asn Ser Pro Pro Leu Xaa
1               5                   10


<210>  430
<211>  10
<212>  PRT
<213>  Homo sapiens

<400>  430

Gln Gln Tyr Ala Gly Ser Pro Pro Val Thr
1               5                   10


<210>  431
<211>  10
<212>  PRT
<213>  Homo sapiens

<400>  431


<210>  432
<211>  10
<212>  PRT
<213>  Homo sapiens

<400>  432

<210>  433
<211>  10
<212>  PRT
<213>  Homo sapiens

<400>  433

Gln Gln Arg Ser Asn Cys Ser Gly Leu Thr
1               5                   10


<210>  434
<211>  10
<212>  PRT
<213>  Homo sapiens

<400>  434


<210>  435
<211>  10
<212>  PRT
<213>  Homo sapiens

<400>  435


<210>  436
<211>  10
<212>  PRT
<213>  Homo sapiens

<400>  436

Gln Gln Tyr Asn Asn Trp Pro Pro Cys Thr
1               5                   10


<210>  437
<211>  10
<212>  PRT
<213>  Homo sapiens

<400>  437


<210>  438
<211>  10
<212>  PRT
<213>  Homo sapiens

<400>  438

Gln Gln Arg Ser Phe Trp Pro Pro Leu Thr
1               5                   10


<210>  439
<211>  10

<212>  PRT
<213>  Homo sapiens

<400>  439

Gln Gln Arg Ser Asn Trp Pro Ser Ile Thr
1               5                   10


<210>  440
<211>  10
<212>  PRT
<213>  Homo sapiens

<400>  440

Gln Gln Arg Ser Asn Trp Pro Pro Leu Thr
1               5                   10


<210>  441
<211>  10
<212>  PRT
<213>  Homo sapiens

<400>  441

Gln Gln Arg Thr Asn Trp Pro Ile Phe Thr
1               5                   10


<210>  442
<211>  10
<212>  PRT
<213>  Homo sapiens

<400>  442

Gln Gln Arg Ser Asn Trp Pro Pro Gly Thr
1               5                   10


<210>  443
<211>  10
<212>  PRT
<213>  Homo sapiens

<400>  443

Gln Gln Tyr Asn Asn Trp Pro Pro Leu Thr
1               5                   10


<210>  444
<211>  10
<212>  PRT
<213>  Homo sapiens

<400>  444

Gln Gln Tyr Asn Asn Trp Pro Thr Trp Thr
1               5                   10


<210>  445
<211>  10
<212>  PRT
<213>  Homo sapiens

<400>  445

Gln Gln Arg Met Arg Trp Pro Pro Leu Thr
1               5                   10


<210>  446
<211>  10
<212>  PRT
<213>  Homo sapiens

<400>  446

Gln Gln Tyr Gly Ser Ser Pro Lys Trp Thr
1               5                   10


<210>  447
<211>  10
<212>  PRT
<213>  Homo sapiens

<400>  447

Gln Gln Tyr Gly Ser Ser Pro Gln Tyr Thr
1               5                   10


<210>  448
<211>  10
<212>  PRT
<213>  Homo sapiens

<400>  448

Gln Gln Tyr Gly Ser Ser Pro Pro Tyr Thr
1               5                   10


<210>  449
<211>  10
<212>  PRT
<213>  Homo sapiens

<400>  449

Gln Gln Tyr Asp Arg Ser Leu Pro Arg Thr
1               5                   10


<210>  450
<211>  10
<212>  PRT
<213>  Homo sapiens

<400>  450

Gln Gln Tyr Gly Asn Ser Pro Leu Phe Ser
1               5                   10


<210>  451
<211>  10
<212>  PRT
<213>  Homo sapiens

<400>  451

Gln Gln Tyr Gly Gly Ser Pro Leu Phe Ser
1               5                   10

<210>  452
<211>  10
<212>  PRT
<213>  Homo sapiens

<400>  452

Gln Gln Tyr Asn Asn Trp Pro Thr Trp Thr
1               5                   10

<210>  453
<211>  10
<212>  PRT
<213>  Homo sapiens

<400>  453

Met Gln Ala Leu Gln Thr Leu Gly Leu Thr
1               5                   10

<210>  454
<211>  10
<212>  PRT
<213>  Homo sapiens

<400>  454

Met Gln Ala Leu Gln Thr Leu Gly Leu Thr
1               5                   10

<210>  455
<211>  10
<212>  PRT
<213>  Homo sapiens

<400>  455

Gln Gln Ser His Ser Ala Pro Pro Tyr Thr
1               5                   10

<210>  456
<211>  10
<212>  PRT
<213>  Homo sapiens

<400>  456

Gln Gln Tyr Gly Ser Ser Pro Leu Phe Thr
1               5                   10

<210>  457
<211>  10
<212>  PRT
<213>  Homo sapiens

<400>  457

Gln Gln Tyr Asn Asp Trp Pro Pro Trp Thr

1               5                   10


<210>  458
<211>  10
<212>  PRT
<213>  Homo sapiens

<400>  458

Gln Gln Tyr Asn Gly Asn Ser Pro Leu Thr
1               5                   10


<210>  459
<211>  10
<212>  PRT
<213>  Homo sapiens

<400>  459

Gln Gln Leu Asn Thr Tyr Pro Pro Trp Thr
1               5                   10


<210>  460
<211>  10
<212>  PRT
<213>  Homo sapiens

<400>  460

His Lys Tyr Gly Gly Ser Pro Pro Tyr Thr
1               5                   10


<210>  461
<211>  10
<212>  PRT
<213>  Homo sapiens

<400>  461

Met Gln Asp Thr His Trp Pro Pro Trp Thr
1               5                   10


<210>  462
<211>  10
<212>  PRT
<213>  Homo sapiens

<400>  462

Gln His Tyr Gly Arg Ser Pro Pro Leu Thr
1               5                   10


<210>  463
<211>  10
<212>  PRT
<213>  Homo sapiens

<400>  463

Gln Gln Tyr Gly Asn Ser Pro Pro Tyr Thr
1               5                   10

<210>  464
<211>  10
<212>  PRT
<213>  Homo sapiens

<400>  464

Gln Gln Tyr Gly Ser Ser Pro Pro Tyr Thr
1               5                   10


<210>  465
<211>  10
<212>  PRT
<213>  Homo sapiens

<400>  465

Gln Gln Tyr Phe Asn Val Pro Pro Val Thr
1               5                   10


<210>  466
<211>  10
<212>  PRT
<213>  Homo sapiens

<400>  466

Gln His Tyr His Asn Leu Pro Pro Thr Thr
1               5                   10


<210>  467
<211>  10
<212>  PRT
<213>  Homo sapiens

<400>  467

Ile Gln Gly Thr His Trp Pro Gln Tyr Thr
1               5                   10


<210>  468
<211>  10
<212>  PRT
<213>  Homo sapiens

<400>  468

Gln Gln Tyr Gly Ser Ser Arg Ala Leu Thr
1               5                   10


<210>  469
<211>  10
<212>  PRT
<213>  Homo sapiens

<400>  469

Gln Gln Tyr Tyr Ser Thr Pro Ser Tyr Thr
1               5                   10


<210>  470

<211>  10
<212>  PRT
<213>  Homo sapiens

<400>  470

Met Gln Ala Leu Gln Thr Leu Met Cys Ser
1               5                   10


<210>  471
<211>  10
<212>  PRT
<213>  Homo sapiens

<400>  471

Gln Gln Ser Tyr Ser Thr Pro Pro Leu Thr
1               5                   10


<210>  472
<211>  10
<212>  PRT
<213>  Homo sapiens

<400>  472

Gln Gln Ser Tyr Ser Thr Pro Pro Ile Thr
1               5                   10


<210>  473
<211>  10
<212>  PRT
<213>  Homo sapiens

<400>  473

Gln Gln Tyr Gly Gly Ser Leu Pro Ile Thr
1               5                   10


<210>  474
<211>  10
<212>  PRT
<213>  Homo sapiens

<400>  474

Gln Gln Tyr Gly Ser Ser Thr Pro Leu Thr
1               5                   10


<210>  475
<211>  10
<212>  PRT
<213>  Homo sapiens

<400>  475

Gln Gln Arg Ser Ser Trp Pro Pro Leu Thr
1               5                   10


<210>  476
<211>  10
<212>  PRT

<213>  Homo sapiens

<400>  476

Gln Gln Arg Tyr Ser Trp Pro Pro Leu Thr
1               5                   10


<210>  477
<211>  10
<212>  PRT
<213>  Homo sapiens

<400>  477

Gln Gln Arg Tyr Asn Trp Pro Pro Leu Thr
1               5                   10


<210>  478
<211>  10
<212>  PRT
<213>  Homo sapiens

<400>  478

Gln Gln Arg Ser Asn Trp Pro Pro Leu Thr
1               5                   10


<210>  479
<211>  10
<212>  PRT
<213>  Homo sapiens

<400>  479

Gln Gln Arg Ser Ser Trp Pro Pro Leu Thr
1               5                   10


<210>  480
<211>  10
<212>  PRT
<213>  Homo sapiens

<400>  480


<210>  481
<211>  10
<212>  PRT
<213>  Homo sapiens

<400>  481

Gln Gln Arg Ser Asn Trp Pro Pro Tyr Thr
1               5                   10


<210>  482
<211>  10
<212>  PRT
<213>  Homo sapiens

<400>  482


<210>  483
<211>  10
<212>  PRT
<213>  Homo sapiens

<400>  483


<210>  484
<211>  10
<212>  PRT
<213>  Homo sapiens

<400>  484

Gln Gln Tyr Gly Ser Ser Pro Pro Ile Thr
1               5                   10


<210>  485
<211>  10
<212>  PRT
<213>  Homo sapiens

<400>  485

Gln Gln Tyr Asn Asn Trp Pro Pro Ile Thr
1               5                   10


<210>  486
<211>  10
<212>  PRT
<213>  Homo sapiens

<400>  486

Gln Gln Arg Ser Ser Trp Pro Pro Ile Thr
1               5                   10


<210>  487
<211>  10
<212>  PRT
<213>  Homo sapiens

<400>  487

Gln Gln Tyr Gly Ser Ser Pro Arg Val Thr
1               5                   10


<210>  488
<211>  10
<212>  PRT
<213>  Homo sapiens

<400>  488

Gln Gln Tyr Asn Thr Asn Ser Pro Ile Ser
1               5                   10


<210>  489
<211>  10
<212>  PRT
<213>  Homo sapiens

<400>  489

Gln Asn Tyr Gly Ser Ser Pro Arg Ile Thr
1               5                   10


<210>  490
<211>  10
<212>  PRT
<213>  Homo sapiens

<400>  490

Gln Gln Tyr Gly Ser Ser Pro Pro Ile Thr
1               5                   10


<210>  491
<211>  10
<212>  PRT
<213>  Homo sapiens

<400>  491

Met Gln Ser Ile Gln Leu Pro Arg Phe Thr
1               5                   10


<210>  492
<211>  10
<212>  PRT
<213>  Homo sapiens

<400>  492

Met Gln Ser Val Gln Leu Pro Arg Phe Thr
1               5                   10


<210>  493
<211>  10
<212>  PRT
<213>  Homo sapiens

<400>  493

Met Gln Ser Val Gln Leu Pro Arg Phe Thr
1               5                   10


<210>  494
<211>  10
<212>  PRT
<213>  Homo sapiens

<400>  494

Gln Gln Tyr Asp Lys Trp Pro Pro Val Thr
1               5                   10

<210>  495
<211>  10
<212>  PRT
<213>  Homo sapiens

<400>  495

Met Gln Ser Ile Gln Phe Pro Arg Trp Thr
1               5                   10


<210>  496
<211>  10
<212>  PRT
<213>  Homo sapiens

<400>  496

Met Gln Gly Ile His Leu Pro Pro Tyr Ile
1               5                   10


<210>  497
<211>  10
<212>  PRT
<213>  Homo sapiens

<400>  497

Asn Gln Gly Thr Gln Trp Leu Leu Tyr Thr
1               5                   10


<210>  498
<211>  10
<212>  PRT
<213>  Homo sapiens

<400>  498

Gln Gln Tyr Asn Ser Tyr Ala Pro Tyr Thr
1               5                   10


<210>  499
<211>  10
<212>  PRT
<213>  Homo sapiens

<400>  499

Gln His Tyr Gly Leu Ser Pro Pro Ile Thr
1               5                   10


<210>  500
<211>  10
<212>  PRT
<213>  Homo sapiens

<400>  500

Gln Glu Tyr Gly Ser Ser Pro Pro Arg Thr
1               5                   10

<210>  501
<211>  10
<212>  PRT
<213>  Homo sapiens

<400>  501

Gln Gln Tyr Phe Asn Val Pro Pro Val Thr
1               5                   10


<210>  502
<211>  10
<212>  PRT
<213>  Homo sapiens

<400>  502

Gln Gln Leu Thr Ser Tyr Pro Pro Trp Thr
1               5                   10


<210>  503
<211>  10
<212>  PRT
<213>  Homo sapiens

<400>  503

Gln Gln Val Asn Ser Tyr Pro Gly Leu Thr
1               5                   10


<210>  504
<211>  10
<212>  PRT
<213>  Homo sapiens

<400>  504

Gln Gln Val Phe Ser Tyr Pro Gly Ile Thr
1               5                   10


<210>  505
<211>  10
<212>  PRT
<213>  Homo sapiens

<400>  505

Gln Gln Tyr Thr Ser Leu Pro Gly Ile Thr
1               5                   10


<210>  506
<211>  10
<212>  PRT
<213>  Homo sapiens

<400>  506

Gln His Ser Tyr Ser Thr Leu Pro Leu Thr
1               5                   10


<210>  507
<211>  10

<212>  PRT
<213>  Homo sapiens

<400>  507

Gln Gln Tyr Tyr Asn Ile Pro Tyr Ile Thr
1               5                   10


<210>  508
<211>  10
<212>  PRT
<213>  Homo sapiens

<400>  508

Gln Leu Tyr Gly Ser Ser Pro Arg Val Thr
1               5                   10


<210>  509
<211>  10
<212>  PRT
<213>  Homo sapiens

<400>  509

Gln Gln Tyr Ala Asn Trp Pro Pro Ile Thr
1               5                   10


<210>  510
<211>  10
<212>  PRT
<213>  Homo sapiens

<400>  510

Gln Gln Tyr Asn Ile Ser Pro Arg Asp Thr
1               5                   10


<210>  511
<211>  10
<212>  PRT
<213>  Homo sapiens

<400>  511

Gln Gln Phe Gly Ser Ser Pro Leu Ile Thr
1               5                   10


<210>  512
<211>  10
<212>  PRT
<213>  Homo sapiens

<400>  512

Gln Gln Tyr Gly Asp Phe Pro Arg Val Thr
1               5                   10


<210>  513
<211>  10
<212>  PRT
<213>  Homo sapiens

<400>  513

Gln Gln Tyr Gly Asp Trp Pro Pro Tyr Thr
1               5                   10


<210>  514
<211>  10
<212>  PRT
<213>  Homo sapiens

<400>  514

Gln Gln Tyr Tyr Thr Thr Leu Ser Trp Thr
1               5                   10


<210>  515
<211>  10
<212>  PRT
<213>  Homo sapiens

<400>  515

Gln Gln Tyr Asn Lys Trp Pro Pro Leu Thr
1               5                   10


<210>  516
<211>  10
<212>  PRT
<213>  Homo sapiens

<400>  516

Met Gln Gly Thr His Trp Leu Pro Val Thr
1               5                   10


<210>  517
<211>  10
<212>  PRT
<213>  Homo sapiens

<400>  517

Gln Gln Tyr Asp Lys Trp Pro Pro Val Thr
1               5                   10


<210>  518
<211>  10
<212>  PRT
<213>  Homo sapiens

<400>  518

Gln Gln Tyr Asp Asn Leu Pro Pro Ile His
1               5                   10


<210>  519
<211>  10
<212>  PRT
<213>  Homo sapiens

<400>  519

Gln Gln Leu Asn Asn Tyr Pro Pro Phe Thr
1               5                   10


<210>  520
<211>  10
<212>  PRT
<213>  Homo sapiens

<400>  520

Gln Gln Ser Tyr Ser Thr Pro Pro Tyr Thr
1               5                   10


<210>  521
<211>  10
<212>  PRT
<213>  Homo sapiens

<400>  521

Gln Gln Ser Tyr Ser Thr Pro Pro Tyr Ser
1               5                   10


<210>  522
<211>  10
<212>  PRT
<213>  Homo sapiens

<400>  522

Gln Gln Ser Tyr Ser Thr Pro Pro Tyr Thr
1               5                   10


<210>  523
<211>  10
<212>  PRT
<213>  Homo sapiens

<400>  523

Gln Gln Tyr Asn Asn Trp Leu Pro Phe Thr
1               5                   10


<210>  524
<211>  10
<212>  PRT
<213>  Homo sapiens

<400>  524

Ala Ala Trp Asp Asp Ser Leu Thr Leu Met
1               5                   10


<210>  525
<211>  22
<212>  PRT
<213>  Homo sapiens

<400>  525

Glu Val Gln Leu Val Glu Ser Gly Gly Gly Leu Val Gln Pro Gly Gly

1               5                   10                  15

Ser Leu Lys Leu Ser Cys
            20

<210>  526
<211>  11
<212>  PRT
<213>  Homo sapiens

<400>  526

Leu Ser Cys Val Ala Ser Gly Phe Ile Phe Ser
1               5                   10

<210>  527
<211>  6
<212>  PRT
<213>  Homo sapiens

<400>  527

Phe Ser Asn His Trp Met
1               5

<210>  528
<211>  18
<212>  PRT
<213>  Homo sapiens

<400>  528

His Trp Met Asn Trp Val Arg Gln Ala Pro Gly Lys Gly Leu Glu Trp
1               5                   10                  15

Val Ala

<210>  529
<211>  5
<212>  PRT
<213>  Homo sapiens

<400>  529

Ile Arg Ser Lys Ser
1               5

<210>  530
<211>  5
<212>  PRT
<213>  Homo sapiens

<400>  530

Ala Thr His Tyr Ala
1               5

<210>  531
<211>  15

<212>  PRT
<213>  Homo sapiens

<400>  531

His Tyr Ala Glu Ser Val Lys Gly Arg Phe Thr Ile Ser Arg Asp
1               5                   10                  15


<210>  532
<211>  12
<212>  PRT
<213>  Homo sapiens

<400>  532

Arg Phe Thr Ile Ser Arg Asp Asp Ser Lys Ser Ile
1               5                   10


<210>  533
<211>  6
<212>  PRT
<213>  Homo sapiens

<400>  533

Ile Val Tyr Leu Gln Met
1               5


<210>  534
<211>  8
<212>  PRT
<213>  Homo sapiens

<400>  534

Tyr Leu Gln Met Thr Asp Leu Arg
1               5


<210>  535
<211>  11
<212>  PRT
<213>  Homo sapiens

<400>  535

Leu Arg Thr Glu Asp Thr Gly Val Tyr Tyr Cys
1               5                   10


<210>  536
<211>  8
<212>  PRT
<213>  Homo sapiens

<400>  536

Val Tyr Tyr Cys Ser Arg Asn Tyr
1               5


<210>  537
<211>  5
<212>  PRT
<213>  Homo sapiens

<400>  537

Asn Tyr Tyr Gly Ser
1               5


<210>  538
<211>  4
<212>  PRT
<213>  Homo sapiens

<400>  538

Gly Ser Thr Tyr
1


<210>  539
<211>  9
<212>  PRT
<213>  Homo sapiens

<400>  539

Thr Tyr Asp Tyr Trp Gly Gln Gly Thr
1               5


<210>  540
<211>  13
<212>  PRT
<213>  Homo sapiens

<400>  540

Asp Tyr Trp Gly Gln Gly Thr Thr Val Thr Val Ser Ser
1               5                   10


<210>  541
<211>  6
<212>  PRT
<213>  Homo sapiens

<400>  541

Asp Ile Leu Leu Thr Gln
1               5


<210>  542
<211>  20
<212>  PRT
<213>  Homo sapiens

<400>  542

Leu Thr Gln Ser Pro Ala Ile Leu Ser Leu Ser Pro Gly Glu Arg Ala
1               5                   10                  15


Thr Leu Ser Cys
            20


<210>  543
<211>  17

<212>  PRT
<213>  Homo sapiens

<400>  543

Leu Ser Leu Ser Pro Gly Glu Arg Ala Thr Leu Ser Cys Arg Ala Ser
1               5                   10                  15

Gln

<210>  544
<211>  4
<212>  PRT
<213>  Homo sapiens

<400>  544

Val Gly Ser Ser
1

<210>  545
<211>  7
<212>  PRT
<213>  Homo sapiens

<400>  545

Ile His Trp Tyr Gln Gln Lys
1               5

<210>  546
<211>  13
<212>  PRT
<213>  Homo sapiens

<400>  546

Gln Gln Lys Pro Asn Gln Ser Pro Lys Leu Leu Ile Lys
1               5                   10

<210>  547
<211>  7
<212>  PRT
<213>  Homo sapiens

<400>  547

Leu Leu Ile Lys Tyr Ala Ser
1               5

<210>  548
<211>  4
<212>  PRT
<213>  Homo sapiens

<400>  548

Tyr Ala Ser Glu
1

<210>  549
<211>  21
<212>  PRT
<213>  Homo sapiens

<400>  549

Pro Ser Arg Phe Ser Gly Ser Gly Ser Gly Thr Asp Phe Thr Leu Thr
1               5                   10                  15

Ile Asn Ser Leu Glu
            20

<210>  550
<211>  8
<212>  PRT
<213>  Homo sapiens

<400>  550

Ser Leu Glu Ser Glu Asp Ala Ala
1               5

<210>  551
<211>  7
<212>  PRT
<213>  Homo sapiens

<400>  551

Ala Asp Tyr Tyr Cys Gln Gln
1               5

<210>  552
<211>  8
<212>  PRT
<213>  Homo sapiens

<400>  552

Tyr Tyr Cys Gln Gln Ser His Ser
1               5

<210>  553
<211>  4
<212>  PRT
<213>  Homo sapiens

<400>  553

His Ser Trp Pro
1

<210>  554
<211>  9
<212>  PRT
<213>  Homo sapiens

<400>  554

Trp Pro Phe Thr Phe Gly Gln Gly Thr
1               5

<210>  555
<211>  11
<212>  PRT
<213>  Homo sapiens

<400>  555

Thr Phe Gly Gln Gly Thr Asn Leu Glu Ile Lys
1               5                   10


<210>  556
<211>  24
<212>  DNA
<213>  Artificial

<220>
<223>  Primer

<400>  556
atgtacaaca ctgtgtcatt taac                                              24


<210>  557
<211>  30
<212>  DNA
<213>  Artificial

<220>
<223>  Primer

<400>  557
ttatttggag cttttaaact taaggatacc                                        30


<210>  558
<211>  15
<212>  PRT
<213>  Bougainvillea spectabilis

<400>  558

Ala Lys Val Asp Arg Lys Asp Leu Glu Leu Gly Val Tyr Lys Leu
1               5                   10                  15


<210>  559
<211>  5
<212>  PRT
<213>  Homo sapiens

<400>  559

Ala Ala Lys Ala Asp
1               5


<210>  560
<211>  5
<212>  PRT
<213>  Homo sapiens

<400>  560

Ala Lys Ala Asp Arg
1               5

<210>  561
<211>  5
<212>  PRT
<213>  Homo sapiens

<400>  561

Lys Ala Asp Arg Lys
1               5


<210>  562
<211>  6
<212>  PRT
<213>  Homo sapiens

<400>  562

Ala Ala Lys Ser Asp Arg
1               5


<210>  563
<211>  6
<212>  PRT
<213>  Homo sapiens

<400>  563

Lys Ser Asp Arg Lys Asp
1               5


<210>  564
<211>  5
<212>  PRT
<213>  Homo sapiens

<400>  564

Ala Ala Lys Thr Asp
1               5


<210>  565
<211>  5
<212>  PRT
<213>  Homo sapiens

<400>  565

Ala Lys Thr Asp Arg
1               5


<210>  566
<211>  5
<212>  PRT
<213>  Homo sapiens

<400>  566

Lys Thr Asp Arg Lys
1               5

<210>  567
<211>  15
<212>  PRT
<213>  Bougainvillea spectabilis

<400>  567

Leu Gly Val Tyr Lys Leu Glu Phe Ser Ile Glu Ala Ile His Gly
1               5                   10                  15


<210>  568
<211>  5
<212>  PRT
<213>  Homo sapiens

<400>  568

Glu Leu Gly Pro Gln
1               5


<210>  569
<211>  5
<212>  PRT
<213>  Homo sapiens

<400>  569

Leu Gly Pro Gln Lys
1               5


<210>  570
<211>  6
<212>  PRT
<213>  Homo sapiens

<400>  570

Gly Pro Gln Lys Leu Glu
1               5


<210>  571
<211>  5
<212>  PRT
<213>  Homo sapiens

<400>  571

Glu Leu Gly Gly Lys
1               5


<210>  572
<211>  6
<212>  PRT
<213>  Homo sapiens

<400>  572

Leu Gly Gly Lys Lys Leu
1               5


<210>  573
<211>  6

<212>  PRT
<213>  Homo sapiens

<400>  573

Gly Gly Lys Lys Leu Glu
1               5


<210>  574
<211>  5
<212>  PRT
<213>  Homo sapiens

<400>  574

Glu Leu Gly Asn Ser
1               5


<210>  575
<211>  6
<212>  PRT
<213>  Homo sapiens

<400>  575

Leu Gly Asn Ser Lys Leu
1               5


<210>  576
<211>  6
<212>  PRT
<213>  Homo sapiens

<400>  576

Gly Asn Ser Lys Leu Glu
1               5


<210>  577
<211>  7
<212>  PRT
<213>  Homo sapiens

<400>  577

Glu Leu Gly Gln Ala Lys Leu
1               5


<210>  578
<211>  7
<212>  PRT
<213>  Homo sapiens

<400>  578

Leu Gly Gln Ala Lys Leu Glu
1               5


<210>  579
<211>  5
<212>  PRT
<213>  Homo sapiens

<400>  579

Glu Leu Gly Gln Asp
1               5


<210>  580
<211>  5
<212>  PRT
<213>  Homo sapiens

<400>  580

Leu Gly Gln Asp Lys
1               5


<210>  581
<211>  5
<212>  PRT
<213>  Homo sapiens

<400>  581

Gln Asp Lys Leu Glu
1               5


<210>  582
<211>  15
<212>  PRT
<213>  Bougainvillea spectabilis

<400>  582

Asn Gly Gln Glu Ile Ala Lys Phe Phe Leu Ile Val Ile Gln Met
1               5                   10                  15


<210>  583
<211>  5
<212>  PRT
<213>  Homo sapiens

<400>  583

Gly Gln Glu Gln Ala
1               5


<210>  584
<211>  5
<212>  PRT
<213>  Homo sapiens

<400>  584

Gln Glu Gln Ala Lys
1               5


<210>  585
<211>  5
<212>  PRT
<213>  Homo sapiens

<400>  585

Glu Gln Ala Lys Phe
1               5


<210>  586
<211>  5
<212>  PRT
<213>  Homo sapiens

<400>  586

Gly Gln Glu Arg Ala
1               5


<210>  587
<211>  5
<212>  PRT
<213>  Homo sapiens

<400>  587

Gln Glu Arg Ala Lys
1               5


<210>  588
<211>  5
<212>  PRT
<213>  Homo sapiens

<400>  588

Glu Arg Ala Lys Phe
1               5


<210>  589
<211>  15
<212>  PRT
<213>  Hirudo medicinalis

<400>  589

Cys Ile Leu Gly Ser Asp Gly Glu Lys Asn Gln Cys Val Thr Gly
1               5                   10                  15


<210>  590
<211>  4
<212>  PRT
<213>  Homo sapiens

<400>  590

Lys Cys Arg His
1


<210>  591
<211>  15
<212>  PRT
<213>  Artificial

<220>
<223>  Modified Peptide

<400>  591

Cys Arg His Gly Ser Asp Gly Glu Lys Asn Gln Cys Val Thr Gly
1               5                   10                  15


<210>  592
<211>  20
<212>  PRT
<213>  Homo sapiens

<400>  592

Gly Gly Ser Asn Asn Leu Ser Cys Leu Thr Ile Pro Ala Ser Ala Asn
1               5                   10                  15


Asn Gly Gly Ser
            20


<210>  593
<211>  120
<212>  PRT
<213>  Homo sapiens

<400>  593

Glu Val Gln Leu Val Glu Ser Gly Gly Gly Leu Val Gln Pro Gly Gly
1               5                   10                  15


Ser Leu Arg Leu Ser Cys Ala Ala Ser Gly Phe Asn Ile Lys Asp Thr
            20                  25                  30


Tyr Ile His Trp Val Arg Gln Ala Pro Gly Lys Gly Leu Glu Trp Val
        35                  40                  45


Ala Arg Ile Tyr Pro Thr Asn Gly Tyr Thr Arg Tyr Ala Asp Ser Val
    50                  55                  60


Lys Gly Arg Phe Thr Ile Ser Ala Asp Thr Ser Lys Asn Thr Ala Tyr
65                  70                  75                  80


Leu Gln Met Asn Ser Leu Arg Ala Glu Asp Thr Ala Val Tyr Tyr Cys
                85                  90                  95


Ser Arg Trp Gly Gly Asp Gly Phe Tyr Ala Met Asp Val Trp Gly Gln
            100                 105                 110


Gly Thr Leu Val Thr Val Ser Ser
        115                 120


<210>  594
<211>  108
<212>  PRT
<213>  Homo sapiens

<400>  594

Asp Ile Gln Met Thr Gln Ser Pro Ser Ser Leu Ser Ala Ser Val Gly

1                   5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Glu Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys Arg
            100                 105

<210>  595
<211>  121
<212>  PRT
<213>  Homo sapiens

<400>  595

Glu Val Gln Leu Val Glu Ser Gly Gly Gly Val Val Gln Pro Gly Arg
1               5                   10                  15

Ser Leu Arg Leu Ser Cys Ser Ser Ser Gly Phe Thr Phe Ser Asp Tyr
            20                  25                  30

Tyr Met Tyr Trp Val Arg Gln Ala Pro Gly Lys Gly Leu Glu Trp Val
        35                  40                  45

Ala Tyr Met Ser Asn Val Gly Ala Ile Thr Asp Tyr Pro Asp Thr Val
    50                  55                  60

Lys Gly Arg Phe Thr Ile Ser Arg Asp Asn Ala Lys Ser Thr Leu Tyr
65                  70                  75                  80

Leu Gln Met Asp Ser Leu Arg Pro Glu Asp Thr Gly Val Tyr Phe Cys
                85                  90                  95

Ala Arg Gly Thr Arg Asp Gly Ser Trp Phe Ala Tyr Ala Val Trp Gly
            100                 105                 110

Gln Gly Thr Pro Val Thr Val Ser Ser
        115                 120

<210>  596
<211>  112

<212>  PRT
<213>  Homo sapiens

<400>  596

Asp Ile Gln Met Thr Gln Ser Pro Ser Ser Leu Ser Ala Ser Val Gly
1               5                   10                  15

Asp Arg Val Thr Ile Thr Cys Arg Ser Ser Gln Arg Ile Val His Ser
            20                  25                  30

Asn Gly Asn Thr Tyr Leu Glu Trp Tyr Gln Gln Thr Pro Gly Lys Ala
        35                  40                  45

Pro Lys Leu Leu Ile Tyr Lys Val Ser Asn Arg Phe Ser Gly Val Pro
    50                  55                  60

Ser Arg Phe Ser Gly Ser Gly Ser Gly Thr Asp Phe Thr Phe Thr Ile
65                  70                  75                  80

Ser Ser Leu Gln Pro Glu Asp Ile Ala Thr Tyr Tyr Cys Phe Gln Gly
                85                  90                  95

Ser His Val Pro Phe Thr Phe Gly Gln Gly Thr Lys Leu Gln Ile Thr
            100                 105                 110


<210>  597
<211>  120
<212>  PRT
<213>  Homo sapiens

<400>  597

Glu Val Gln Leu Val Glu Ser Gly Gly Gly Leu Val Gln Pro Gly Gly
1               5                   10                  15

Ser Leu Arg Leu Ser Cys Ala Val Ser Gly Tyr Ser Ile Thr Ser Gly
            20                  25                  30

Tyr Ser Trp Asn Trp Ile Arg Gln Ala Pro Gly Lys Gly Leu Glu Trp
        35                  40                  45

Val Ala Ile Thr Tyr Asp Gly Ser Thr Asn Tyr Asn Pro Ser Val Lys
    50                  55                  60

Gly Arg Ile Thr Ile Ser Arg Asp Asp Ser Lys Asn Thr Phe Tyr Leu
65                  70                  75                  80

Gln Met Asn Ser Leu Arg Ala Glu Asp Thr Ala Val Tyr Tyr Cys Ala
                85                  90                  95

Arg Gly Ser His Tyr Phe Gly His Trp His Phe Ala Val Trp Gly Gln
            100                 105                 110

Gly Thr Leu Val Thr Val Ser Ser
        115                 120

<210>  598
<211>  111
<212>  PRT
<213>  Homo sapiens

<400>  598

<210>  599
<211>  192
<212>  PRT
<213>  Homo sapiens

<400>  599

Glu Val Lys Leu Val Glu Ser Gly Gly Gly Leu Val Gln Pro Gly Gly
1               5                   10                  15

Ser Met Arg Leu Ser Cys Val Ala Ser Gly Phe Thr Phe Ser Asn His
            20                  25                  30

Trp Met Asn Trp Val Arg Gln Ala Pro Gly Lys Gly Leu Glu Trp Val
        35                  40                  45

Ala Glu Ile Arg Ser Lys Ser Ile Asn Ser Ala Thr His Tyr Ala Glu
    50                  55                  60

Ser Val Lys Gly Arg Phe Thr Ile Ser Arg Asp Asp Ser Lys Ser Ser
65                  70                  75                  80

Val Tyr Leu Gln Met Asn Asp Leu Lys Thr Glu Asp Thr Gly Val Glu
                85                  90                  95

Val Lys Leu Glu Glu Ser Gly Gly Gly Leu Val Gln Pro Gly Gly Ser
                100                 105                 110

Met Lys Leu Ser Cys Val Ala Ser Gly Phe Ile Phe Ser Asn His Trp
            115                 120                 125

Met Asn Trp Tyr Arg Gln Ser Pro Glu Lys Gly Leu Glu Trp Val Ala
    130                 135                 140

Glu Ile Arg Ser Lys Ser Ile Asn Ser Ala Thr His Tyr Ala Glu Ser
145                 150                 155                 160

Val Lys Gly Gly Arg Phe Thr Ile Ser Arg Asp Asp Ser Lys Ser Ala
                165                 170                 175

Val Tyr Leu Gln Met Thr Asp Leu Arg Thr Glu Asp Thr Gly Val Tyr
                180                 185                 190


<210>  600
<211>  200
<212>  PRT
<213>  Homo sapiens

<400>  600

Asp Ile Leu Leu Thr Gln Ser Pro Asp Ile Gln Ser Val Thr Pro Lys
1               5                   10                  15

Glu Lys Val Thr Ile Thr Cys Arg Ala Ser Gln Phe Gly Ser Ser Ile
            20                  25                  30

His Trp Tyr Gln Gln Lys Thr Asp Gln Ser Pro Lys Leu Leu Ile Lys
        35                  40                  45

Tyr Ala Ser Glu Ser Met Ser Gly Ile Pro Ser Arg Phe Ser Gly Ser
    50                  55                  60

Gly Ser Gly Thr Asp Phe Thr Leu Thr Ile Asn Ser Leu Glu Ser Glu
65                  70                  75                  80

Asp Ala Ala Asp Tyr Tyr Cys Gln Gln Ser His Ser Trp Pro Phe Thr
                85                  90                  95

Phe Gly Gln Asp Ile Leu Leu Thr Gln Ser Pro Ala Ile Leu Ser Val
            100                 105                 110

Ser Pro Gly Glu Arg Val Ser Phe Ser Cys Arg Ala Ser Gln Phe Val
        115                 120                 125

Gly Ser Ser Ile His Trp Tyr Gln Gln Arg Thr Asn Gly Ser Pro Arg
    130                 135                 140

Leu Leu Ile Lys Tyr Ala Ser Glu Ser Met Ser Gly Ile Pro Ser Arg
145                 150                 155                 160

Phe Ser Gly Ser Gly Ser Gly Thr Asp Phe Thr Leu Ser Ile Asn Thr
                165                 170                 175

Val Glu Ser Glu Asp Ile Ala Asp Tyr Tyr Cys Gln Gln Ser His Ser
            180                 185                 190

Trp Pro Phe Thr Phe Gly Ser Gly
        195                 200